(19) Europäisches Patentamt
European Patent Office
Office européen des brevets

(11) EP 3 821 947 A1

# (12) EUROPEAN PATENT APPLICATION

(43) Date of publication:
**19.05.2021 Bulletin 2021/20**

(21) Application number: **19208888.8**

(22) Date of filing: **13.11.2019**

(51) Int Cl.:
*A61P 25/02* (2006.01) *A61P 25/28* (2006.01)
*C07D 207/06* (2006.01) *C07D 209/08* (2006.01)
*C07D 211/14* (2006.01) *C07D 211/38* (2006.01)
*C07D 211/46* (2006.01) *C07D 217/04* (2006.01)
*C07D 277/32* (2006.01) *C07D 295/04* (2006.01)
*C07D 295/205* (2006.01) *C07D 333/18* (2006.01)
*C07D 333/24* (2006.01) *C07D 333/70* (2006.01)
*C07D 401/12* (2006.01) *C07D 409/04* (2006.01)
*C07D 471/08* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Libra Therapeutics, Inc.**
**Wilmington, DE 19801 (US)**

(72) Inventors:
- **PEVARELLO, Paolo**
  **20091 Bresso (MI) (IT)**
- **CUSANO, Valentina**
  **20091 Bresso (MI) (IT)**
- **SODANO, Mariangela**
  **20091 Bresso (MI) (IT)**
- **TORINO, Domenica**
  **20091 Bresso (MI) (IT)**
- **VITALONE, Rocco**
  **20091 Bresso (MI) (IT)**
- **LIBERATI, Chiara**
  **20091 Bresso (MI) (IT)**

(74) Representative: **Bianchetti Bracco Minoja S.r.l.**
**Via Plinio, 63**
**20129 Milano (IT)**

## (54) HETEROCYCLIC TRPML1 AGONISTS

(57) The invention relates to a compound of formula (I)

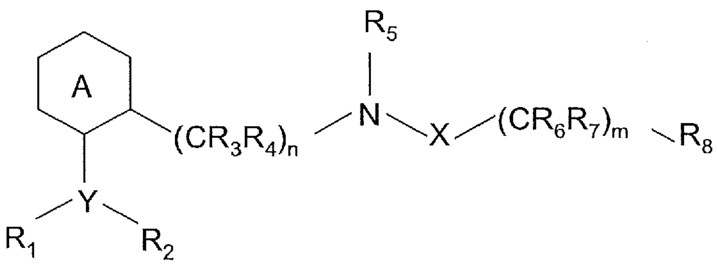

(I)

or a stereoisomer thereof, or a salt of any of the foregoing and to processes for its preparation. The compounds of formula (I) are useful in the treatment TRPML1- mediated disorders or diseases.

EP 3 821 947 A1

## Description

Disclosed herein are novel heterocyclic compounds having Transient Receptor Potential Cation Channel, Mucolipin subfamily, member 1 (TRPML1) agonistic properties, pharmaceutical compositions comprising these compounds, chemical processes for preparing these compounds and their use in the treatment or prophylaxis of diseases associated with TRPML1 receptor activity in animals, in particular humans.

TRPML1, also named Mucolipin-1, is a ligand-gated cation channel expressed mostly in intracellular organelles like the late endosome and lysosome of many mammalian cells. This channel is member of the large family of Transient receptor potential (TRP) channels and has, with TRPML2 and TRPML3, two close analogues. Loss-of-function mutations in the gene encoding for TRPML1, the 12’000 base pair gene MCOLN-1 located in human chromosome 19p13, are the direct cause of Type IV mucolipidosis (MLIV), an autosomal recessive lysosomal storage disease.

At the molecular level, TRPML1 is a $Ca^{2+}$-permeable, non-selective cation channel formed of four six-transmembrane spanning proteins each of 580 amino acids. The channel opens upon binding of its endogenous ligand phosphatidylinositol-3,5-bisphosphate (PtdIns(3,5)P2)) to its pore region. Channel activity is modulated by pH and PtdIns(4,5)P2 levels. TRPML1 is an inwardly rectifying channel permeable for different mono- and divalent cations, including $Na^+$, $K^+$, $Ca^{2+}$ and $Fe^{2+}$. Its N-terminal AP1 sequence targets the channel to the lysosome while a C-terminal AP2 sequence is responsible for intracellular trafficking and internalization. In addition, TRPML1 has four putative N-linked glycosylation sites in its luminal loop between TM1 and 2. It is reported that TRPML channels can be formed as homotetramers (e.g. TRPML1, TRPML2, TRPML3) but also in some cases as hetero-tetramers where one channel is composed of different members of the TRPML family.

TRPML1 is found in all mammalian tissues with highest expression levels in brain, spleen, liver, kidney and heart. Expression is found in many cell types, including neurons, myeloid cells, macrophages, microglia, podocytes and muscle cells. TRPML1 is involved in function of late endosome / lysosomes (LELs), more specifically in protein trafficking and lysis as well as autophagy.

Lysosomes are organelles filled with hydrolytic enzymes, characterized by low luminal pH of about 5, a high luminal $Ca_2^+$ concentration of about 0.5 mM and a membrane polarization of about +60 mV.

TRPML1 in LELs is reported to be responsible for the formation of transport vesicles and required for the reformation of lysosomes from LEL hybrid organelles and autolysosomes, mostly due to its $Ca_2^+$ permeability. It seems also important for iron release from the lysosome after degradation of iron-binding proteins like cytochrome C. In addition, TRPML1 is reported to regulate autophagy, probably in an mTOR-independent manner, by promoting TFEB translocation to the nucleus via calcineurin activation.

In MLIV, the lack of functional TRPML1 leads to severe intellectual disability, motor deficits, retinal degeneration and systemic symptoms leading to a strongly reduced life expectancy. Cells from MLIV patients show increased autophagosomes, accumulation of lysofuscin and lipid accumulation in the lysosomes.

Failure of TRPML1-dependent autophagosome-lysosome fusion is also thought to impair clearance of apoptotic neurons by macrophages and microglia cells. Experimental results suggest involvement of TRPML1 in neurodegenerative diseases like Alzheimer’s and amyotrophic lateral sclerosis (ALS). For example, Alzheimer’s disease related loss-of-function mutations in presenilin 1 lead to dysregulation of lysosomal $Ca_2^+$ homeostasis via TRPML1 modulation. On the other side, over-expression of TRPML1 in rodent Alzheimer’s models reduced neuronal apoptosis and rescued memory impairments. Pharmacological activation of TRPML1 showed similar effects, clearing accumulated sphingolipids and $A\beta$ peptides from lysosomes. In another study TRPML1 activation was sufficient to upregulate lysosomal exocytosis, rescue defective $\alpha$-syn secretion and prevent $\alpha$-syn accumulation in iPSC-derived dopaminergic neurons from patients expressing mutant PARK9. Similarly, TRPML1 activation rescued motor neurons from death and ER stress induced by the cycad neurotoxin beta-methylamino-L-alanine, L-BMAA as a model for ALS.

Therefore, it is desired to develop TRPML1 modulators to rescue impaired lysosomal function and cellular autophagy in neurodegenerative diseases.

WO2018005713 describes TRPML1 agonist molecules. Unfortunately, despite widespread interest for several years across the pharmaceutical industry, there are no drug-like small molecule agonists or positive allosteric modulators of any kind of TRPML1.

Consequently, there is still an unmet need for compounds which can efficiently stimulate TRPML1 and that can be delivered to the different target organs which are sites of any TRPML1-mediated pathology. Such compounds are provided herein.

Various embodiments of the compounds provided herein are presented hereafter.

## DETAILED DESCRIPTION

Disclosed herein are compounds of the following formula (I)

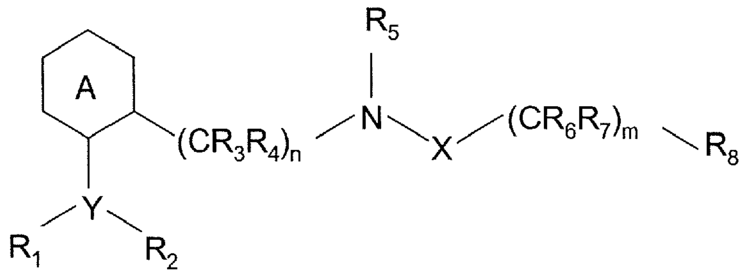

(I)

or a stereoisomer thereof, or a salt of any of the foregoing, wherein:

A is a six membered aliphatic, aromatic or heteroaromatic ring, optionally substituted by one or more substituents selected from the group consisting of $C_1$-$C_4$ alkyl, $C_1$-$C_4$ alkyloxy, $C_3$-$C_6$ cycloalkyl, halogen, and cyano.
Y is chosen from CH, or N;
each of $R_1$ and $R_2$ independently is a $C_1$-$C_4$ alkyl group or $R_1$ and $R_2$ taken together with Y form a four, five, six or seven membered heteroaliphatic ring, containing at least one nitrogen atom, optionally fused with a six membered aromatic ring, optionally substituted by one or more substituents selected from the group consisting of:

- halogen;
- $C_1$-$C_4$ alkyl, $C_1$-$C_4$ alkyloxy, $C_1$-$C_4$ alkylcarbonyl, $C_1$-$C_4$ alkoxycarbonyl, wherein the $C_1$-$C_4$ alkyl chain is optionally substituted by one or more halogen and $C_1$-$C_4$-alkyloxy;
- phenyl, benzyl, benzoyl, phenoxy, benzyloxy, pyrazin-2-yl-oxy, pyridin-2-yl-oxy, pyridin-3-yl-oxy, pyrimidin-2-yl-oxy thiophene-2-carbonyl, 1,3 thiazole-5-carbonyl, thiophene-2-yl-acetyl, wherein the aromatic ring is optionally substituted by one or more halogen, $C_1$-$C_4$ alkyl, $C_1$-$C_4$-alkyloxy;
- a $C_1$-$C_2$ alkylene bridge; and
- $R_{11}R_{12}$N-CO- wherein each of $R_{11}$ and $R_{12}$ is $C_1$-$C_4$ alkyl;

X is -CO- or -$SO_2$-;
each of $R_3$, $R_4$, $R_5$, $R_6$ and $R_7$ independently is hydrogen or $C_1$-$C_4$ alkyl;
each of m and n independently is 0 or 1;
$R_8$ is a five or six membered monocyclic or a nine or ten membered bicyclic aromatic or heteroaromatic compound, optionally substituted by one or more substituents selected from the group consisting of halogen, pentafluorosulfanyl ($SF_5$), trifluoromethylthio ($SCF_3$), $C_1$-$C_4$ alkyl or $C_1$-$C_6$ cycloalkyl, and 1,3-dioxolan-2-yl, optionally substituted by a cyano group, -$NO_2$, $C_1$-$C_4$ alkyloxy, $C_1$-$C_4$ alkylthio, -$SO_2NR_9R_{10}$ or -$NR_9R_{10}$, wherein each of $R_9$ and $R_{10}$ independently is hydrogen or $C_1$-$C_4$ alkyl or $R_9$ and $R_{10}$ are taken together to the nitrogen atom to which they are bound to form a five or six membered heteroaliphatic ring;
provided that when $R_1$ and $R_2$ taken together with Y form a piperazine substituted by phenyl or benzyl, then $R_8$ is not phenyl.

$C_1$-$C_4$ alkyl group means a straight or branched alkyl chain containing 1 to 4 carbon atoms such as methyl, ethyl, n-propyl, sec-propyl, n-butyl, sec-butyl and tert-butyl, preferably is methyl, ethyl or tert-butyl, most preferably methyl.
Preferably, A is cyclohexyl or phenyl.
Preferably, $R_1$ and $R_2$ are methyl and Y is N or $R_1$ and $R_2$ taken together with Y form a piperidine, piperazine, pyrrolidine, azetidine, azepane, morpholine, 1,2,3,4-tetrahydroisoquinoline or 2,3- dihydroindole, wherein each of said moieties is optionally substituted by: halogen, preferably fluorine or chlorine; $C_1$-$C_4$ alkyl, preferably methyl, ethyl, n-propyl or tert-butyl; $C_1$-$C_4$ alkyloxy, preferably methoxy; $C_1$-$C_4$ alkylcarbonyl (wherein the $C_1$-$C_4$ alkyl group is optionally substituted by one or more halogen, preferably fluorine or chlorine, or $C_1$-$C_4$-alkyloxy, preferably methoxy), preferably methylcarbonyl, methoxyethylcarbonyl; $C_1$-$C_4$ alkoxycarbonyl (wherein the $C_1$-$C_4$ alkyl group is optionally substituted by one or more halogen, preferably fluorine or chlorine or $C_1$-$C_4$-alkyloxy, preferably methoxy), preferably methoxycarbonyl, ethoxycarbonyl, tert-butoxycarbonyl, 2,2,2-trifluoroethoxycarbonyl; $C_1$-$C_4$ alkylaminocarbonyl, preferably propylaminocarbonyl or isobutylaminocarbonyl; $C_1$-$C_4$ alkylcarbonyl, preferably isobutylcarbonyl; a compound containing an aromatic ring selected from phenyl, benzyl, phenoxy, benzyloxy, pyrazin-2-yl-oxy and pyridin-2-yl-oxy (wherein the aromatic ring is optionally substituted by one or more halogen, preferably fluorine or chlorine, or $C_1$-$C_4$-alkyloxy); or a $C_1$-$C_2$ alkylene bridge, preferably a methylene bridge.

Preferably, each of $R_3$, $R_4$, $R_5$, $R_6$ and $R_7$ are hydrogen or methyl, most preferably hydrogen.

Preferably, both m and n are 0 or one of m and n is 0 and the other is 1.

Preferably, $R_8$ is selected from the group consisting of phenyl, thiophene, benzothiophene, optionally substituted by one or more substituents selected from the group consisting of halogen, preferably fluorine or chlorine or bromo, or pentafluorosulfanyl ($SF_5$), or trifluoromethylthio ($SCF_3$); $C_1$-$C_4$ alkyl optionally substituted by a cyano group, preferably methyl, ethyl, n-propyl, tert-butyl or cyanomethyl; -$NO_2$; $C_1$-$C_4$ alkoxy, preferably methoxy, ethoxy; $C_1$-$C_4$ alkylthio, preferably methylthio, ethylthio; -$SO_2NR_9R_{10}$ or -$NR_9R_{10}$, wherein each of $R_9$ and $R_{10}$ independently is hydrogen or $C_1$-$C_4$ alkyl, preferably methyl, ethyl, most preferably both $R_9$ and $R_{10}$ are methyl, or $R_9$ and $R_{10}$ are taken together with the nitrogen atom to form a five or six membered heteroaliphatic ring, optionally containing one or two oxygen atoms, preferably piperidine, pyrrolidine, morpholine, 1,3 dioxolane.

A preferred embodiment of the invention relates to compounds of formula (I) or a stereoisomer or a salt thereof as defined above wherein:

A is phenyl or cyclohexyl;
Y is N and $R_1$ and $R_2$ taken together with Y form a cyclic compound selected from piperidine, pyrrolidine, azete, azepane, 2-azabicyclo[2.2.1]heptane, 4-phenylpiperidine, tert-butyl piperazine-1-carboxylate, 4-phenoxypiperidine, 4-(benzyloxy)piperidine, 4-methoxypiperidine, morpholine, dimethylamine, pyrrolidine, 4,4-difluoropiperidine, 3-phenylpiperidine, 3-methoxypiperidine, 1-(4-chloro-2-fluorophenyl)piperazine, 3-phenylpyrrolidine, 2,3-dihydro-1H-indole, 1,2,3,4-tetrahydroquinoline, methyl piperazine-1-carboxylate, 2,2,2-trifluoroethyl piperazine-1-carboxylate, ethyl piperazine-1-carboxylate, 1-(piperazin-1-yl)propan-1-one, 3-methoxy-1-(piperazin-1-yl)propan-1-one, 1-benzoylpiperazine, 2-(piperidin-4-yloxy)pyridine, 2-(piperidin-4-yloxy)pyrazine, tert-butyl piperidine-4-carboxylate, tert-butyl piperidine-1-carboxylate, 4-(pyridin-2-yl)piperidine, 4-(pyrazin-2-yl)piperidine, 4-(thiophene-2-carbonyl)piperazine, 4-(4-methyl-1,3 -thiazole-5 -carbonyl)piperazine, 4-(piperazin-1-yl)-2-(thiophen-2-yl)ethan-1-one, N-methyl-N-propylpiperazine-1-carboxamide, 4-(thiophene-2-carbonyl)piperazine, 4-(4-methyl-1,3-thiazole-5-carbonyl)piperazine, 4-(piperazin-1-yl)-2-(thiophen-2-yl)ethan-1-one, N-methyl-N-propylpiperazine-1-carboxamide, 3 -(piperidin-4-yloxy)pyridine, N-propylpiperidine-4-carboxamide, N-(2-methylpropyl)piperidine-4-carboxamide, 2-(piperidin-4-yloxy)pyrimidine, piperidin-4-yl 2,2-dimethylpropanoate;
both m and n are 0 or one of m and n is 0 and the other is 1;
$R_3$, $R_4$, $R_6$ and $R_7$ are hydrogen;
$R_5$ is hydrogen or methyl;
X is -CO- or -$SO_2$-;
$R_8$ is selected from phenyl, thiophene or benzothiophene, optionally substituted by dimethylsulfamoyl, methyl, ethyl, propyl, tert-butyl, 1,3-dioxolan-2-yl, cyanomethyl, monoalkylamino, dialkylamino, piperidine-1-sulfonyl, pyrrolidine-1-sulfonyl, morpholine-4-sulfonyl, fluoro, chloro, bromo, $SF_5$, $SCF_3$, nitro, and $C_1$-$C_4$ alkoxy.

In certain embodiments, a compound of formula (I) disclosed herein is chosen from the compounds set forth in Table 1 or a stereoisomer or a salt thereof.

**Table 1.**

| Ex. | Chemical Name |
|---|---|
| 1 | 4-methyl-N-[2-(piperidin-1-yl)phenyl]benzene-1-sulfonamide |
| 2 | 4-methyl-N-[2-(piperidin-1-yl)phenyl]thiophene-2-sulfonamide |
| 3 | 5-tert-butyl-N-[2-(piperidin-1-yl)phenyl]thiophene-2-sulfonamide |
| 4 | N4-(2-{2-azabicyclo[2.2.1]heptan-2-yl}phenyl)-N1,N1-dimethylbenzene-1,4-disulfonamide |
| 5 | N-(2-{2-azabicyclo[2.2.1]heptan-2-yl}phenyl)-4-methylbenzene-1-sulfonamide |
| 6 | N1,N1-dimethyl-N4-[2-(piperidin-1-yl)phenyl]benzene-1,4-disulfonamide |
| 7 | 5-ethyl-N-[2-(piperidin-1-yl)phenyl]thiophene-2-sulfonamide |
| 8 | 3,5-dimethyl-N-[2-(piperidin-1-yl)phenyl]thiophene-2-sulfonamide |
| 9 | N-[2-(piperidin-1-yl)phenyl]-5-propylthiophene-2-sulfonamide |
| 10 | N-[2-(piperidin-1-yl)phenyl]-1-benzothiophene-2-sulfonamide |
| 11 | 3-(1,3-dioxolan-2-yl)-N-[2-(piperidin-1-yl)phenyl]thiophene-2-sulfonamide |
| 12 | N 1,N1-dimethyl-N4-[2-(4-phenylpiperidin-1-yl)phenyl]benzene-1,4-disulfonamide |

(continued)

| Ex. | Chemical Name |
|---|---|
| 13 | tert-butyl 4-{2-[4-(dimethylsulfamoyl)benzenesulfonamido]phenyl}piperazine-1-carboxylate |
| 14 | 5-tert-butyl-N-{2-[4-(4-chloro-2-fluorophenyl)piperazin-1-yl]phenyl}thiophene-2-sulfonamide |
| 15 | N-(2-{2-azabicyclo[2.2.1 ]heptan-2-yl}phenyl)-5-methylthiophene-2-sulfonamide |
| 16 | N-(2-{2-azabicyclo[2.2.1 ]heptan-2-yl} phenyl)-5-tert-butylthiophene-2-sulfonamide |
| 17 | N-(2-{2-azabicyclo[2.2.1]heptan-2-yl}phenyl)-5-ethylthiophene-2-sulfonamide |
| 18 | N-(2-{2-azabicyclo[2.2.1]heptan-2-yl}phenyl)-4-(dimethylsulfamoyl)benzamide |
| 19 | 4-(dimethylsulfamoyl)-N-[2-(piperidin-1-yl)phenyl]benzamide |
| 20 | 4-(cyanomethyl)-N-[2-(piperidin-1-yl)phenyl]benzene-1-sulfonamide |
| 21 | 4-tert-butyl-N-[2-(piperidin-1-yl)phenyl]benzene-1-sulfonamide |
| 22 | 4-(dimethylamino)-N-[2-(piperidin-1-yl)phenyl]benzene-1-sulfonamide |
| 23 | 3-methyl-N-[2-(piperidin-1-yl)phenyl]benzene-1-sulfonamide |
| 24 | N1,N1-dimethyl-N4-[2-(4-phenoxypiperidin-1-yl)phenyl]benzene-1,4-disulfonamide |
| 25 | N4-{2-[4-(benzyloxy)piperidin-1-yl]phenyl}-N1,N1-dimethylbenzene-1,4-disulfonamide |
| 26 | N4-[2-(4-methoxypiperidin-1-yl)phenyl]-N1,N1-dimethylbenzene-1,4-disulfonamide |
| 27 | N1,N1-dimethyl-N4-[2-(morpholin-4-yl)phenyl]benzene-1,4-disulfonamide |
| 28 | N4-[2-(dimethylamino)phenyl]-N1,N1-dimethylbenzene-1,4-disulfonamide |
| 29 | N-[2-(piperidin-1-yl)phenyl]-4-(piperidine-1-sulfonyl)benzene-1-sulfonamide |
| 30 | N-[2-(piperidin-1-yl)phenyl]-4-(pyrrolidine-1-sulfonyl)benzene-1-sulfonamide |
| 31 | 4-(morpholine-4-sulfonyl)-N-[2-(piperidin-1-yl)phenyl]benzene-1-sulfonamide |
| 32 | N1,N1-dimethyl-N4-[2-(pyrrolidin-1-yl)phenyl]benzene-1,4-disulfonamide |
| 33 | N4-[2-(4,4-difluoropiperidin-1-yl)phenyl]-N1,N1-dimethylbenzene-1,4-disulfonamide |
| 34 | 2-[4-(dimethylsulfamoyl)phenyl]-N-[2-(pyrrolidin-1-yl)phenyl]acetamide |
| 35 | N1,N1-dimethyl-N4-[2-(3-phenylpiperidin-1-yl)phenyl]benzene-1,4-disulfonamide |
| 36 | N4-[2-(3-methoxypiperidin-1-yl)phenyl]-N1,N1-dimethylbenzene-1,4-disulfonamide |
| 37 | N1,N1-dimethyl-N4-[2-(3-phenylpyrrolidin-1-yl)phenyl]benzene-1,4-disulfonamide |
| 38 | N-{2-[4-(4-chloro-2-fluorophenyl)piperazin-1-yl]phenyl}-4-(dimethylsulfamoyl)benzamide |
| 39 | N1,N1-dimethyl-N4-{[2-(pyrrolidin-1-yl)phenyl] methyl }benzene-1,4-disulfonamide |
| 40 | N4-[2-(2,3-dihydro-1 H-indol-1-yl)phenyl] -N1,N1-dimethylbenzene-1,4-disulfonamide |
| 41 | N1,N1-dimethyl-N4-[2-(1,2,3,4-tetrahydroisoquinolin-2-yl)phenyl]benzene-1,4-disulfonamide |
| 42 | N1,N1-dimethyl-N4-[2-(4-methylpiperazin-1-yl)phenyl]benzene-1,4-disulfonamide |
| 43 | N-(2-{2-azabicyclo[2.2.1]heptan-2-yl}phenyl)-2-[4-(dimethylsulfamoyl)phenyl] acetamide |
| 44 | N4-[2-(4-acetylpiperazin-1-yl)phenyl]-N1,N1-dimethylbenzene-1,4-disulfonamide |
| 45 | 2-[4-(dimethylsulfamoyl)phenyl]-N-[2-(piperidin-1-yl)phenyl]acetamide |
| 47 | N-{2-[4-(4-chloro-2-fluorophenyl)piperazin-1-yl]phenyl}-2-[4-(dimethylsulfamoyl)phenyl]acetamide |
| 48 | N-{2-[4-(4-chloro-2-fluorophenyl)piperazin-1 -yl]phenyl}-4-(dimethylsulfamoyl)-N-methylbenzamide |
| 49 | N-(2-{2-azabicyclo[2.2.1]heptan-2-yl}phenyl)-4-(dimethylsulfamoyl)-N-methylbenzamide |
| 50 | 4-(dimethylsulfamoyl)-N-methyl-N-[2-(piperidin-1-yl)phenyl]benzamide |
| 51 | N-(2- {2-azabicyclo[2.2.1]heptan-2-yl}phenyl)-5-ethyl-N-methylthiophene-2-sulfonamide |

(continued)

| Ex. | Chemical Name |
|---|---|
| 52 | methyl 4-{2-[4-(dimethylsulfamoyl)benzenesulfonamido]phenyl}piperazine-1-carboxylate |
| 53 | 2,2,2-trifluoroethyl 4-{2-[4-(dimethylsulfamoyl)benzenesulfonamido]phenyl}piperazine-1-carboxylate |
| 54 | N4-{2-[4-(2-methoxyphenyl)piperazin-1-yl]cyclohexyl}-N1,N1-dimethylbenzene-1,4-disulfonamide |
| 55 | N1,N1-dimethyl-N4-[2-(4-phenylpiperidin-1-yl)cyclohexyl]benzene-1,4-disulfonamide |
| 56 | N1,N1-dimethyl-N4-[2-(4-phenylpiperazin-1-yl)cyclohexyl]benzene-1,4-disulfonamide |
| 57 | N1,N1-dimethyl-N4-[2-(4-phenoxypiperidin-1-yl)cyclohexyl]benzene-1,4-disulfonamide |
| 58 | N1,N1-dimethyl-N4-[2-(piperidin-1-yl)cyclohexyl]benzene-1,4-disulfonamide |
| 59 | N4-{2-[4-(4-chloro-2-fluorophenyl)piperazin-1-yl]cyclohexyl}-N1,N1-dimethylbenzene-1,4-disulfonamide |
| 61 | N1,N1-dimethyl-N4-[2-(morpholin-4-yl)cyclohexyl]benzene-1,4-disulfonamide |
| 62 | N1,N1-dimethyl-N4-[2-(4-phenoxypiperidin-1-yl)cyclohexyl]benzene-1,4-disulfonamide |
| 63 | N1,N1-dimethyl-N4-[2-(4-phenoxypiperidin-1-yl)cyclohexyl]benzene-1,4-disulfonamide |
| 64 | N4-[2-(2,3-dihydro-1H-indol-1-yl)cyclohexyl]-N1,N1-dimethylbenzene-1,4-disulfonamide |
| 65 | 4-chloro-N-[2-(piperidin-1-yl)phenyl]benzene-1-sulfonamide |
| 66 | 4-(dimethylsulfamoyl)-N-[2-(pyrrolidin-1-yl)phenyl]benzamide |
| 67 | 4-(dimethylsulfamoyl)-N-methyl-N-[2-(pyrrolidin-1-yl)phenyl]benzamide |
| 68 | 4-nitro-N-[2-(piperidin-1-yl)phenyl]benzene-1-sulfonamide |
| 69 | 4-methoxy-N-[2-(piperidin-1-yl)phenyl]benzene-1-sulfonamide |
| 70 | ethyl 4-{2-[4-(dimethylsulfamoyl)benzenesulfonamido]phenyl-carboxylate |
| 71 | N1,N1-dimethyl-N4-[2-(4-propanoylpiperazin-1-yl)phenyl]benzene-1,4-disulfonamide |
| 72 | N4-{2-[4-(3-methoxypropanoyl)piperazin-1-yl]phenyl}-N1,N1-dimethylbenzene-1,4-disulfonamide |
| 73 | N4-[2-(4-benzoylpiperazin-1-yl)phenyl]-N1,N1-dimethylbenzene-1,4-disulfonamide |
| 74 | N1,N1-dimethyl-N4-{[2-(piperidin-1-yl)phenyl]methyl}benzene-1,4-disulfonamide |
| 75 | N1,N1-dimethyl-N4-{2-[4-(pyridin-2-yloxy)piperidin-1 -yl]phenyl }benzene-1,4-disulfonamide |
| 76 | *t*-butyl 1-{2-[4-(dimethylsulfamoyl)benzenesulfonamido]phenyl}piperidine-4-carboxylate |
| 77 | N1,N1-dimethyl-N4-{2-[4-(pyrazin-2-yloxy)piperidin-1-yl]phenyl}benzene-1,4-disulfonamide |
| 78 | *t*-butyl 4-{2-[4-(dimethylsulfamoyl)benzenesulfonamido]phenyl}piperidine-1-carboxylate |
| 79 | N1,N1-dimethyl-N4-{2-[4-(thiophene-2-carbonyl)piperazin-1-yl]phenyl}benzene-1,4-disulfonamide |
| 80 | N1,N1-dimethyl-N4-{2-[4-(4-methyl-1,3-thiazole-5-carbonyl)piperazin-1-yl]phenyl}benzene-1,4-disulfonamide |
| 81 | N1,N1-dimethyl-N4-(2-{4-[2-(thiophen-2-yl)acetyl]piperazin-1-yl}phenyl)benzene-1,4-disulfonamide |
| 82 | 4- {2-[4-(dimethylsulfamoyl)benzenesulfonamido]phenyl} -N-methyl-N-propylpiperazine-1-carboxamide |
| 83 | *t*-butyl 4-[2-(4-methylbenzenesulfonamido)phenyl]piperazine-1-carboxylate |
| 84 | *t*-butyl 4-[2-(4-methoxybenzenesulfonamido)phenyl]piperazine-1-carboxylate |
| 85 | N4-[2-(azepan-1-yl)phenyl]-N1,N1-dimethylbenzene-1,4-disulfonamide |
| 86 | N1,N1-dimethyl-N4-{2-[4-(pyridin-3-yloxy)piperidin-1-yl]phenyl}benzene-1,4-disulfonamide |
| 87 | 1-{2-[4-(dimethylsulfamoyl)benzenesulfonamido]phenyl}-N-propylpiperidine-4-carboxamide |
| 88 | 1-{2-[4-(dimethylsulfamoyl)benzenesulfonamido]phenyl}-N-(2-methylpropyl)piperidine-4-carboxamide |
| 89 | N1,N1-dimethyl-N4-{2-[4-(pyrimidin-2-yloxy)piperidin-1-yl]phenyl}benzene-1,4-disulfonamide |

(continued)

| Ex. | Chemical Name |
|---|---|
| 90 | 1-[2-(4-methylbenzenesulfonamido)phenyl]piperidin-4-yl 2,2-dimethylpropanoate |
| 91 | 1-[2-(4-methoxybenzenesulfonamido)phenyl]piperidin-4-yl 2,2-dimethylpropanoate |

Also provided herein is a compound as disclosed herein for use as a medicament.

Also provided herein is a compound as disclosed herein, or a stereoisomer thereof, or a salt of any of the foregoing, for use in the treatment of a TRPML1-mediated disease.

Also provided herein is a compound as disclosed herein, or a stereoisomer thereof, or a salt of any of the foregoing, for use in the manufacture of a medicament for the prevention or treatment of a disease or condition ameliorated by the modulation of TRPML1.

Also provided herein is a pharmaceutical composition comprising a compound as disclosed herein, or a stereoisomer thereof, or a salt of any of the foregoing, together with a pharmaceutically acceptable carrier.

Also provided herein is a method of treatment of a TRPML1-mediated disease comprising the administration of a therapeutically effective amount of a compound as disclosed herein, or a stereoisomer thereof, or a salt of any of the foregoing, to a subject in need thereof.

In certain embodiments, the disease is chosen from cancer, an inflammatory disorder, pain, a neurodegenerative disorder, a cognitive disorder, and a psychiatric disorder.

In certain embodiments, the disease is Alzheimer's Disease.

Also provided herein is a method of modulation of TRPML1 comprising contacting
TRPML1 with a compound as disclosed herein, or a stereoisomer thereof, or a salt of any of the foregoing.

Also provided herein is a method of making a compound disclosed herein, comprising the procedure(s) described below and variations thereupon.

Compounds of formula (I) can be prepared by reacting a compound of formula (II) or formula (III):

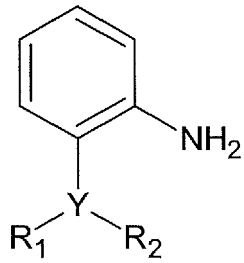

(II)

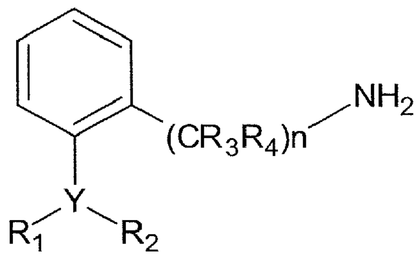

(III)

wherein Y, R1, R2, R3, R4 and n are as defined above, with a compound of formula (IV) or formula (V)

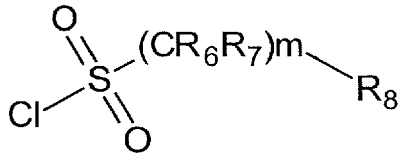

(IV)

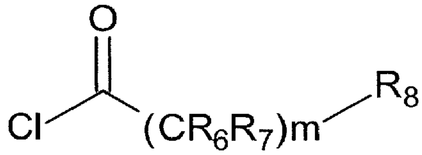

(V)

wherein R6, R7, m and R8 are as defined above.

The reaction of a compound of formula (II) or formula (III) with a compound of formula (IV) or with compound of formula (V) may be carried out in a reaction-inert solvent such as DCM or THF, and in the presence of a suitable base such as TEA or pyridine. The reaction may conveniently be carried out at temperatures between room temperature and the reflux temperature of the reaction mixture and optionally converting the obtained compound of formula (I) into an addition salt thereof, and/or preparing stereochemically isomeric forms thereof.

Reagents of formula (IV) and formula (V) are known in the art.

Reagents of formula (II) or formula (III) either are commercially available, or can be prepared according to the following scheme:

1)

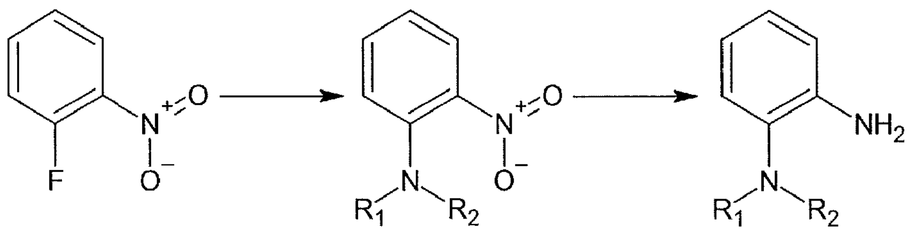

(CAS:1493-27-2) (A) (II)

wherein R1 and R2 are as defined above;

Compounds of formula (I) can also be prepared by reacting a compound of formula (VI):

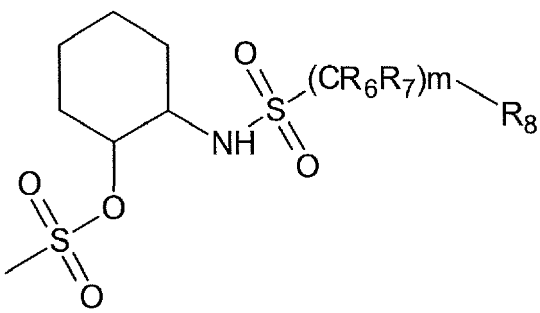

(VI)

wherein $R_6$, $R_7$, m and $R_8$ are as defined above; with a suitable amine, optionally substituted. The reaction may be carried out in a reaction-inert solvent such as DCM or THF, and in the presence of a suitable base such as TEA at room temperature.

Compounds of formula (VI) can be prepared according to the following schemes:

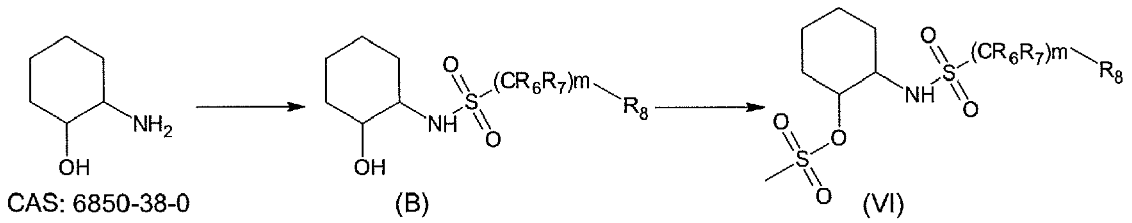

CAS: 6850-38-0 (B) (VI)

Compounds of formula (I) may also be prepared for reaction of compound of formula (VII) or compound of formula (VIII) with $CH_3I$;

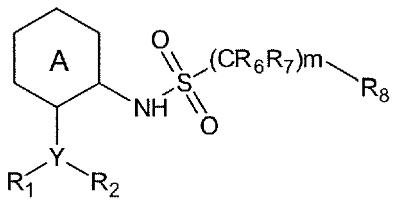

(VII)

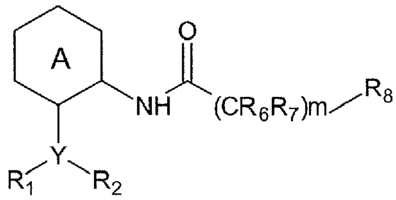

(VIII)

wherein A, Y, $R_1$, $R_2$, $R_6$, $R_7$, m and $R_8$ are as defined above. The reaction may be carried out in a reaction-inert solvent such as DCM or THF, and in the presence of a suitable base such as TEA or NaH at room temperature. Compounds of formula (VII) and formula (VIII) can be prepared according to the previous synthetic schemes.

Compounds of formula (I) may also be prepared for reaction of compound of formula (IX)

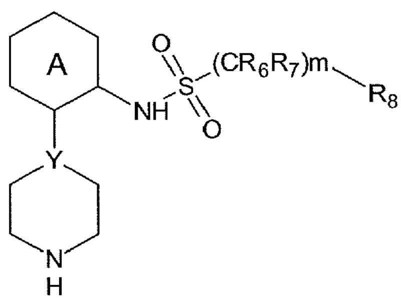

(IX)

wherein A, Y, $R_6$, $R_7$, m and $R_8$ are as defined above, with a suitable compounds of formula (X)

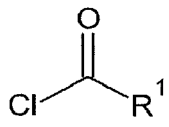

(X)

wherein $R_1$ is as defined above. This reaction may be carried out in a reaction-inert solvent such as DCM or THF, and in the presence of a suitable base such as TEA at room temperature.

Compounds of formula (X) are known in the art.

Compounds of formula (IX) can be prepared according to the following scheme:

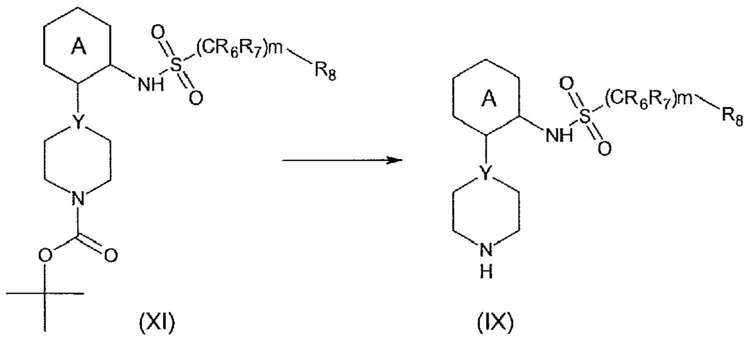

(XI) (IX)

Compounds of formula (I) may also be prepared for reaction of compound of formula (XII)

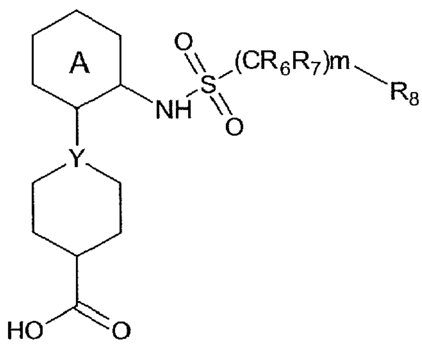

(XII)

wherein A, Y, R6, R7, m and R8 are as defined above, with a suitable compounds of formula (XIII)

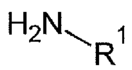

(XIII)

wherein R1 is as defined above. This reaction may be carried out in a reaction-inert solvent such as DCM, and in the presence of an activator such as HATU at room temperature.

Compounds of formula (XII) can be prepared according to the previous schemes.

Compounds of formula (XIII) are known in the art.

The compounds disclosed herein may be synthesized as enantiomers or in the form of racemic mixtures of enantiomers which can be separated from one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example, by selective

or fractional crystallization and the enantiomers are liberated there from by alkali. An alternative manner of separating the enantiomeric forms of the compounds of formula (1) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds of formula (I), the pharmaceutically acceptable salts and stereoisomeric forms thereof possess TRPML1 receptor agonism as demonstrated in the Pharmacological Examples. The compounds of formula (I) as prepared in the hereinabove described processes may be synthesized in the form of racemic mixtures of enantiomers which can be separated from one another following art-known resolution procedures. Those compounds of formula (I) that are obtained in racemic form may be converted into the corresponding diastereomeric salt forms by reaction with a suitable chiral acid. Said diastereomeric salt forms are subsequently separated, for example, by selective or fractional crystallization and the enantiomers are liberated there from by alkali. An alternative manner of separating the enantiomeric forms of the compounds of formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials. In the preparation of the compounds of formula I and the starting materials and/or intermediates described herein it may be useful to protect certain groups which are sensitive to the reaction conditions. The evaluation of the usefulness of the optional protection, as well as the selection of the suitable protecting agent, according to the reaction carried out in the preparation of the compounds provided herein and the functional group to be protected, are within the common knowledge of the skilled person. The removal of the optional protective groups is carried out according to conventional techniques.

The preparation of the salts of the compounds of formula I is carried out according to known methods.

The present compounds of formula (I) are useful in the treatment of a condition or disease mediated by the TRPML1 receptor, in particular TRPML1 receptor agonistic activity. Furthermore, the present compounds may be used for the manufacture of a medicine for treatment of a condition or a disease mediated by TRPML1 receptor activity, in particular TRPML1 receptor agonistic activity.

The present disclosure also provides the use of a compound of formula (I) or a pharmaceutically acceptable salt thereof for the manufacture of a medicament for the treatment of conditions or diseases selected from TRPML1 receptor mediated conditions or diseases.

**Definitions**

As used in the foregoing definitions :

The term "stereochemically isomeric forms" as used hereinbefore defines all the possible isomeric forms which the compounds of formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration.

Stereochemically isomeric forms of the compounds of formula (I) are obviously intended to be embraced within the scope of this invention.

The absolute stereochemical configuration of the compounds of formula (I) and of the intermediates used in their preparation may easily be determined by those skilled in the art while using well-known methods such as, for example, X-ray diffraction.

Furthermore, some compounds of formula (I) and some of the intermediates used in their preparation may exhibit polymorphism. It is to be understood that the present invention encompasses any polymorphic forms possessing properties useful in the treatment of the conditions noted hereinabove.

The pharmaceutically acceptable salts as mentioned hereinabove are meant to comprise the therapeutically active non-toxic acid addition salt forms that the compounds of formula (I) are able to form. These pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, trifluoromethanesulfonic, ethanesulfonic, benzenesulfonic, p-toluenesulfonic, cyclamic, salicylic, p-aminosalicylic, pamoic and the like.

Conversely said salt forms can be converted by treatment with an appropriate base into the free base form.

The compounds of formula (I) may exist in both unsolvated and solvated forms. The term 'solvate' is used

herein to describe a molecular association comprising a compound of the invention and one or more pharmaceutically acceptable solvent molecules, e.g. water or ethanol. The term 'hydrate' is used when said solvent is water.

As used herein, the term "about" is intended to qualify the numerical values which it modifies, denoting such a value as variable within a range. When no range, such as a margin of error or a standard deviation to a mean value given in a chart or table of data, is recited, the term "about" should be understood to mean the greater of the range which would encompass the recited value and the range which would be included by rounding up or down to that figure as well, considering significant figures, and the range which would encompass the recited value plus or minus 20%.

As used herein, the term "agonist" refers to a moiety that interacts with, and activates, a receptor and thereby initiates a physiological or pharmacological response characteristic of that receptor.

The term "disease" as used herein is intended to be generally synonymous, and is used interchangeably with, the terms "disorder," "syndrome," and "condition" (as in medical condition), in that all reflect an abnormal condition of the human or animal body or of one of its parts that impairs normal functioning, is typically manifested by distinguishing signs and symptoms, and causes the human or animal to have a reduced duration or quality of life.

The term "patient" is generally synonymous with the term "subject" and includes all mammals including humans. Examples of patients include humans, livestock such as cows, goats, sheep, pigs, and rabbits, and companion animals such as dogs, cats, rabbits, and horses. Preferably, the patient is a human.

The phrase "therapeutically effective" is intended to qualify the amount of active ingredients used in the treatment of a disease or disorder or on the effecting of a clinical endpoint.

The term "therapeutically acceptable" refers to those compounds (or salts, prodrugs, tautomers, zwitterionic forms, etc.) which are suitable for use in contact with the tissues of patients without undue toxicity, irritation, and allergic response, are commensurate with a reasonable benefit/risk ratio, and are effective for their intended use.

As used herein, "treating," "treatment," and the like means ameliorating a disease, so as to reduce, ameliorate, or eliminate its cause, its progression, its severity, or one or more of its symptoms, or otherwise beneficially alter the disease in a subject. In certain embodiments, reference to "treating" or "treatment" of a subject at risk for developing a disease, or at risk of disease progression to a worse state, is intended to include prophylaxis. Prevention of a disease may involve complete protection from disease, for example as in the case of prevention of infection with a pathogen, or may involve prevention of disease progression, for example from prediabetes to diabetes. For example, prevention of a disease may not mean complete foreclosure of any effect related to the diseases at any level, but instead may mean prevention of the symptoms of a disease to a clinically significant or detectable level. Prevention of diseases may also mean prevention of progression of a disease to a later stage of the disease.

**Pharmaceutical Compositions**

Additionally, pharmaceutical compositions/formulations comprising at least one pharmaceutically acceptable carrier and a therapeutically effective amount of a compound of formula (I) are provided herein.

In order to prepare the pharmaceutical compositions of the compounds provided herein, an effective amount of the particular compound, optionally in base or acid addition salt form, as the active ingredient, is combined with at least one pharmaceutically acceptable carrier, which carrier may take a variety of forms depending on the form of preparation desired for administration. These pharmaceutical compositions may be in unitary dosage form suitable for oral administration, rectal administration, percutaneous administration or parenteral injection.

For example in preparing the compositions in oral dosage form, any of the usual liquid pharmaceutical carriers may be employed, such as for instance water, glycols, oils, alcohols and the like in the case of oral liquid preparations such as suspensions, syrups, elixirs and solutions; or solid pharmaceutical carriers such as starches, sugars, kaolin, lubricants, binders, disintegrating agents and the like in the case of powders, pills, capsules and tablets. Because of their easy administration, tablets and capsules represent the most advantageous oral dosage unit form, in which case solid pharmaceutical carriers are obviously employed. For parenteral injection compositions, the pharmaceutical carrier will mainly comprise sterile water, although other ingredients may be included in order to improve solubility of the active ingredient.

Injectable solutions may be prepared for instance by using a pharmaceutical carrier comprising a saline solution, a glucose solution or a mixture of both. Injectable suspensions may also be prepared by using appropriate liquid carriers, suspending agents and the like. In compositions suitable for percutaneous administration, the pharmaceutical carrier may optionally comprise a penetration enhancing agent and/or a suitable wetting agent, optionally combined with minor proportions of suitable additives which do not cause a significant deleterious effect to the skin. Said additives may be selected in order to facilitate administration of the active ingredient to the skin and/or be helpful for preparing the desired compositions. These topical compositions may be administered in various ways, e.g., as a transdermal patch, a spot-on or an ointment. Addition salts of the compounds of formula (1), due to their increased water solubility over the corresponding base form, are obviously more suitable in the preparation of aqueous compositions.

It is especially advantageous to formulate the pharmaceutical compositions of the compounds disclosed herein

in dosage unit form for ease of administration and uniformity of dosage.

"Dosage unit form" as used herein refers to physically discrete units suitable as unitary dosages, each unit containing a predetermined amount of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

For oral administration, the pharmaceutical compositions of the compounds disclosed herein may take the form of solid dose forms, for example, tablets (both swallowable and chewable forms), capsules or gelcaps, prepared by conventional means with pharmaceutically acceptable excipients and carriers such as binding agents (e.g. pregelatinised maize starch, polyvinylpyrrolidone, hydroxypropylmethylcellulose and the like), fillers (e.g. lactose, microcrystalline cellulose, calcium phosphate and the like), lubricants (e.g. magnesium stearate, tale, silica and the like), disintegrating agents (e.g. potato starch, sodium starch glycollate and the like), wetting agents (e.g. sodium lauryl sulphate) and the like. Such tablets may also be coated by methods well known in the art.

Liquid preparations for oral administration may take the form of e.g. solutions, syrups or suspensions, or they may be formulated as a dry product for admixture with water and/or another suitable liquid carrier before use. Such liquid preparations may be prepared by conventional means, optionally with other pharmaceutically acceptable additives such as suspending agents (e.g. sorbitol syrup, methylcellulose, hydroxypropylmethylcellulose or hydrogenated edible fats), emulsifying agents (e.g. lecithin or acacia), non-aqueous carriers (e.g. almond oil, oily esters or ethyl alcohol), sweeteners, flavours, masking agents and preservatives (e.g. methyl or propyl p-hydroxybenzoates or sorbic acid).

Pharmaceutically acceptable sweeteners useful in the pharmaceutical compositions of the disclosure comprise preferably at least one intense sweetener such as aspartame, acesulfame potassium, sodium cyclamate, alitarne, dihydrochalcone sweetener, monellin, stevioside sucralose (4,1',6'-trichloro-4,1',6'-trideoxygalactosucrose) or, preferably, saccharin, sodium or calcium saccharin, and optionally at least one bulk sweetener such as sorbitol, mannitol, fructose, sucrose, maltose, isomalt, glucose, hydrogenated glucose syrup, xylitol, caramel or honey. Intense sweeteners are conveniently used in low concentrations. For example, in the case of sodium saccharin, the said concentration may range from about 0.04% to 0.1% (weight/volume) of the final formulation. The bulk sweetener can effectively be used in larger concentrations ranging from about 10% to about 35%, preferably from about 10% to 15% (weight/volume). The pharmaceutically acceptable flavours which can mask the bitter tasting ingredients in the low-dosage formulations comprise preferably fruit flavours such as cherry, raspberry, black currant or strawberry flavour. A combination of two flavours may yield very good results. In the high-dosage formulations, stronger pharmaceutically acceptable flavours may be required such as Caramel Chocolate, Mint Cool, Fantasy and the like.

Each flavour may be present in the final composition in a concentration ranging from about 0.05% to 1% (weight/volume). Combinations of said strong flavours are advantageously used. Preferably a flavour is used that does not undergo any change or loss of taste and/or color under the circumstances of the formulation.

The compounds of formula (I) may be formulated for parenteral administration by injection, conveniently intravenous, intra-muscular or subcutaneous injection, for example by bolus injection or continuous intravenous infusion. Formulations for injection may be presented in unit dosage form, e.g. in ampoules or multi-dose containers, including an added preservative. They may take such forms as suspensions, solutions or emulsions in oily or aqueous vehicles, and may contain formulating agents such as isotonizing, suspending, stabilizing and/or dispersing agents. Alternatively, the active ingredient may be present in powder form for mixing with a suitable vehicle, e.g. sterile pyrogen-free water, before use.

The compounds of formula (I) may also be formulated in rectal compositions such as suppositories or retention enemas, e.g. containing conventional suppository bases such as cocoa butter and/or other glycerides.

Those of skill in the treatment of diseases linked to the mediation of the ligand-gated ion channels will easily determine the therapeutically effective amount of a compound of formula (I) from the test results presented hereinafter. In general, it is contemplated that a therapeutically effective dose will be from about 0.001 mg/kg to about 50 mg/kg of body weight, more preferably from about 0.01 mg/kg to about 10 mg/kg of body weight of the patient to be treated. It may be appropriate to administer the therapeutically effective dose in the form of two or more sub-doses at appropriate intervals throughout the day. Said sub-doses may be formulated as unit dosage forms, for example each containing from about 0.1 mg to about 1000 mg, more particularly from about 1 to about 500 mg, of the active ingredient per unit dosage form.

As used herein, a "therapeutically effective amount" of a compound, is the quantity of a compound which, when administered to an individual or animal, results in a sufficiently high level of that compound in the individual or animal to cause a discernible GPR120 receptor modulating response.

The exact dosage and frequency of administration depends on the particular compound of formula (I) used, the particular condition being treated, the severity of the condition being treated, the age, weight and general physical condition of the particular patient as well as the other medication, the patient may be taking, as is well known to those skilled in the art. Furthermore, said "therapeutically effective amount" may be lowered or increased depending on the

response of the treated patient and/or depending on the evaluation of the physician prescribing the compounds of the instant invention. The effective daily amount ranges mentioned hereinabove are therefore only guidelines.

## Indications and Methods of Treatment

Also provided herein are methods for treating TRPML1-mediated disorders in a human or animal subject in need of such treatment comprising administering to said subject an amount of a compound disclosed herein effective to reduce or prevent said disorder in the subject, in combination with at least one additional agent for the treatment of said disorder that is known in the art. Certain embodiments provide therapeutic compositions comprising at least one compound disclosed herein in combination with one or more additional agents for the treatment of TRPML1-mediated disorders.

Also provided herein are: the use of a compound of formula (I) or a pharmaceutically acceptable salt thereof as a medicament; the use of a compound of formula (I) or a pharmaceutically acceptable salt thereof in, or in the treatment of, a TRPML1 mediated disease; the use of a compound of formula (I) or a pharmaceutically acceptable salt thereof in the manufacture of a medicament for the treatment of a TRPML1 mediated disease; and a method of treatment of TRPML1 mediated disease comprising administering a compound of formula (I) or a pharmaceutically acceptable salt thereof. Further provided herein is a method of treatment of a disease mediated by TRPML1 activity, in a mammalian subject, which comprising administering a therapeutically effective amount of a compound of formula (I) or a pharmaceutically acceptable salt thereof.

TRPML1-mediated diseases include proliferative disorders such as cancers, inflammatory disorders, pain, neurodegenerative disorders, cognitive and psychiatric disorders, and other diseases as disclosed below.

Compounds disclosed herein are useful for the treatment of neurodegenerative

disorders of various origins such as Alzheimer's disease and other dementia conditions such as Lewy body dementia, fronto-temporal dementia and other tauopathies; amyotrophic lateral sclerosis, multiple sclerosis, Parkinson's disease and other parkinsonian syndromes; HIV-induced neuroinflammation; essential tremors; other spinocerebellar degenerations, neuropathies such as Charcot-Marie-Tooth neuropathy and other TRPML1-mediated diseases such as Type IV mucolipidosis (MLIV). The compounds disclosed herein are also useful for the treatment of neurological conditions such as epilepsy including simple partial seizure, complex partial seizure, secondary generalized seizure, further including absence seizure, myoclonic seizure, clonic seizure, tonic seizure, tonic clonic seizure and atonic seizure, and for prevention and treatment of status epilepticus (SE).

The compounds disclosed herein are also useful for the treatment of cognitive disorders and of psychiatric disorders. Psychiatric disorders include, and are not limited to major depression, dysthymia, mania, bipolar disorder (such as bipolar disorder type I, bipolar disorder type II), cyclothymic disorder, rapid cycling, ultradian cycling, mania, hypomania, schizophrenia, schizophreniform disorders, schizoaffective disorders, personality disorders, attention disorders with or without hyperactive behaviour, delusional disorders, brief psychotic disorders, shared psychotic disorders, psychotic disorder due to a general medical condition, substance-induced psychotic disorders or a psychotic disorder not otherwise specified, anxiety disorders such as generalised anxiety disorder, panic disorders, posttraumatic stress disorder, impulse control disorders, phobic disorders, dissociative states and moreover in smoke, drug addiction and alcoholism. In particular bipolar disorders, psychosis, anxiety and addiction.

The compounds disclosed herein are useful in the prevention or treatment of neuroinflammation and CNS damage induced by HIV infection and of HIV-associated neurocognitive deficits. The compounds disclosed herein are useful in the prevention or treatment of neuropathic pain. Neuropathic pain syndromes include, and are not limited to: chemotherapy-induced peripheral neuropathy, diabetic neuropathy; sciatica; non-specific lower back pain; multiple sclerosis pain; fibromyalgia; HIV-related neuropathy; neuralgia, such as post-herpetic neuralgia and trigeminal neuralgia, Morton's neuralgia, causalgia; and pain resulting from physical trauma, amputation, phantom limb, cancer, toxins or chronic inflammatory conditions; central pain such as the one observed in thalamic syndromes, mixed central and peripheral forms of pain such as complex regional pain syndromes (CRPS) also called reflex sympathetic dystrophies.

The compounds disclosed herein are also useful for the treatment of pain, including chronic pain. Chronic pain includes, and is not limited to, chronic pain caused by inflammation or an inflammatory-related condition, osteoarthritis, rheumatoid arthritis, acute injury or trauma, upper back pain or lower back pain (resulting from systematic, regional or primary spine disease such as radiculopathy), bone pain (due to osteoarthritis, osteoporosis, bone metastasis or unknown reasons), pelvic pain, spinal cord injury-associated pain, cardiac chest pain, non-cardiac chest pain, central post-stroke pain, myofascial pain, sickle cell pain, cancer pain, Fabry's disease, AIDS pain, geriatric pain or pain caused by headache, temporomandibular joint syndrome, gout, fibrosis or thoracic outlet syndromes, in particular rheumatoid arthritis and osteoarthritis.

The compounds disclosed herein are also useful in the treatment of acute pain caused by acute injury, illness, sport-medicine injuries, carpal tunnel syndrome, burns, musculoskeletal sprains and strains, musculotendinous strain, cervicobrachial pain syndromes, dyspepsia, gastric ulcer, duodenal ulcer, dysmenorrhea, endometriosis or surgery (such

as open heart or bypass surgery), post-operative pain, kidney stone pain, gallbladder pain, gallstone pain, obstetric pain or dental pain.

The compounds disclosed herein are also useful in the treatment of headaches such as migraine, tension type headache, transformed migraine or evolutive headache, cluster headache, as well as secondary headache disorders, such as the ones derived from infections, metabolic disorders or other systemic illnesses and other acute headaches, paroxysmal hemicrania and the like, resulting from a worsening of the above mentioned primary and secondary headaches.

Compounds disclosed herein are also useful in the treatment of diseases such as vertigo, tinnitus, muscle spasm, and other disorders including and not limited to cardiovascular diseases (such as cardiac arrhythmia, cardiac infarction or angina pectoris, hypertension, cardiac ischemia, cerebral ischemia) endocrine disorders (such as acromegaly or diabetes insipidus) diseases in which the pathophysiology of the disorder involves excessive or hypersecretory or otherwise inappropriate cellular secretion of an endogenous substance (such as catecholamine, a hormone or a growth factor).

The compounds disclosed herein are also useful in the selective treatment of liver disease, such as inflammatory liver diseases, for example chronic viral hepatitis B, chronic viral hepatitis C, alcoholic liver injury, primary biliary cirrhosis, autoimmune hepatitis, liver fibrosis, non-alcoholic steatohepatitis and liver transplant rejection.

The compounds disclosed herein inhibit inflammatory processes affecting all body systems. Therefore, they are useful in the treatment of inflammatory processes of the muscular skeletal system of which the following is a list of examples but it is not comprehensive of all target disorders: arthritic conditions such as ankylosing spondylitis, cervical arthritis, fibromyalgia, gout, juvenile rheumatoid arthritis, lumbosacral arthritis, osteoarthritis, osteoporosis, psoriatic arthritis, rheumatic disease; disorders affecting skin and related tissues: eczema, psoriasis, dermatitis and inflammatory conditions such as sunburn; disorders of the respiratory system: asthma, allergic rhinitis and respiratory distress syndrome, lung disorders in which inflammation is involved such as asthma and bronchitis; chronic obstructive pulmonary disease; disorders of the immune and endocrinological systems: periarthritis nodosa, thyroiditis, aplastic anaemia, scleroderma, myasthenia gravis, multiple sclerosis and other demyelinating disorders, encephalomyelitis, sarcoidosis, nephritic syndrome, Bechet's syndrome, polymyositis, gingivitis.

Compounds disclosed herein are also useful in the treatment of gastrointestinal (GI) tract disorders such as inflammatory bowel disorders (IBD) including but not limited to ulcerative colitis, Crohn's disease, ileitis, proctitis, celiac disease, enteropathies, microscopic or collagenous colitis, eosinophilic gastroenteritis, or pouchitis resulting after proctocolectomy and post ileonatal anastomosis, and irritable bowel syndrome including any disorders associated with abdominal pain and/or abdominal discomfort such as pylorospasm, nervous indigestion, spastic colon, spastic colitis, spastic bowel, intestinal neurosis, functional colitis, mucous colitis, laxative colitis and functional dyspepsia; but also for treatment of atrophic gastritis, gastritis varialoforme, ulcerative colitis, peptic ulceration, pyrosis, and other damage to the GI tract, for example, by Helicobacter pylori, gastroesophageal reflux disease, gastroparesis, such as diabetic gastroparesis; and other functional bowel disorders, such as non-ulcerative dyspepsia (NUD); emesis, diarrhoea, and visceral inflammation.

Compounds disclosed herein are also useful in the treatment of disorders of the genito-urinary tract such as overactive bladder, prostatitis (chronic bacterial and chronic nonbacterial prostatitis), prostadynia, interstitial cystitis, urinary incontinence and benign prostatic hyperplasia, annexities, pelvic inflammation, bartholinities and vaginitis. In particular, overactive bladder and urinary incontinence.

Compounds disclosed herein are also useful in the treatment of renal disorders including diabetic nephropathy, renal allograft rejection, infectious renal diseases, IgA nephropathy, fibrotic kidney disease, lupus nephritis and glomerulonephritis, acute kidney injury and renal carcinoma.

The compounds disclosed herein are also useful in the treatment of ophthalmic diseases such as retinitis, retinopathies, uveitis and acute injury to the eye tissue, age-related macular degeneration or glaucoma, conjunctivitis.

The compounds disclosed herein are also useful in the treatment of eating disorders such as anorexia nervosa including the subtypes restricting type and binge-eating/purging type; bulimia nervosa including the subtypes purging type and non-purging type; obesity; compulsive eating disorders; binge eating disorder; and eating disorder not otherwise specified.

The compounds disclosed herein are also useful in the treatment of allergic dermatitis, hyper-responsiveness of the airway, chronic obstructive pulmonary disease (COPD), bronchitis, septic shock, Sjögren's syndrome, glomerulonephritis, atherosclerosis, growth and metastases of malignant cells, myoblastic leukaemia, diabetes, meningitis, osteoporosis, burn injury, ischaemic heart disease, stroke, peripheral vascular disease, varicose veins, glaucoma.

In some embodiments, the compounds and pharmaceutical compositions of the present disclosure may be useful in the treatment or prevention of progression of cancer. The cancer may be a hematologic malignancy or solid tumor. Hematologic malignancies include leukemias, lymphomas, multiple myeloma, and subtypes thereof. Lymphomas can be classified various ways, often based on the underlying type of malignant cell, including Hodgkin's lymphoma (often cancers of Reed-Sternberg cells, but also sometimes originating in B cells; all other lymphomas are non-Hodgkin's

lymphomas), B-cell lymphomas, T-cell lymphomas, mantle cell lymphomas, Burkitt's lymphoma, follicular lymphoma, and others as defined herein and known in the art.

B-cell lymphomas include, but are not limited to, diffuse large B-cell lymphoma (DLBCL), chronic lymphocytic leukemia (CLL) /small lymphocytic lymphoma (SLL), and others as defined herein and known in the art.

T-cell lymphomas include T-cell acute lymphoblastic leukemia/lymphoma (T-ALL), peripheral T-cell lymphoma (PTCL), T-cell chronic lymphocytic leukemia (T-CLL) Sezary syndrome, and others as defined herein and known in the art.

Leukemias include acute myeloid (or myelogenous) leukemia (AML), chronic myeloid (or myelogenous) leukemia (CML), acute lymphocytic (or lymphoblastic) leukemia (ALL), chronic lymphocytic leukemia (CLL) hairy cell leukemia (sometimes classified as a lymphoma) and others as defined herein and known in the art.

Plasma cell malignancies include lymphoplasmacytic lymphoma, plasmacytoma, and multiple myeloma.

Solid tumors include melanomas, neuroblastomas, gliomas or 5 carcinomas such as tumors of the brain, head and neck, breast, lung (e.g., non-small cell lung cancer, NSCLC), reproductive tract (e.g., ovary), upper digestive tract, pancreas, liver, renal system (e.g., kidneys), bladder, prostate and colorectum.

Besides being useful for human treatment, certain compounds and formulations disclosed herein may also be useful for veterinary treatment of companion animals, exotic animals and farm animals, including mammals, rodents, and the like. More preferred animals include horses, dogs, and cats.

**General Synthetic Methods Nomenclature and Structures**

In general, the nomenclature used in this Application is based on ChemSketch™ (ACDLabs) and generated according to the IUPAC systematic nomenclature. Chemical structures shown herein were prepared using ISIS® version 2.2. Certain compounds were drawn using CambridgeSoft's ChemDraw 18.0. Any open valency appearing on a carbon, oxygen, sulfur, or nitrogen atom in the structures herein indicates the presence of a hydrogen atom unless indicated otherwise. Where a nitrogen-containing heteroaryl ring is shown with an open valency on a nitrogen atom and variables such as $R^1$, $R^2$, $R^3$ etc. are shown on the heteroaryl ring, such variables may be bound or joined to the open valency nitrogen. Where a chiral center exists in a structure, but no specific stereochemistry is shown for the chiral center, both enantiomers associated with the chiral center are encompassed by the structure. Where a structure shown herein may exist in multiple tautomeric forms, all such tautomers are encompassed by the structure. The atoms represented in the structure herein are intended to encompass all naturally occurring isotopes of such atoms. Thus, for example, the hydrogen atoms represented herein are meant to include deuterium and tritium, and the carbon atoms are meant to include$^{13}C$ and $^{14}C$ isotopes.

**Abbreviations**

Abbreviations which are used in the description of the Schemes and the Examples that follows include:

ACN: Acetonitrile
AcOEt: Ethyl acetate
$CH_3I$: Iodomethane
DCM: Dichloromethane
DMF: Dimethylformamide
DMSO: Dimethylsulfoxide
ESI: Electrospray ionization
h: hour
HATU:1-[Bis(dimethylamino)methylene]-1H-1,2,3-triazolo[4,5-b]pyridinium-3-oxide hexafluorophosphate
$K_2CO_3$: Potassium carbonate
M: Molar
MeOH: Methanol
Min: Minute(s)
NMR: Nuclear Magnetic Resonance
NaH : Sodium hydride
NaOH: Sodium hydroxide
$Na_2SO_4$: Sodium sulfate
on: overnight
rt: Room Temperature
TFA: Trifluoroacetic acid
THF: Tetrahydrofuran

TEA: Triethylamine
UPLC-MS: UltraPerformance LiquidChromatography-Mass Spectrometry
y: yield or yields.

## Experimental part

The following examples illustrate the present invention. Unless explicitly stated otherwise, all measurements (especially percentages and amounts) relate to the weight.

## Synthesis of the intermediates

### Amines derivatives (II or III)

Most of amines derivatives (Compounds formula II or formula III) used, as starting materials, were purchased from chemical providers:

| **Structure of amines** | CAS number |
|---|---|
| 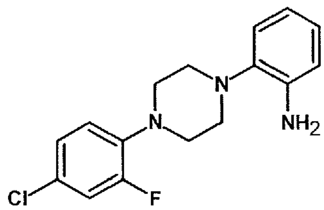 | 2006277-40-1 |
| 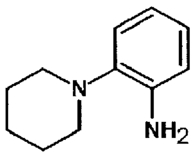 | 39643-31-7 |
| 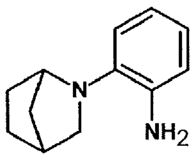 | 1250695-37-4 |
| 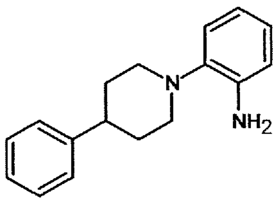 | 252758-95-5 |
| 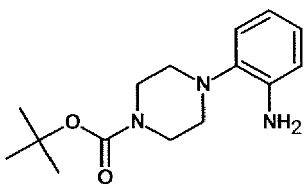 | 170017-74-0 |

(continued)

| **Structure of amines** | CAS number |
|---|---|
| 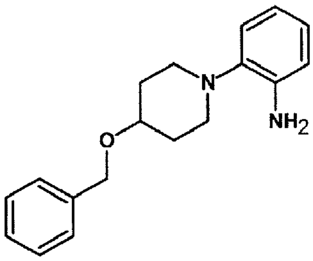 | 1018636-14-0 |
| 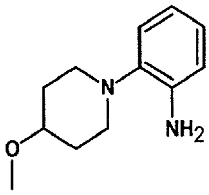 | 1018624-48-0 |
| 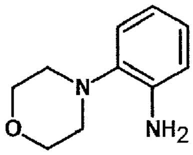 | 5585-33-1 |
| 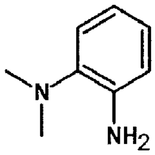 | 1262836-03-5 |
| 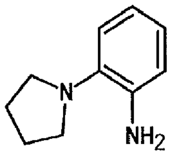 | 21627-58-7 |
| 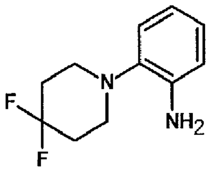 | 854044-39-6 |
| 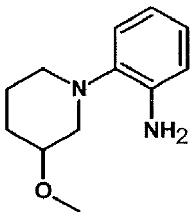 | 1602318-01-3 |

(continued)

| Structure of amines | CAS number |
|---|---|
| 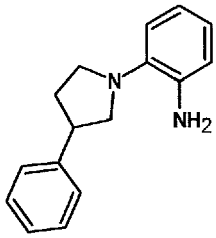 | 1272785-78-0 |
| 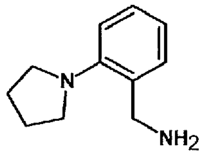 | 72752-53-5 |
| 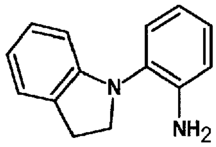 | 180629-70-3 |
| 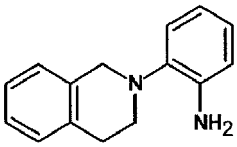 | 246247-91-6 |
| 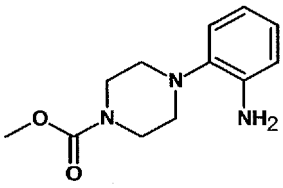 | 1156634-85-3 |
| 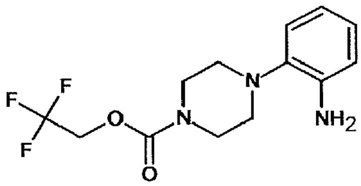 | 2173422-77-8 |
| 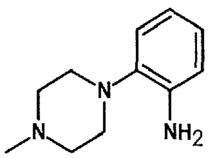 | 180605-36-1 |
| 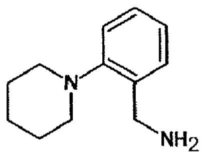 | 72752-54-6 |

(continued)

| Structure of amines | CAS number |
|---|---|
| 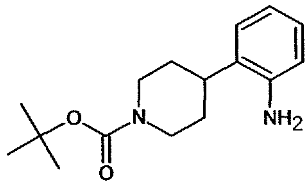 | 199105-39-7 |
| 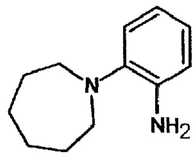 | 51627-46-4 |

In the other cases, the amines derivatives (compounds of formula II) were synthesized according with the followed synthetic scheme:

***Scheme 1***

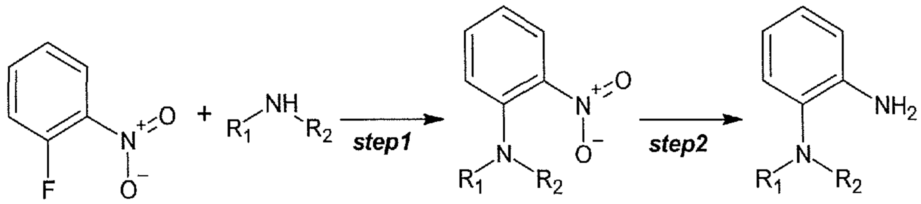


1 2 3 (II)

**Step 1.** To a solution of commercially available cmpd 1 (1.0 eq) in ACN (10 mL) cooled to 0°C, $K_2CO_3$ (3.0 eq) was added, followed by the addition of a suitable compound **2** (1.1 eq). The reaction mixture was stirred at rt on. Then the inorganic salts were filtered off and washed several times with THF. The filtrate was concentrated under reduced pressure. The crude residue was purified by flash chromatography on silica gel using Hexane /AcOEt 9:1 v/v as the eluent. In this way, the pure desired product **3** was obtained.

**Step 2.** To a solution of cmpd 3 (1.0 eq) in MeOH (30 mL) a solution of ammonium chloride (6.0 eq) in water (7.5 mL) and zinc (5.0 eq) were added sequentially; the mixture was stirred at rt on. The inorganic salts were filtered off and washed several times with THF. The organic layers were concentrated under reduced pressure. The residue was diluted with 2N NaOH and extracted with AcOEt, the combined organic layers were dried over $Na_2SO_4$ and concentrated under reduced pressure. The crude was used without purification in the next step. In this way, compounds II were obtained.

Using these procedures, the following compounds II were synthesized:

| Intermediate | Structure of amines | Yield |
|---|---|---|
| IIa | 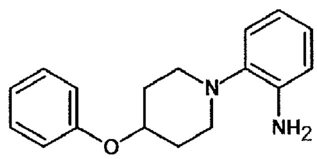 | 85% |
| IIb | 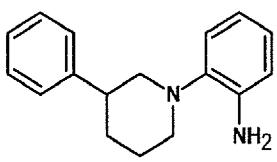 | 94% |

(continued)

| Intermediate | Structure of amines | Yield |
|---|---|---|
| IIc | 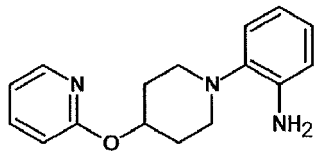 | 80% |
| IId | 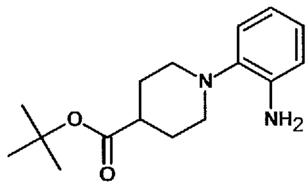 | 97% |
| IIe | 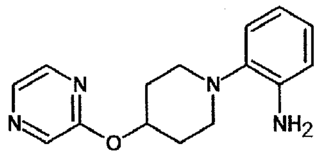 | 75% |
| IIf | 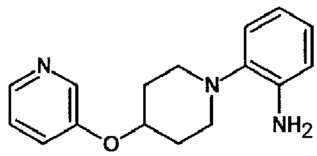 | 81% |
| IIg | 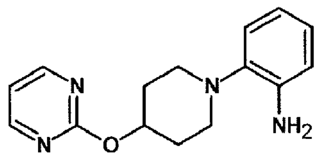 | 74% |
| IIh | 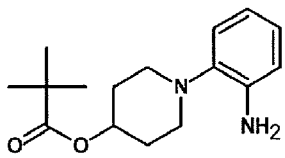 | 90% |

**Sulphonyl chlorides (IV)**

All the sulphonyl chlorides (Compounds formula IV) used as starting materials were purchased from chemical providers:

| Structure of sulphonyl chlorides | CAS number |
|---|---|
| 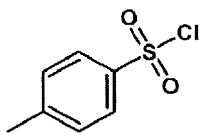 | 98-59-9 |
| 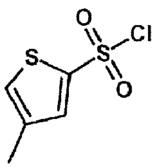 | 69815-97-0 |

(continued)

| Structure of sulphonyl chlorides | CAS number |
|---|---|
| 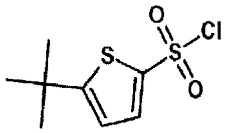 | 179400-17-0 |
| 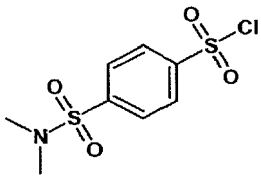 | 677782-39-7 |
| 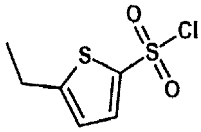 | 56921-00-7 |
| 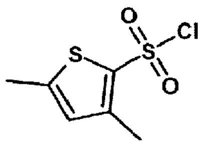 | 1314938-98-1 |
| 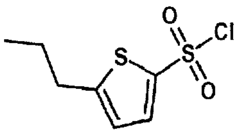 | 883156-08-5 |
| 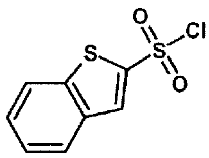 | 90001-64-2 |
| 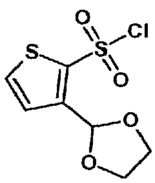 | 103011-38-7 |
| 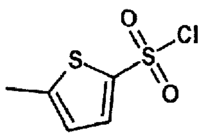 | 55854-45-0 |
| 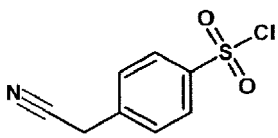 | 28338-22-9 |

(continued)

| Structure of sulphonyl chlorides | CAS number |
|---|---|
| 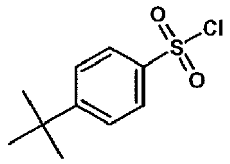 | 15084-51-2 |
| 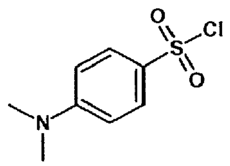 | 19715-49-2 |
| 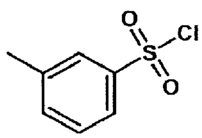 | 1899-93-2 |
| 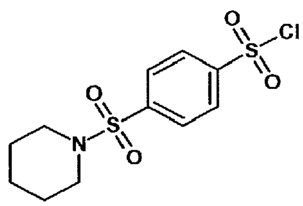 | 921090-59-7 |
| 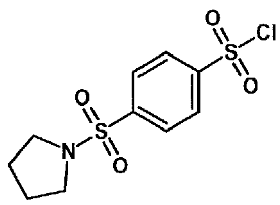 | 165669-32-9 |
| 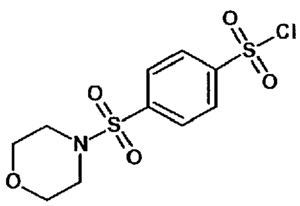 | 465514-13-0 |
| 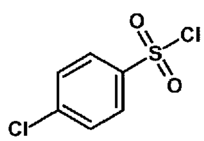 | 98-60-2 |
| 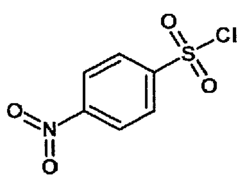 | 98-74-8 |

(continued)

| **Structure of sulphonyl chlorides** | CAS number |
|---|---|
| 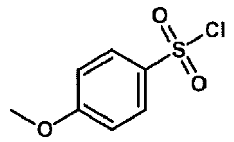 | 98-68-0 |

**Acyl chlorides (V)**

All the acyl chlorides (compounds of formula V) used as starting materials were purchased from chemical providers:

| **Structure of acyl chlorides** | CAS number |
|---|---|
| 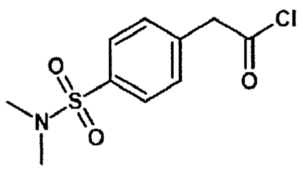 | 1017472-14-8 |
| 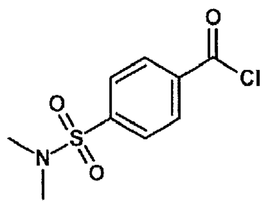 | 29171-70-8 |

**Preparation of intermediates of formula (VI)**

All the intermediates of formula VI were prepared according to the following synthetic scheme:

***Scheme 2***

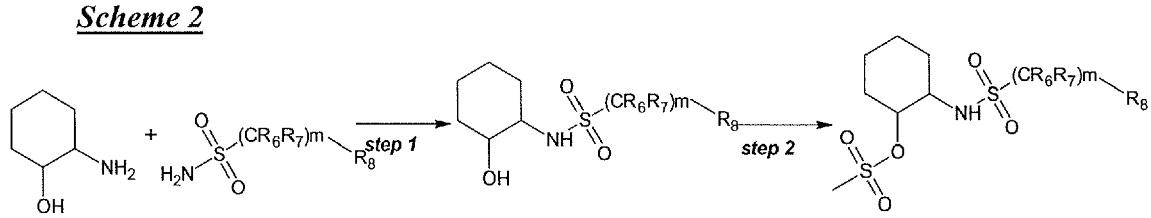


(4) (IV) (5) (VI)

**Step 1.** To a solution of a suitable commercially available cmpd **4** (1.0 eq) in DCM (15 mL) cmpd formula IV (1.1 eq) and then TEA (3.0 eq) were added dropwise. The reaction was stirred at rt on. Then the reaction was partitioned with DCM and water, the organic layer was washed with brine, dried over $Na_2SO_4$ anhydrous, filtered and evaporated under reduced pressure. The crude residue was purified by flash chromatography on silica gel using Hexane /AcOEt 80:20 v/v as the eluent. In this way, the pure desired product **5** were obtained.

**Step 2.** To a cold (0° C) solution of intermediate **5** (1.0 mL) in DCM (15.0 mL) methanesulfonyl chloride (1.2 eq) and then TEA (3.0 eq) were added. The reaction was stirred at rt for 2 hrs. Then the reaction was partitioned with DCM and water, the organic layer was washed with brine, dried over $Na_2SO_4$ anhydrous, filtered and evaporated under reduced pressure. The crude residue was used without purification in the next step. In this way the desired product VI was obtained.

Using the above procedure compound VIa was synthesized:

| Intermediate | Structure | Yield |
|---|---|---|
| VIa | 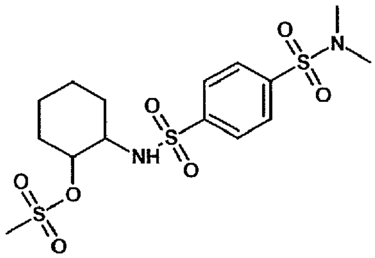 | 90% |

**Preparation of intermediates of formula (IX)**

All the intermediates of formula IX were prepared according to the following synthetic scheme:

*Scheme 3*

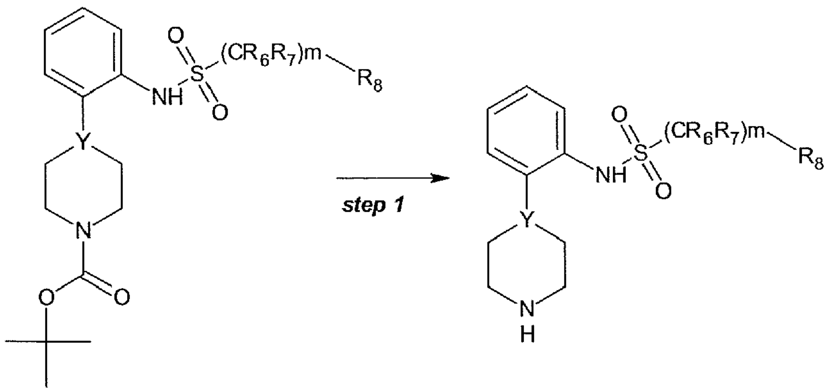


(XI) (IX)

To a solution of a suitable compound of formula XI (1.0 eq) in DCM (5.0 mL), TFA (5.0 eq) was added and the reaction was stirred at rt for 2 hrs. Then the solvent was evaporated under reduced pressure, and the residue was taken up in DCM. The crude was used without purification in the next step.

Using these procedures compound IXa was synthesized:

| Intermediate | Structure | Yield |
|---|---|---|
| IXa | 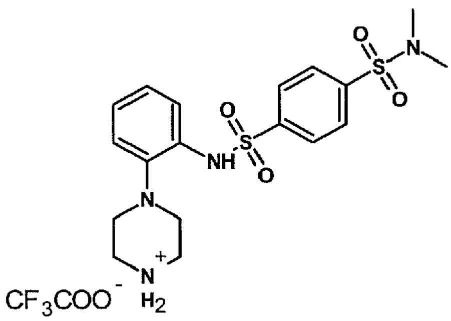 | 99% |

**General procedures for the synthesis of final compounds (compounds of formula I)**

**Preparation of intermediates of formula (XII)**

All the intermediates of formula XII were prepared according to the following synthetic scheme:

*Scheme 4*

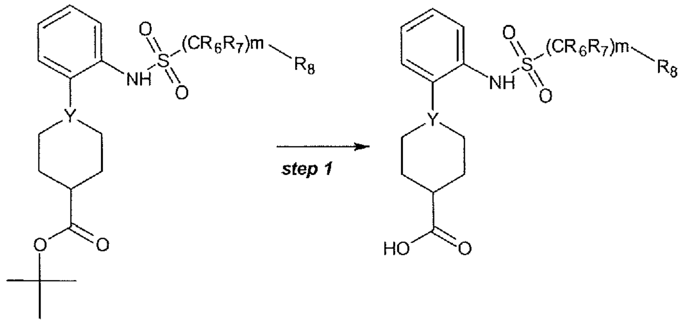


(6) (XII)

To a solution of a suitable compound of formula 6 (1.0 eq) in DCM (5.0 mL), TFA (5.0 eq) was added and the reaction was stirred at rt for 2 hrs. Then the solvent was evaporated under reduced pressure, and the residue was taken up in DCM. The crude was used without purification in the next step.

Using these procedures compound XIIa was synthesized:

| Intermediate | Structure | Yield |
|---|---|---|
| XIIa | 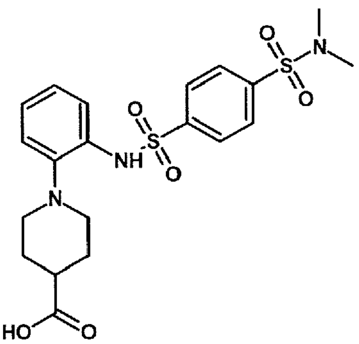 | 99% |

**Method A.**

To a cold (0° C) solution of a suitable compound of formula II or formula III (1.0 eq) in DCM a commercially available sulphonyl chloride (1.2 eq) or acyl chloride was added while maintaining the temperature at 0° C. Then, TEA (2.0 eq) or pyridine (2.0 eq) was added. The reaction was magnetically stirred at rt for 2 hours. The reaction mixture was transferred into a separating funnel and was washed with water, a saturated solution of $NaHCO_3$ and brine. The organic layer was dried over anhydrous $Na_2SO_4$, filtered and evaporated under reduced pressure. The crude was purified by flash chromatography on silica gel using Hexane/AcOEt as the eluent, giving the pure desired compound. (y: 9% - 98%)

**Method B.**

To a cold (0° C) solution of compound of formula VI (1.0 eq) in DCM a suitable commercially available amines (1.2 eq) and then pyridine (3.0 eq) were added dropwise. The reaction was stirred at rt on. Then the reaction was partitioned with DCM and water, the organic layer was washed with brine, dried over $Na_2SO_4$ anhydrous, filtered and evaporated under reduced pressure. The crude residue was purified by flash chromatography on silica gel using AcOEt /Hexane as the eluent, giving the pure desired compound. (y: 8% - 93%)

**Method C.**

To a solution of compound of formula I (1.0 eq) in DMF $K_2CO_3$ (5.0 eq) was added followed by $CH_3I$ (2.0 eq). The reaction was magnetically stirred at rt on. Then the solvent was removed, and water was added. The resulting mixture was extracted with EtOAc. The organic layers were dried over $Na_2SO_4$ anhydrous, filtered and evaporated under reduced pressure. The crude was purified by flash chromatography on silica gel using Hexane/AcOEt 1:1 as the eluent, giving the pure desired compound. (y: 56%)

**Method D.**

To a cold (0° C) solution of compound of formula I (1.0 eq) in THF, NaH (1.5 eq). was added portion wise. Then $CH_3I$ (1.1 eq) was added. The reaction was magnetically stirred at rt for 2 hrs. The reaction mixture was poured into ice cold water and extracted three times with DCM. The organic layer was dried over $Na_2SO_4$ anhydrous, filtered and evaporated under reduced pressure. The crude was purified by flash chromatography on silica gel using Hexane/AcOEt as the eluent, giving the pure desired compound. (y: 54% - 90%)

**Method E.**

To a cold (0° C) solution of compound of formula IX (1.0 eq) in DCM, a commercially available acyl chloride (1.1 eq.) and pyridine (1.5 eq.) were added dropwise. The reaction mixture was stirred at rt on. Then water was added, and the reaction mixture was extracted with DCM. The organic layer was washed with brine, dried over $Na_2SO_4$ anhydrous, filtered and evaporate under reduced pressure. The crude was purified by flash chromatography on silica gel using Hexane/AcOEt as the eluent, giving the pure desired compound. (y: 15% - 92%).

**Method F.**

A mixture of compound of formula XII (1.0 eq) and HATU (1.2 eq) in DCM was stirred at rt for 10 min. Then, the opportune commercially available amine (1.2 eq) and TEA (3.0 eq) were added, and the resulting mixture was stirred at rt on. The solvent was removed *in vacuo.* The crude was purified by flash chromatography on silica gel using Hexane/AcOEt as the eluent, giving the pure desired compound. (y: 90% - 98%)

**Examples**

The invention is further illustrated by the following examples.

**Example 1**

The title compound was prepared by the general method A, starting from commercially available amine CAS: 39643-31-7 and sulphonyl chloride CAS: 98-59-9, using pyridine as the base. (y = 77%).

**Example 2**

The title compound was prepared by the general method A, starting from commercially available amine CAS: 39643-31-7 and sulphonyl chloride CAS: 69815-97-0, using TEA as the base. (y = 67%).

**Example 3**

The title compound was prepared by the general method A, starting from commercially available amine CAS: 39643-31-7 and sulphonyl chloride CAS: 179400-17-0, using TEA as the base. (y = 14%).

**Example 4**

The title compound was prepared by the general method A, starting from commercially available amine CAS: 1250695-37-4 and sulphonyl chloride CAS: 677782-39-7, using TEA as the base. (y = 11%).

**Example 5**

The title compound was prepared by the general method A, starting from commercially available amine CAS:

1250695-37-4 and sulphonyl chloride CAS: 98-59-9, using TEA as the base. (y = 53%).

**Example 6**

The title compound was prepared by the general method A, starting from commercially available amine CAS: 39643-31-7 and sulphonyl chloride CAS: 677782-39-7, using TEA as the base. (y = 69%).

**Example 7**

The title compound was prepared by the general method A, starting from commercially available amine CAS: 39643-31-7 and sulphonyl chloride CAS: 56921-00-7, using TEA as the base. (y = 49%).

**Example 8**

The title compound was prepared by the general method A, starting from commercially available amine CAS: 39643-31-7 and sulphonyl chloride CAS: 1314938-98-1, using TEA as the base. (y = 13%).

**Example 9**

The title compound was prepared by the general method A, starting from commercially available amine CAS: 39643-31-7 and sulphonyl chloride CAS: 883146-08-5, using TEA as the base. (y = 44%).

**Example 10**

The title compound was prepared by the general method A, starting from commercially available amine CAS: 39643-31-7 and sulphonyl chloride CAS: 90001-64-2, using TEA as the base. (y = 63%).

**Example 11**

The title compound was prepared by the general method A, starting from commercially available amine CAS: 39643-31-7 and sulphonyl chloride CAS: 103011-38-7, using TEA as the base. (y = 46%).

**Example 12**

The title compound was prepared by the general method A, starting from commercially available amine CAS: 252758-95-5 and sulphonyl chloride CAS: 677782-39-7, using TEA as the base. (y = 60%).

**Example 13**

The title compound was prepared by the general method A, starting from commercially available amine CAS: 170017-74-0 and sulphonyl chloride CAS: 677782-39-7, using PYRIDINE as the base. (y = 96%).

**Example 14**

The title compound was prepared by the general method A, starting from commercially available amine CAS: 2006277-40-1 and sulphonyl chloride CAS: 179400-17-0, using TEA as the base. (y = 12%).

**Example 15**

The title compound was prepared by the general method A, starting from commercially available amine CAS: 1250695-37-4 and sulphonyl chloride CAS: 55854-45-0, using TEA as the base. (y = 21%).

**Example 16**

The title compound was prepared by the general method A, starting from commercially available amine CAS: 1250695-37-4 and sulphonyl chloride CAS: 179400-17-0, using PYRIDINE as the base. (y = 72%).

**Example 17**

The title compound was prepared by the general method A, starting from commercially available amine CAS: 1250695-37-4 and sulphonyl chloride CAS: 56921-00-7, using PYRIDINE as the base. (y = 62%).

**Example 18**

The title compound was prepared by the general method A, starting from commercially available amine CAS: 1250695-37-4 and acyl chloride CAS: 29171-70-8, using TEA as the base. (y = 85%).

**Example 19**

The title compound was prepared by the general method A, starting from commercially available amine CAS: 39643-31-7 and acyl chloride CAS: 29171-70-8, using TEA as the base. (y = 81%).

**Example 20**

The title compound was prepared by the general method A, starting from commercially available amine CAS: 39643-31-7 and sulphonyl chloride CAS: 28338-22-9, using PYRIDINE as the base. (y = 75%).

**Example 21**

The title compound was prepared by the general method A, starting from commercially available amine CAS: 39643-31-7 and sulphonyl chloride CAS: 15084-51-2, using PYRIDINE as the base. (y = 71%).

**Example 22**

The title compound was prepared by the general method A, starting from commercially available amine CAS: 39643-31-7 and sulphonyl chloride CAS: 19715-49-2, using PYRIDINE as the base. (y = 89%).

**Example 23**

The title compound was prepared by the general method A, starting from commercially available amine CAS: 39643-31-7 and sulphonyl chloride CAS: 1899-93-0, using PYRIDINE as the base. (y = 75%).

**Example 24**

The title compound was prepared by the general method A, starting from intermediate IIa and sulphonyl chloride CAS: 677782-39-7, using pyridine as the base. (y = 9%).

**Example 25**

The title compound was prepared by the general method A, starting from commercially available amine CAS: 1018636-14-0 and sulphonyl chloride CAS: 677782-39-7, using TEA as the base. (y = 13%).

**Example 26**

The title compound was prepared by the general method A, starting from commercially available amine CAS: 1018624-48-0 and sulphonyl chloride CAS: 677782-39-7, using PYRIDINE as the base. (y = 90%).

**Example 27**

The title compound was prepared by the general method A, starting from commercially available amine CAS: 5585-33-1 and sulphonyl chloride CAS: 677782-39-7, using TEA as the base. (y = 39%).

**Example 28**

The title compound was prepared by the general method A, starting from commercially available amine CAS:

2836-03-5 and sulphonyl chloride CAS: 677782-39-7, using TEA as the base. (y = 23%).

**Example 29**

The title compound was prepared by the general method A, starting from commercially available amine CAS: 39643-31-7 and sulphonyl chloride CAS: 921090-59-7, using PYRIDINE as the base. (y = 77%).

**Example 30**

The title compound was prepared by the general method A, starting from commercially available amine CAS: 39643-31-7 and sulphonyl chloride CAS: 165669-32-9, using PYRIDINE as the base. (y = 75%).

**Example 31**

The title compound was prepared by the general method A, starting from commercially available amine CAS: 39643-31-7 and sulphonyl chloride CAS: 465514-13-0, using PYRIDINE as the base. (y = 84%).

**Example 32**

The title compound was prepared by the general method A, starting from commercially available amine CAS: 21627-58-7 and sulphonyl chloride CAS: 677782-39-7, using TEA as the base. (y = 13%).

**Example 33**

The title compound was prepared by the general method A, starting from commercially available amine CAS: 854044-39-6 and sulphonyl chloride CAS: 677782-39-7, using PYRIDINE as the base. (y = 65%).

**Example 34**

The title compound was prepared by the general method A, starting from commercially available amine CAS: 21627-58-7 and acyl chloride CAS: 1017472-14-8, using TEA as the base. (y = 87%).

**Example 35**

The title compound was prepared by the general method A, starting from intermediate IIb and sulphonyl chloride CAS: 677782-39-7, using PYRIDINE as the base. (y = 90%).

**Example 36**

The title compound was prepared by the general method A, starting from commercially available amine CAS: 1602318-01-3 and sulphonyl chloride CAS: 677782-39-7, using pyridine as the base. (y = 91%).

**Example 37**

The title compound was prepared by the general method A, starting from commercially available amine CAS: 1272785-78-0 and sulphonyl chloride CAS: 677782-39-7, using PYRIDINE as the base. (y = 74%).

**Example 38**

The title compound was prepared by the general method A, starting from commercially available amine CAS: 2006277-40-1 and acyl chloride CAS: 29171-70-8, using TEA as the base. (y = 28%).

**Example 39**

The title compound was prepared by the general method A, starting from commercially available amine CAS: 72752-53-5 and sulphonyl chloride CAS: 677782-39-7, using PYRIDINE as the base. (y = 45%).

**Example 40**

The title compound was prepared by the general method A, starting from commercially available amine CAS: 180629-70-3 and sulphonyl chloride CAS: 677782-39-7, using TEA as the base. (y = 41%).

**Example 41**

The title compound was prepared by the general method A, starting from commercially available amine CAS: 246247-91-6 and sulphonyl chloride CAS: 677782-39-7, using TEA as the base. (y =47%).

**Example 42**

The title compound was prepared by the general method A, starting from commercially available amine CAS: 180605-36-1 and sulphonyl chloride CAS: 677782-39-7, using TEA as the base. (y = 21%).

**Example 43**

The title compound was prepared by the general method A, starting from commercially available amine CAS: 1250695-37-4 and acyl chloride CAS: 1017472-14-8, using TEA as the base. (y = 23%).

**Example 44**

The title compound was prepared by the general method E, starting from intermediate IXa and commercially available acyl chloride CAS: 75-36-5. (y = 15%).

**Example 45**

The title compound was prepared by the general method A, starting from commercially available amine CAS: 39643-31-7 and acyl chloride CAS: 1017472-14-8, using TEA as the base. (y = 98%).

**Example** 47

The title compound was prepared by the general method A, starting from commercially available amine CAS: 2006277-40-1 and acyl chloride CAS: 1017472-14-8, using TEA as the base. (y = 18%).

Example 48

The title compound was prepared by the general method D, starting from Example 38. (y =67%).

**Example 49**

The title compound was prepared by the general method D, starting from Example 18. (y =67%).

**Example 50**

The title compound was prepared by the general method D, starting from Example 19. (y =54%).

**Example 51**

The title compound was prepared by the general method C, starting from Example 17. (y =56%).

**Example 52**

The title compound was prepared by the general method E, starting from intermediate IXa and commercially available acyl chloride CAS: 79-22-1. (y = 33%).

**Example 53**

The title compound was prepared by the general method E, starting from intermediate IXa and commercially available acyl chloride CAS: 27746-99-2. (y = 40%).

**Example 54**

The title compound was prepared by the general method B, starting from intermediate VIa and commercially available amine CAS: 35386-24-4. (y = 41%).

**Example 55**

The title compound was prepared by the general method B, starting from intermediate VIa and commercially available amine CAS: 771-99-3. (y = 33%).

**Example 56**

The title compound was prepared by the general method B, starting from intermediate VIa and commercially available amine CAS: 92-54-6. (y = 93%).

**Example 57**

The title compound was prepared by the general method B, starting from intermediate VIa and commercially available amine CAS: 3202-33-3. (y = 49%).

**Example 58**

The title compound was prepared by the general method B, starting from intermediate VIa and commercially available amine CAS: 110-89-4. (y = 87%).

**Example 59**

The title compound was prepared by the general method B, starting from intermediate VIa and commercially available amine CAS: 515160-75-5. (y = 8%).

**Example 61**

The title compound was prepared by the general method B, starting from intermediate VIa and commercially available amine CAS: 110-91-8. (y = 65%).

**Example 62 and Example 63**

The title compounds, were obtained by enantiomeric separation of the racemate of **Example 57;** for separation procedures, see analytical methods.

**Example 64**

The title compound was prepared by the general method B, starting from intermediate VIa and commercially available amine CAS: 496-15-1. (y = 76%).

**Example 65**

The title compound was prepared by the general method A, starting from commercially available amine CAS: 39643-31-7 and sulphonyl chloride CAS: 98-60-2, using TEA as the base. (y = 38%).

**Example 66**

The title compound was prepared by the general method A, starting from commercially available amine CAS:

21627-58-7 and acyl chloride CAS: 29171-70-8, using TEA as the base. (y = 93%).

**Example 67**

The title compound was prepared by the general method D, starting from Example 66. (y =90%).

**Example 68**

The title compound was prepared by the general method A, starting from commercially available amine CAS: 39643-31-7 and sulphonyl chloride CAS: 98-74-8, using TEA as the base. (y = 39%).

**Example 69**

The title compound was prepared by the general method A, starting from commercially available amine CAS: 39643-31-7 and sulphonyl chloride CAS: 98-68-0, using TEA as the base. (y = 39%).

**Example 70**

The title compound was prepared by the general method E, starting from intermediate IXa and commercially available acyl chloride CAS: 541-41-3. (y = 68%).

**Example 71**

The title compound was prepared by the general method E, starting from intermediate IXa and commercially available acyl chloride CAS: 79-03-8. (y = 75%).

**Example 72**

The title compound was prepared by the general method E, starting from intermediate IXa and commercially available acyl chloride CAS: 4244-59-1. (y = 80%).

**Example 73**

The title compound was prepared by the general method E, starting from intermediate IXa and commercially available acyl chloride CAS: 98-88-4. (y = 92%).

**Example 74**

The title compound was prepared by the general method A, starting from commercially available amine CAS: 72752-54-6 and sulphonyl chloride CAS: 677782-39-7, using pyridine as the base. (y = 83%).

**Example 75**

The title compound was prepared by the general method A, starting from intermediate IIc and sulphonyl chloride CAS: 677782-39-7, using PYRIDINE as the base. (y = 98%).

**Example 76**

The title compound was prepared by the general method A, starting from intermediate IId and sulphonyl chloride CAS: 677782-39-7, using PYRIDINE as the base. (y = 80%).

**Example 77**

The title compound was prepared by the general method A, starting from intermediate IIe and sulphonyl chloride CAS: 677782-39-7, using PYRIDINE as the base. (y = 96%).

**Example 78**

The title compound was prepared by the general method A, starting from commercially available amine CAS: 199105-03-8 and sulphonyl chloride CAS: 677782-39-7, using PYRIDINE as the base. (y = 80%).

**Example 79**

The title compound was prepared by the general method E, starting from intermediate IXa and commercially available acyl chloride CAS: 5271-67-0. (y = 66%).

**Example 80**

The title compound was prepared by the general method E, starting from intermediate IXa and commercially available acyl chloride CAS: 54237-09-1. (y = 59%).

**Example 81**

The title compound was prepared by the general method E, starting from intermediate IXa and commercially available acyl chloride CAS: 39098-97-0. (y = 55%).

**Example 82**

The title compound was prepared by the general method E, starting from intermediate IXa and commercially available acyl chloride CAS: 51493-02-8. (y = 45%).

**Example 83**

The title compound was prepared by the general method A, starting from commercially available amine CAS: 170017-74-0 and sulphonyl chloride CAS: 98-59-9, using pyridine as the base. (y = 55%).

**Example 84**

The title compound was prepared by the general method A, starting from commercially available amine CAS: 170017-74-0 and sulphonyl chloride CAS: 98-68-0, using pyridine as the base. (y = 61%).

**Example 85**

The title compound was prepared by the general method A, starting from commercially available amine CAS: 51627-46-4 and sulphonyl chloride CAS: 677782-39-7, using pyridine as the base. (y = 40%).

**Example 86**

The title compound was prepared by the general method A, starting from intermediate IIf and sulphonyl chloride CAS: 677782-39-7, using pyridine as the base. (y = 44%).

**Example 87**

The title compound was prepared by the general method F, starting from intermediate XIIa and commercially available amine CAS: 107-10-8. (y = 98%).

**Example 88**

The title compound was prepared by the general method F, starting from intermediate XIIa and commercially available amine CAS: 78-81-9. (y = 98%).

**Example 89**

The title compound was prepared by the general method A, starting from intermediate IIg and sulphonyl chloride

CAS: 677782-39-7, using PYRIDINE as the base. (y = 97%).

**Example 90**

The title compound was prepared by the general method A, starting from intermediate IIh and sulphonyl chloride CAS: 98-59-9, using PYRIDINE as the base. (y = 98%).

**Example 91**

The title compound was prepared by the general method A, starting from intermediate IIh and sulphonyl chloride CAS: 98-68-0, using PYRIDINE as the base. (y = 98%).

**Table 2** lists final compounds that were prepared according to the experimental procedures described before.

**Table 2**

| Example | Structure |
|---|---|
| 1 | 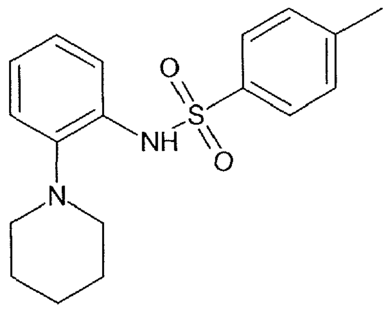 |
| 2 | 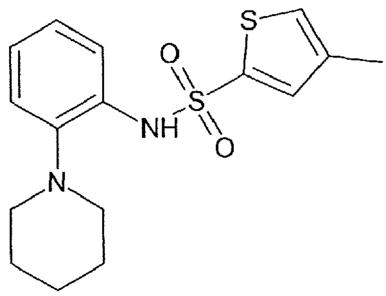 |
| 3 | 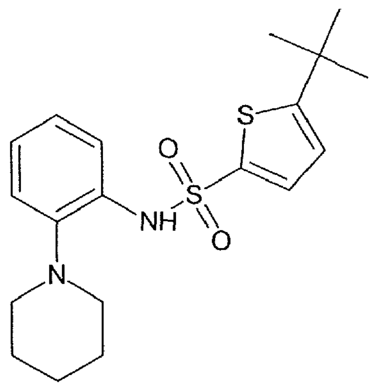 |

(continued)

| Example | Structure |
|---|---|
| 4 | 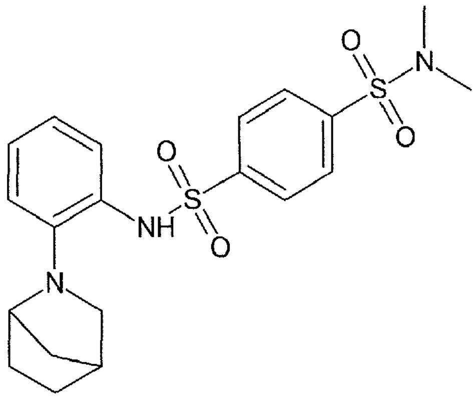 |
| 5 | 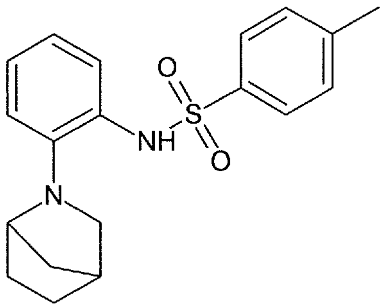 |
| 6 | 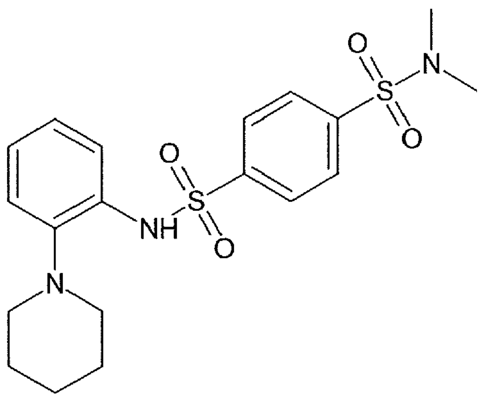 |
| 7 | 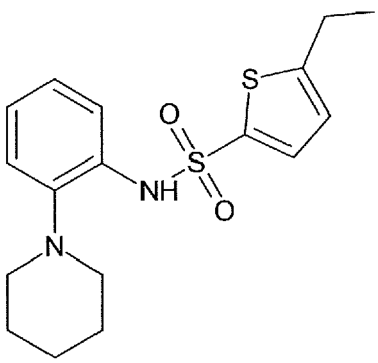 |

(continued)

| Example | Structure |
|---|---|
| 8 | 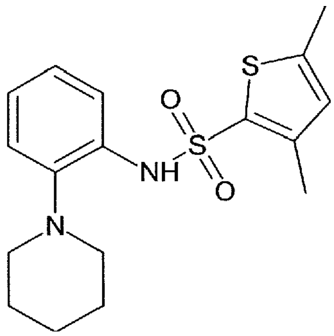 |
| 9 | 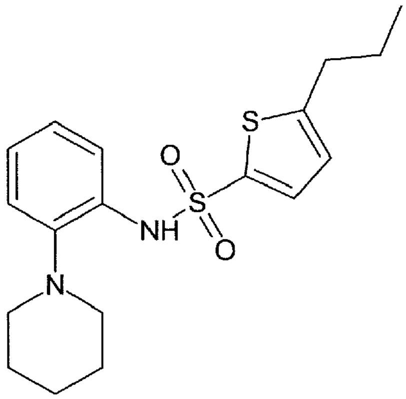 |
| 10 | 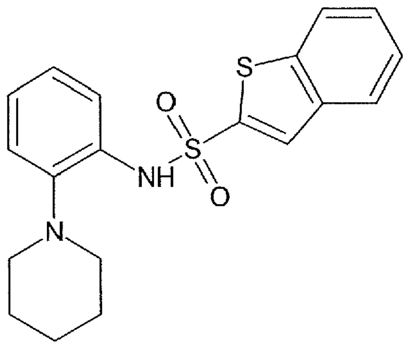 |
| 11 | 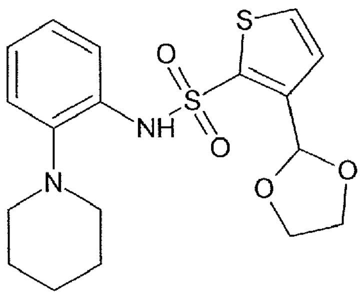 |

(continued)

| Example | Structure |
|---|---|
| 12 | 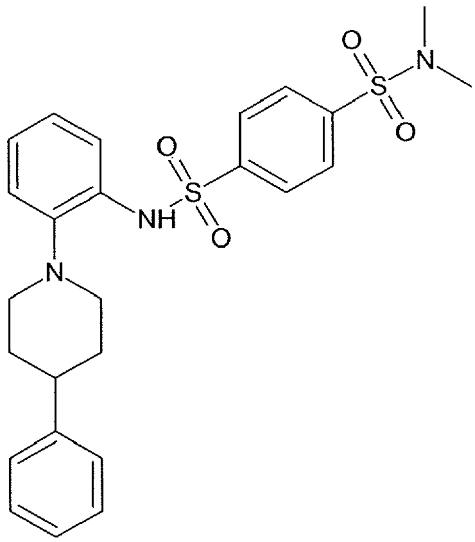 |
| 13 | 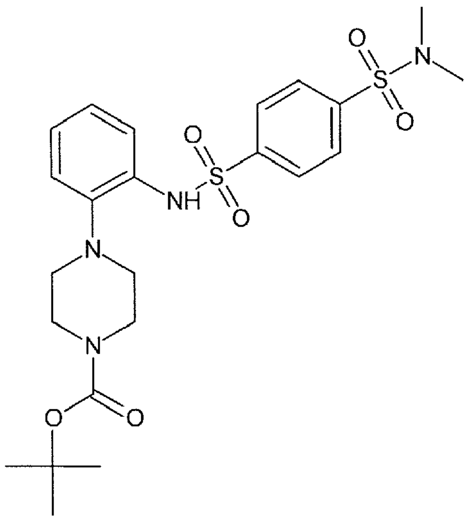 |

(continued)

| Example | Structure |
|---|---|
| 14 | 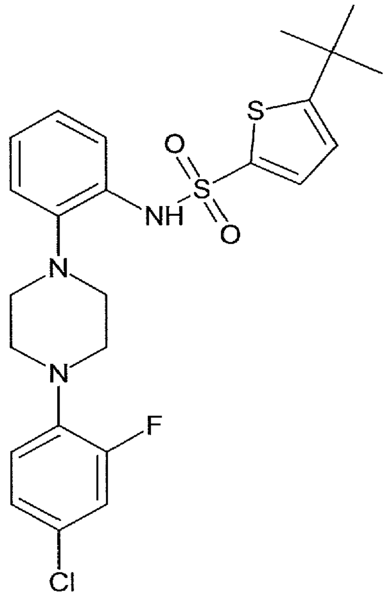 |
| 15 | 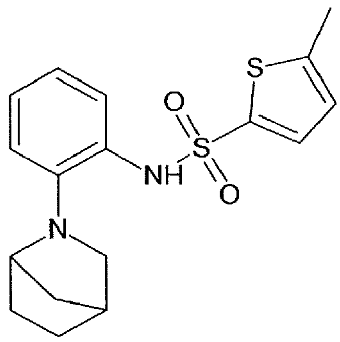 |
| 16 | 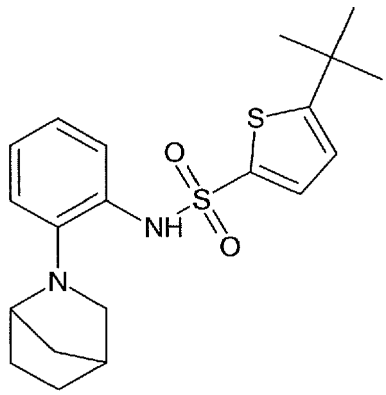 |

(continued)

| Example | Structure |
|---|---|
| 17 | 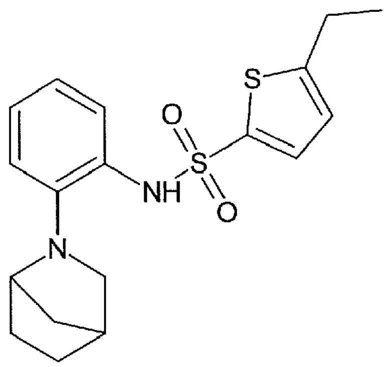 |
| 18 | 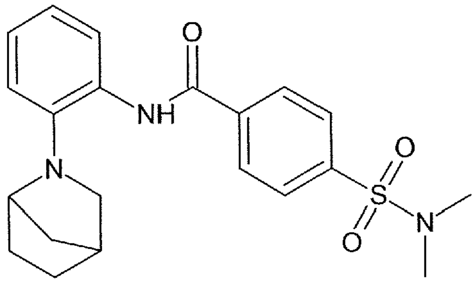 |
| 19 | 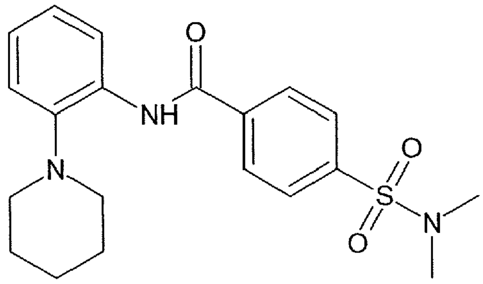 |
| 20 | 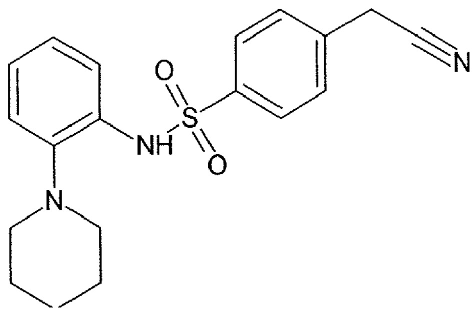 |
| 21 | 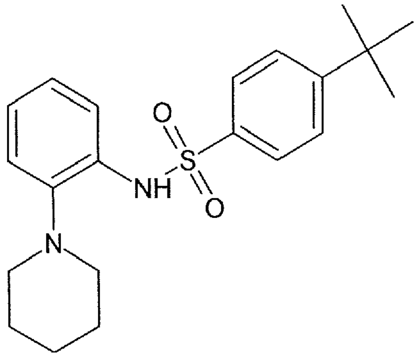 |

(continued)

| Example | Structure |
|---|---|
| 22 | 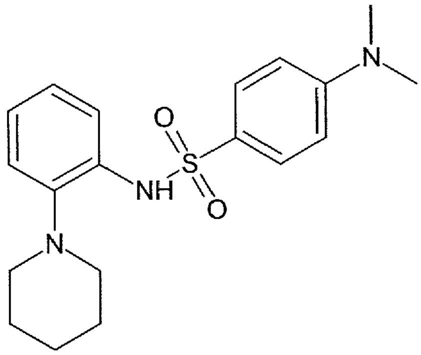 |
| 23 | 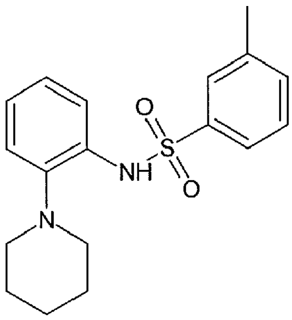 |
| 24 | 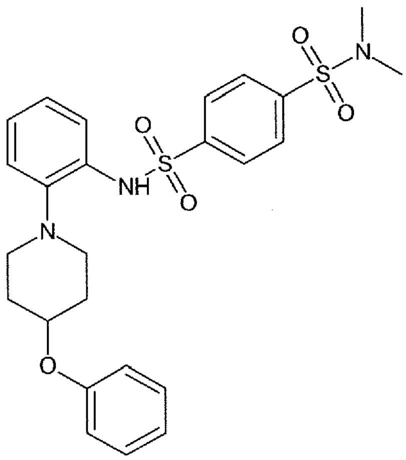 |

(continued)

| Example | Structure |
|---|---|
| 25 | 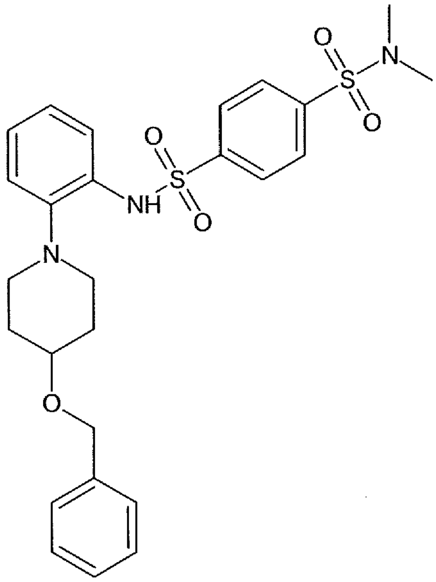 |
| 26 | 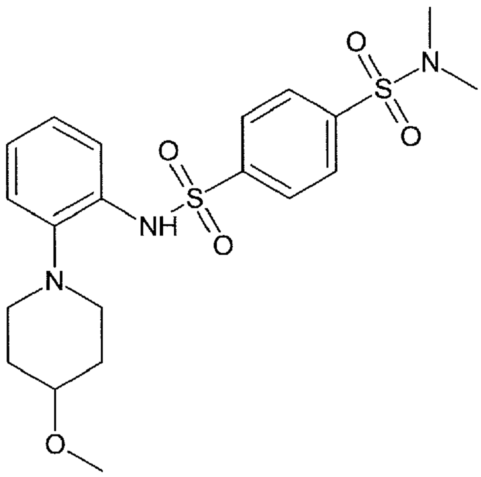 |
| 27 | 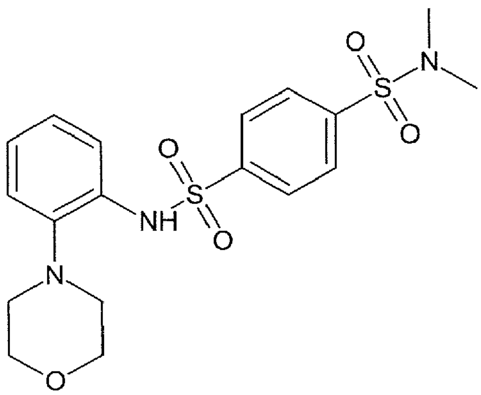 |

(continued)

| Example | Structure |
|---|---|
| 28 | 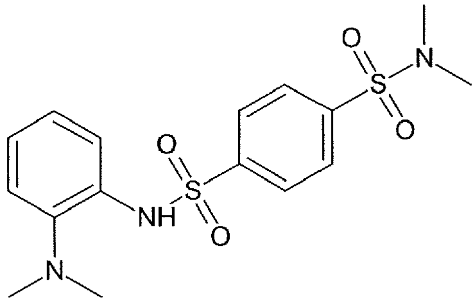 |
| 29 | 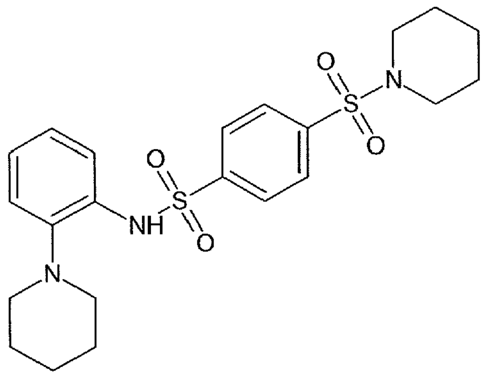 |
| 30 | 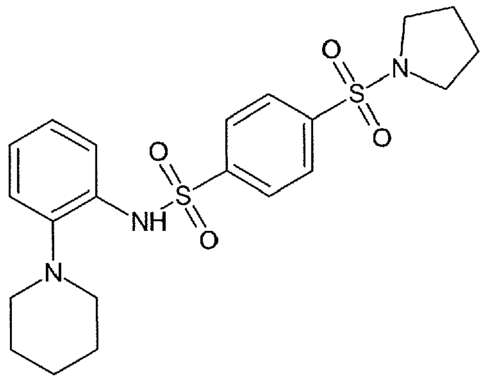 |
| 31 | 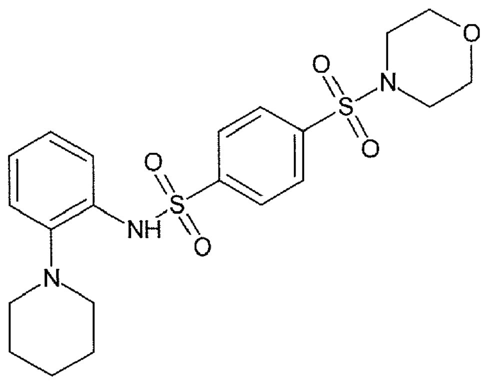 |

(continued)

| Example | Structure |
|---|---|
| 32 | 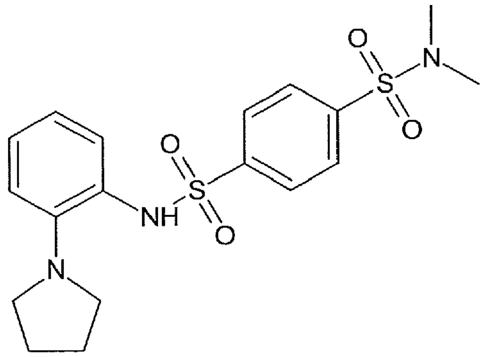 |
| 33 | 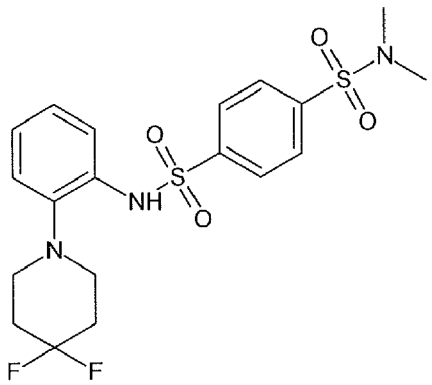 |
| 34 | 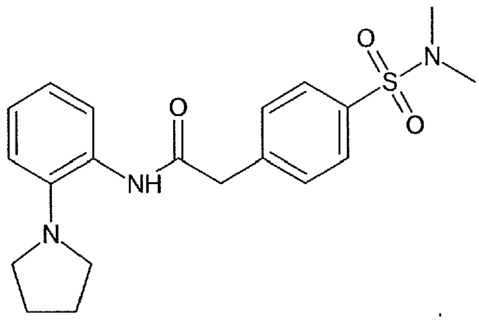 |
| 35 | 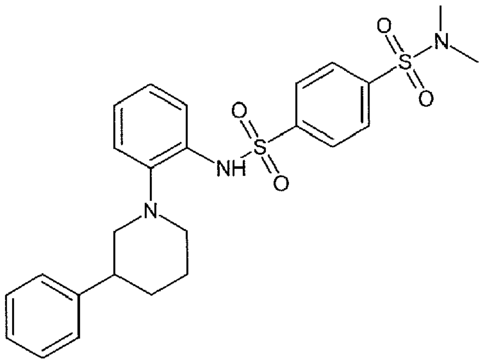 |

(continued)

| Example | Structure |
|---|---|
| 36 | 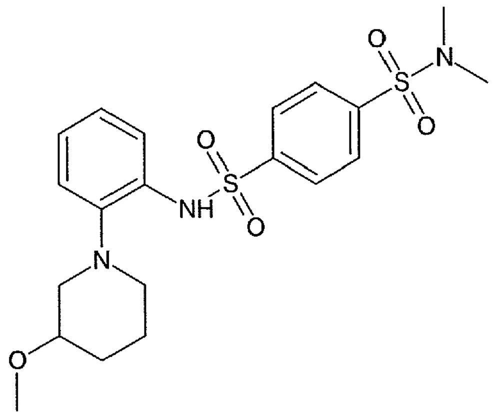 |
| 37 | 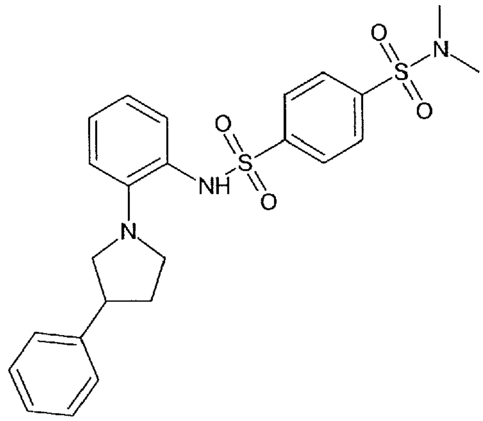 |
| 38 | 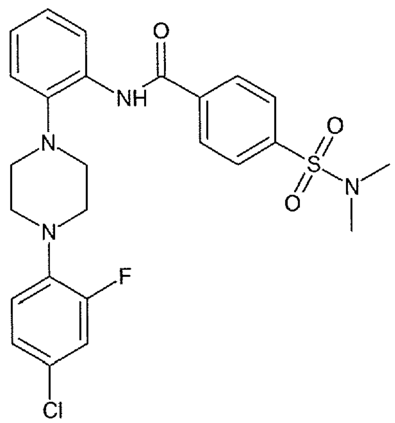 |
| 39 | 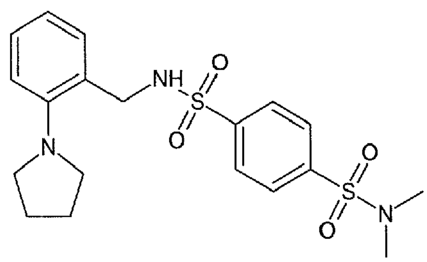 |

(continued)

| Example | Structure |
|---|---|
| 40 | 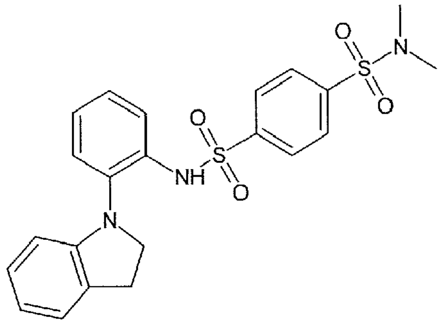 |
| 41 | 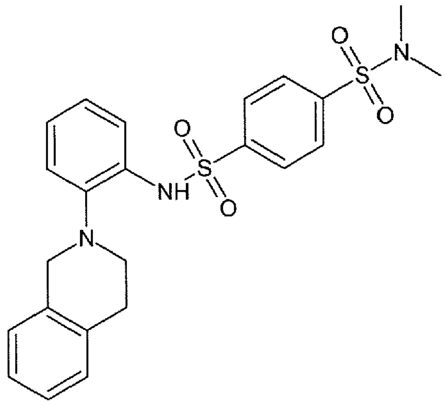 |
| 42 | 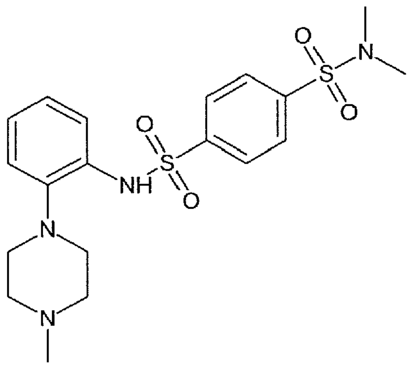 |
| 43 | 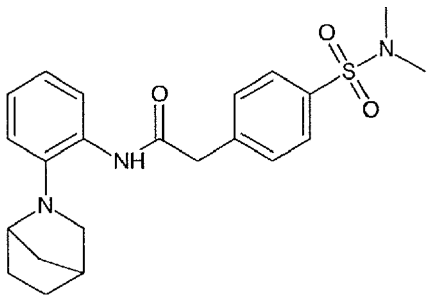 |

(continued)

| Example | Structure |
|---|---|
| 44 | 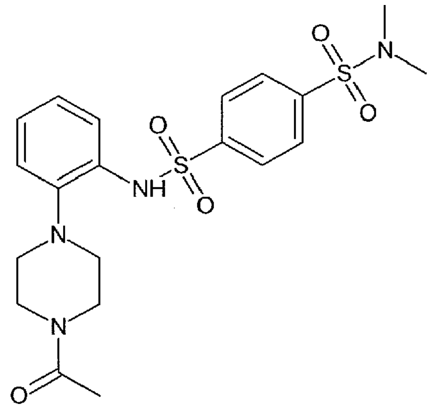 |
| 45 | 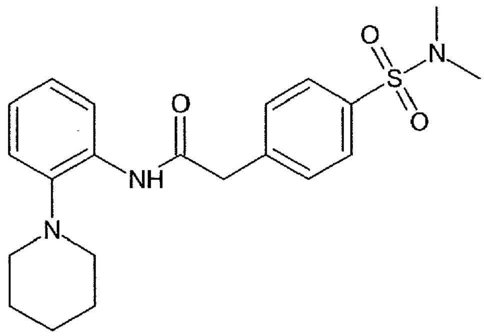 |
| 47 | 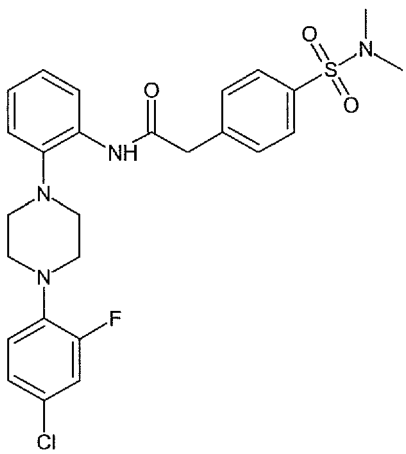 |

(continued)

| Example | Structure |
| --- | --- |
| 48 | 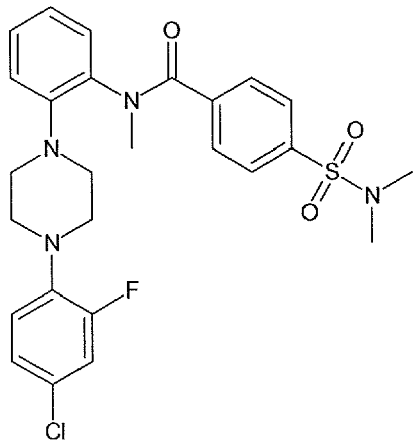 |
| 49 | 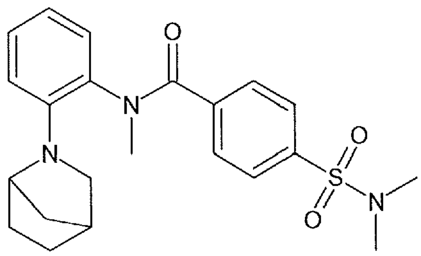 |
| 50 | 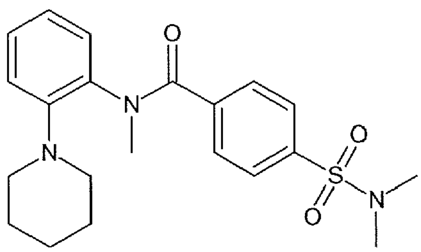 |
| 51 | 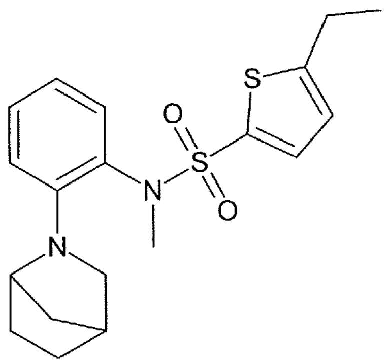 |

(continued)

| Example | Structure |
|---|---|
| 52 | 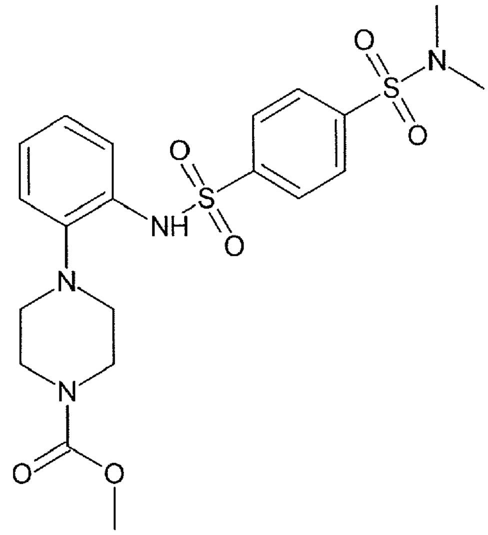 |
| 53 | 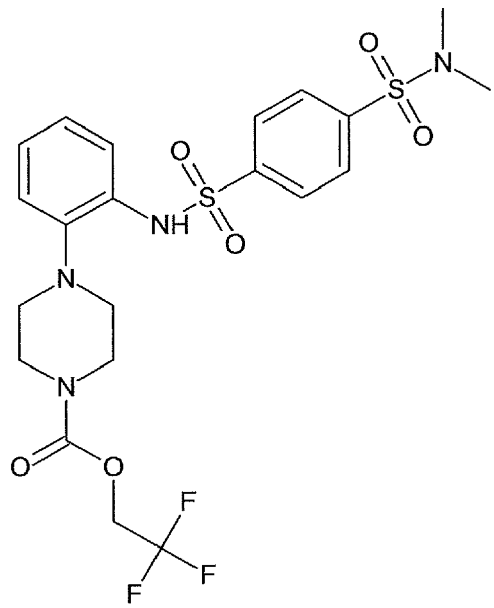 |

(continued)

| Example | Structure |
|---|---|
| 54 | 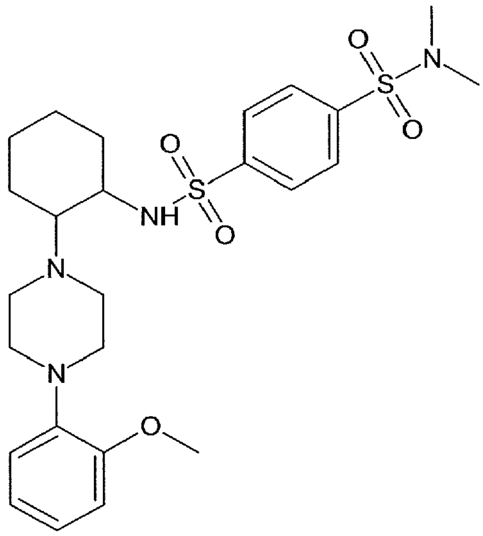 |
| 55 | 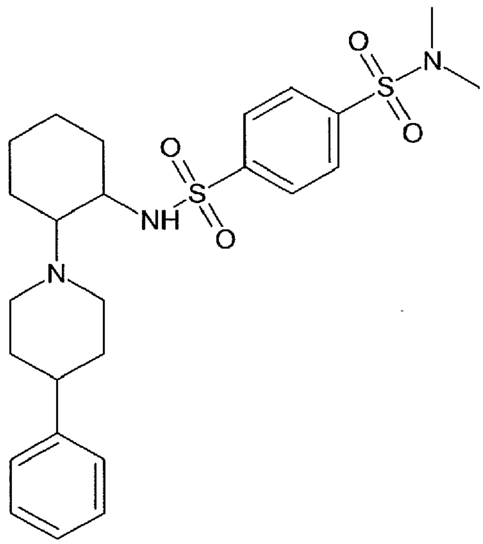 |
| 56 | 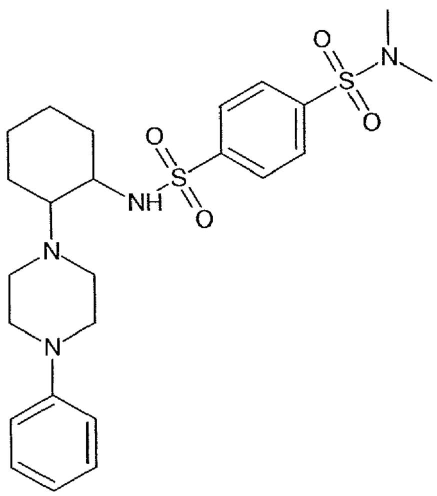 |

(continued)

| Example | Structure |
|---|---|
| 57 | 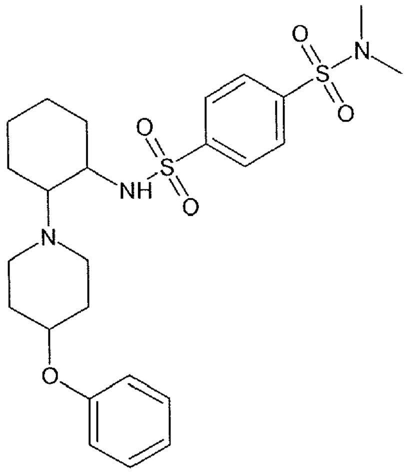 |
| 58 | 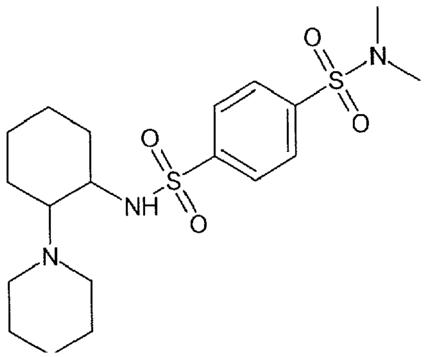 |
| 59 | 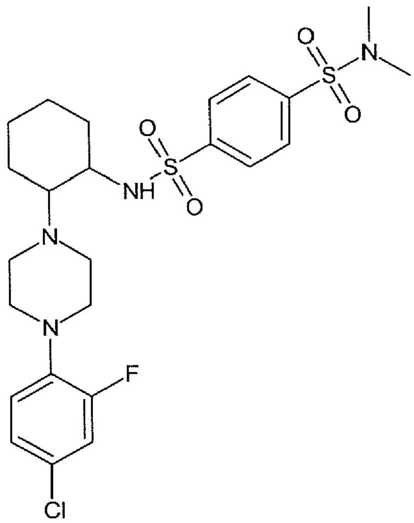 |

(continued)

| Example | Structure |
|---|---|
| 61 | 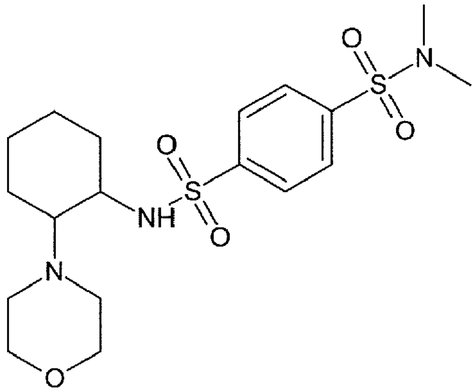 |
| 62 | 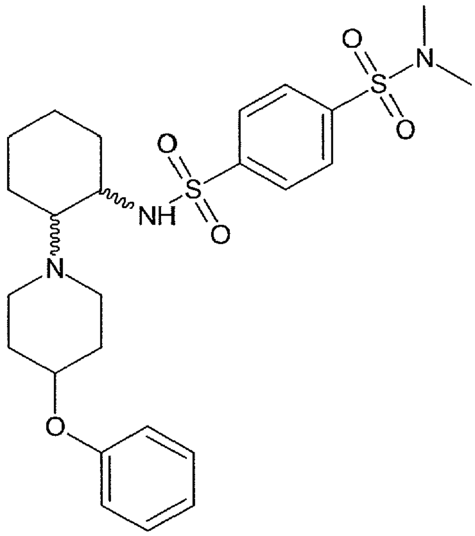 |
| 63 | 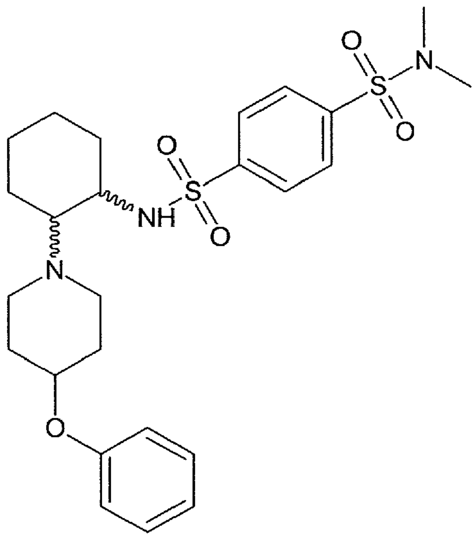 |

(continued)

| Example | Structure |
| --- | --- |
| 64 | 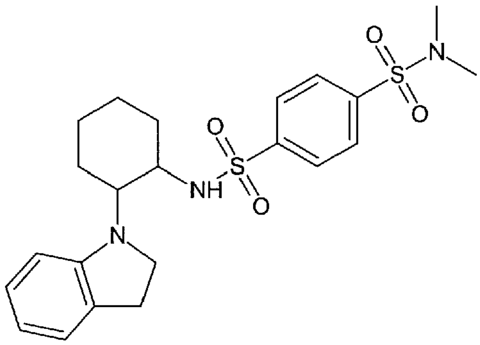 |
| 65 | 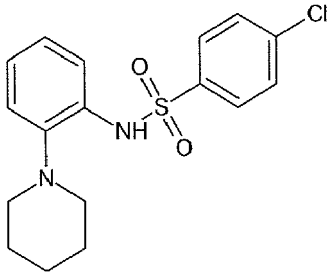 |
| 66 | 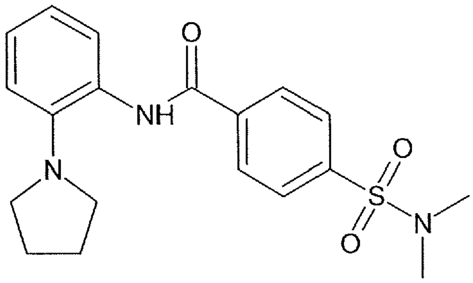 |
| 67 | 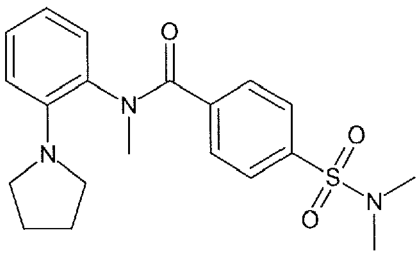 |
| 68 | 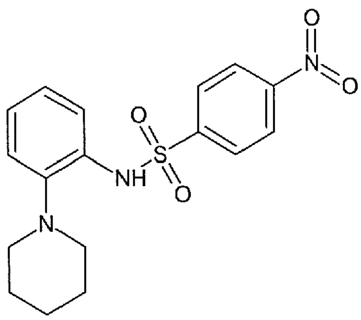 |

(continued)

| Example | Structure |
|---|---|
| 69 | 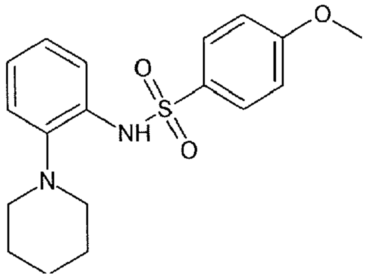 |
| 70 | 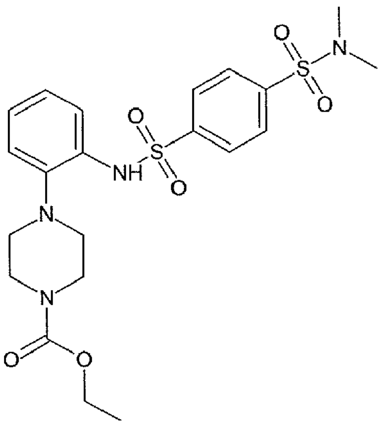 |
| 71 | 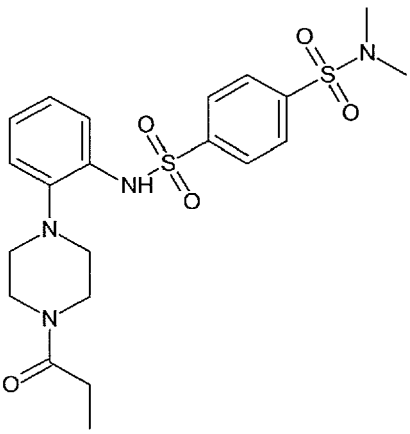 |

(continued)

| Example | Structure |
|---|---|
| 72 | 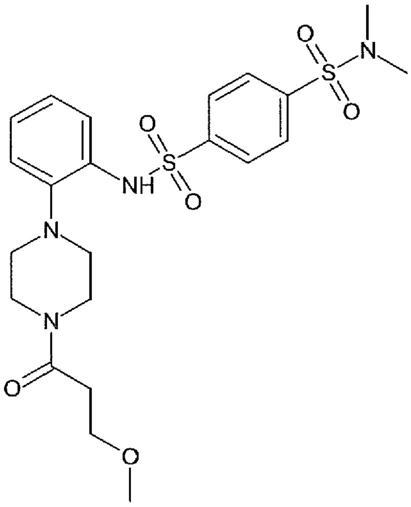 |
| 73 | 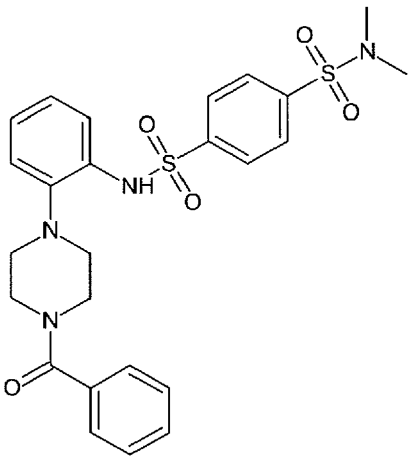 |
| 74 | 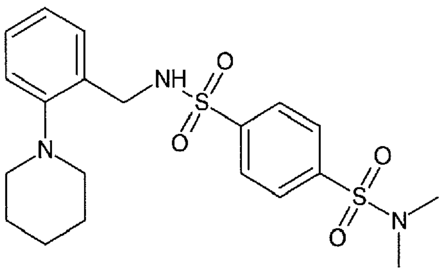 |

(continued)

| Example | Structure |
|---|---|
| 75 | 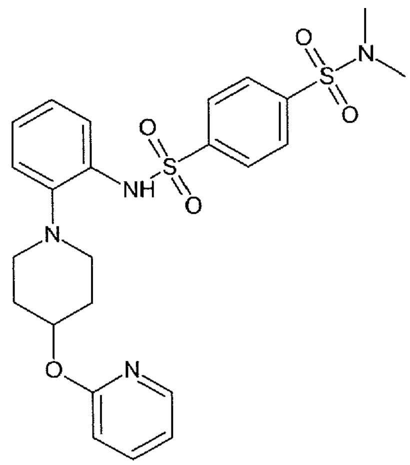 |
| 76 | 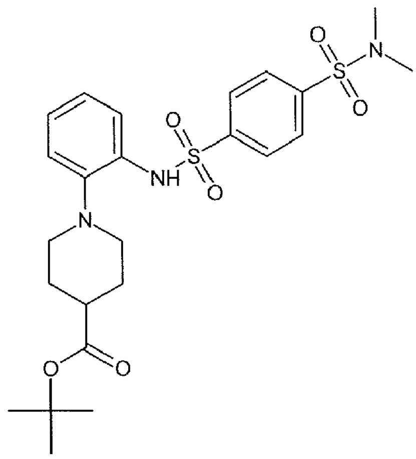 |
| 77 | 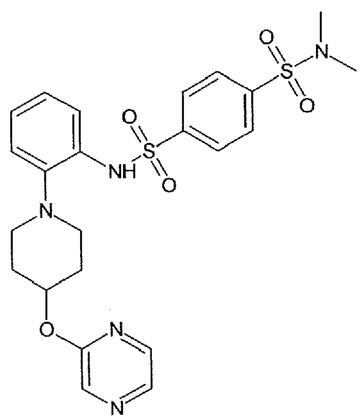 |

(continued)

| Example | Structure |
|---|---|
| 78 | 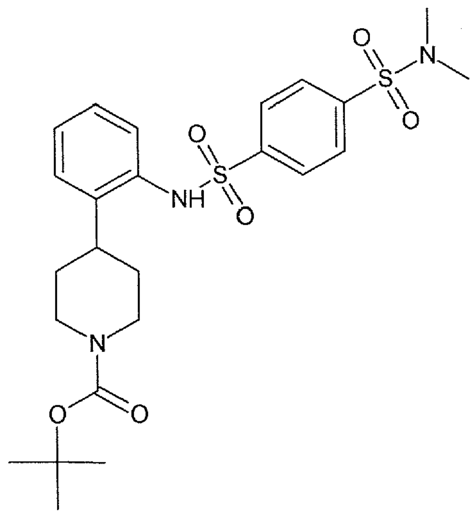 |
| 79 | 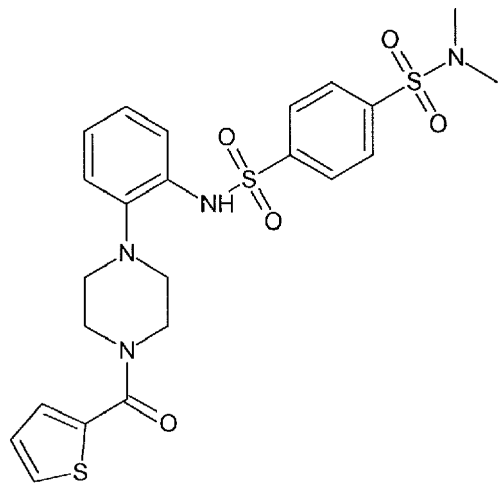 |
| 80 | 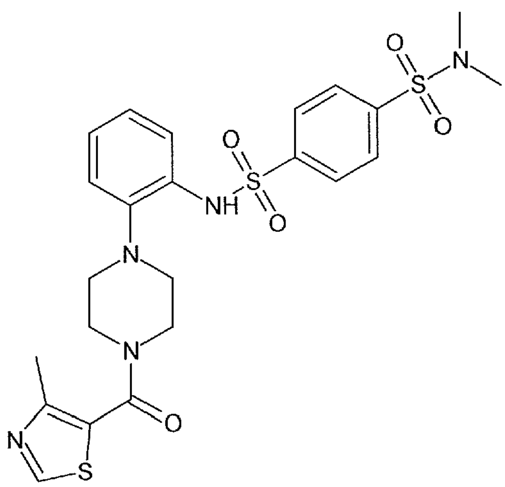 |

(continued)

| Example | Structure |
| --- | --- |
| 81 | 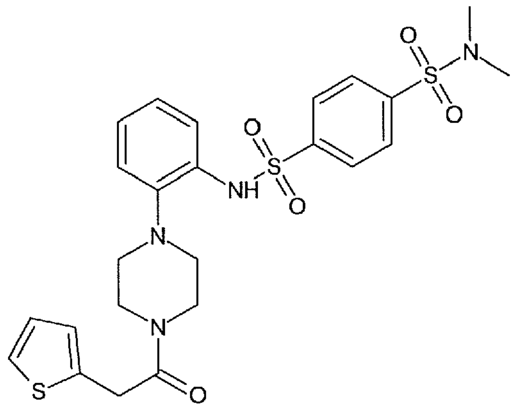 |
| 82 | 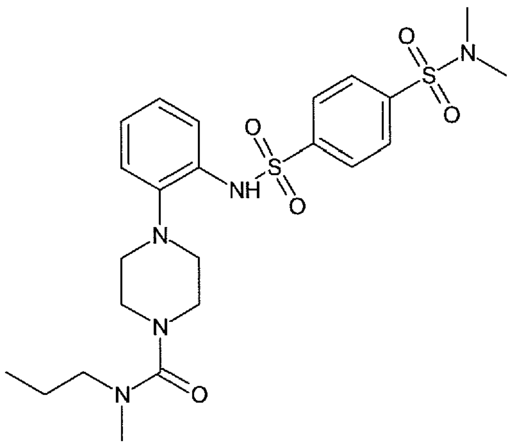 |
| 83 | 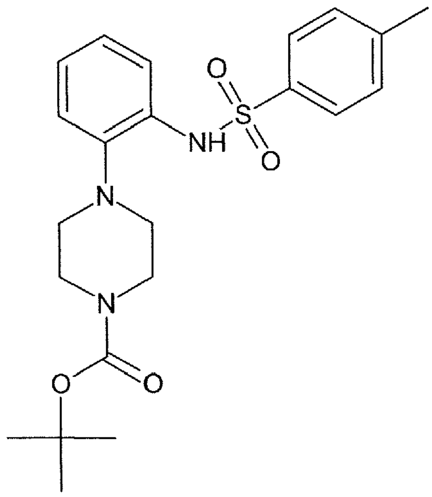 |

(continued)

| Example | Structure |
|---|---|
| 84 | 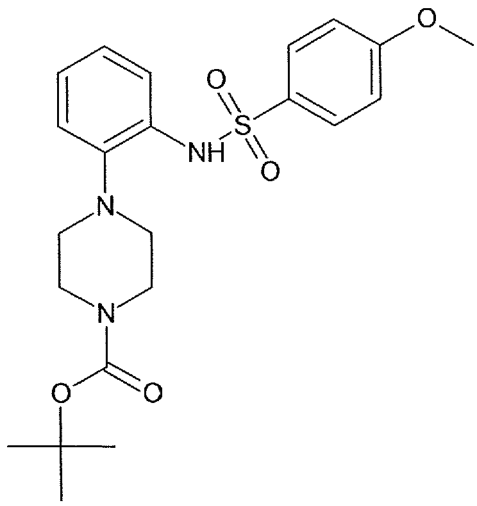 |
| 85 | 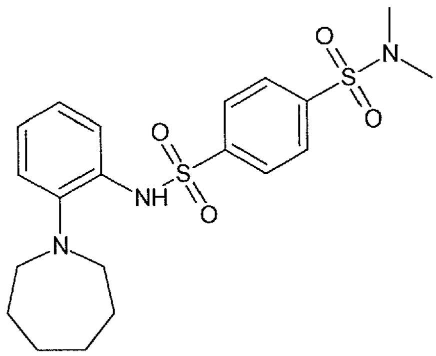 |
| 86 | 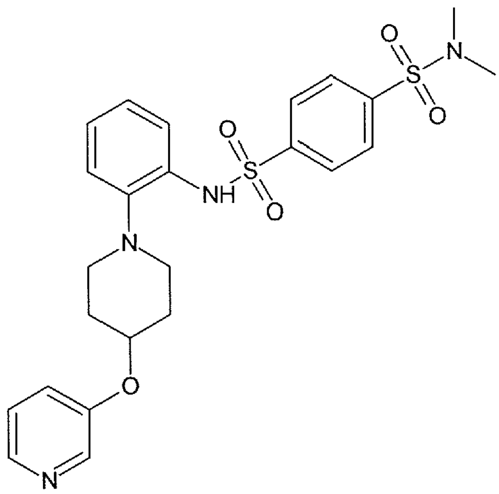 |

(continued)

| Example | Structure |
|---|---|
| 87 | 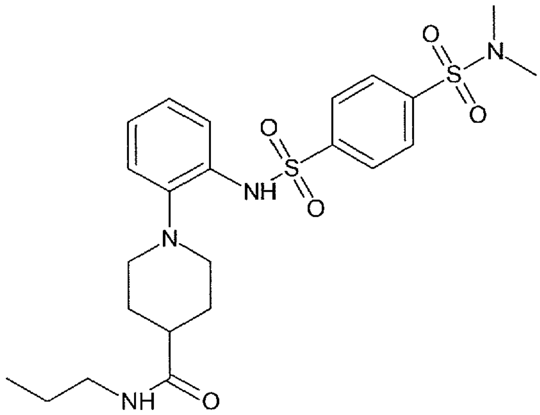 |
| 88 | 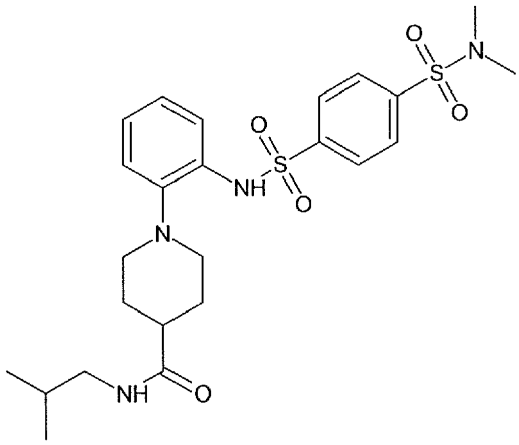 |
| 89 | 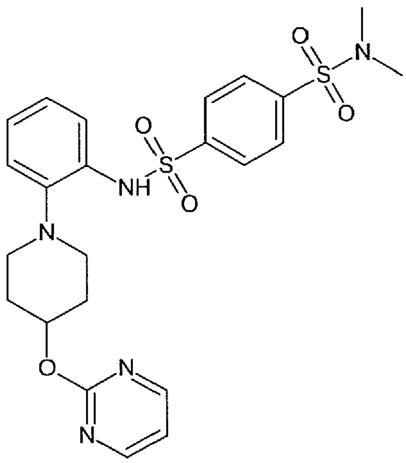 |

(continued)

| Example | Structure |
|---|---|
| 90 | 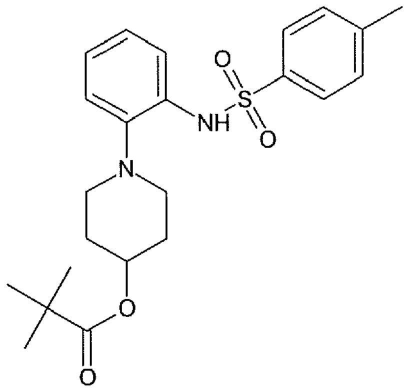 |
| 91 | 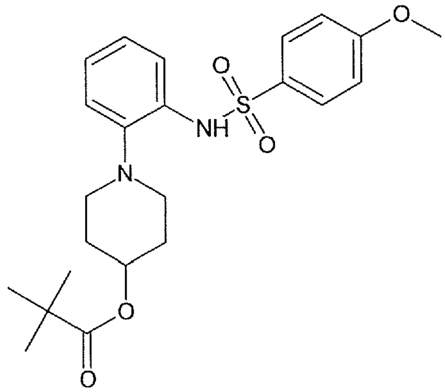 |

## Analytical Procedures and Data

### Purification system

Flash Chromatography (FC)

FC separations were performed on Biotage Isolera F our equipped with UV detector. Type of silica columns: Claricep Screw-on, Irregular, 40-60 μm, 12-80 g.

### System purification

Preparative HPLC

HPLC system WATERS Quaternary Gradient Mobile 2535 equipped with WATERS UV/Visible Detector 2489 set to dual-wavelength UV detection. Two mobile phases were used, mobile phase A: water (MilliQ) 0,05 % TFA; mobile phase B: acetonitrile (Chromasolv Sigma-Aldrich) 0,05 % TFA, and the run gradient conditions were set specifically for each compound. The purifications were achieved on a LUNA 5 μm C18 150 x 21.2 column. An injection volume between 100 and 500 μl was applied, and the flow was 15 ml/ min.

Racemate separation

The two enantiomers Example 62 and 63 were obtained by resolution of the racemic mixture (Example 57) using a WATERS Quaternary Gradient Mobile 2535 equipped with WATERS UV/Visible Detector 2489 set to a dual-wavelength UV detection at 245 and 275 nm. The chiral resolution was achieved on the Lux Amylose-1 column (250 mm × 21.6 mm) using Hexane-Isopropanol 68:32 (v/v) as isocratic mobile phase; The sample was eluted from the column at a flow rate of 1.0 ml/min at room temperature (Pressure: ≈ 800 psi). The racemic mixture was dissolved in Ethanol and heated, at concentration of 1.0 % (w/v) and the injection volume was 100 μL.

LCMS

**LCMS - procedure 1**

The HPLC measurement was performed using a Dionex 3000 module comprising a quaternary pump with degasser, an autosampler, a column oven (set at 29°C), a diode-array detector DAD and a column as specified in the respective methods below. Flow from the column was split to a MS spectrometer. The MS detector (LCQ Fleet Thermo Scientific) was configured with an electrospray ionization source. Mass spectra were acquired by scanning from 50 to 800 in 0.48 second. The capillary needle voltage was 5 kV in positive and negative ionization mode and the source temperature was maintained at 275°C. Nitrogen was used as the nebulizer gas, the flow was 8 l/min. Data acquisition was performed with Thermo Xcalibur Qual Browser. Reversed phase HPLC was carried out on a Kinetex XB-C18 column Phenomenex (1.7 μm, 50 x 2.1 mm) with a flow rate of 0.300 ml/min. Two mobile phases were used, mobile phase A: ammonium formate buffer solution at pH 3.5; mobile phase B: acetonitrile (Chromasolv Sigma-Aldrich), and they were employed to run a gradient conditions from 15 % B for 0.5 minutes, from 15 % to 98 % in 4.0 minutes, 98 % B for 1.35 minutes and 15 % B in 0.10 minutes and hold these conditions for 2.75 minutes in order to reequilibrate the column (Total Run Time 8.7 minutes). An injection volume of 1 μl was used.

**LCMS - procedure 2**

The HPLC measurement was performed using a Vanquish module comprising a quaternary pump with degasser, an autosampler, a column oven (set at 29° C), a diode-array detector DAD and a column as specified in the respective methods below. Flow from the column was split to a MS spectrometer. The MS detector (ISQEC) was configured with an electrospray ionization source. Mass spectra were acquired by scanning from 50 to 600 in 0.2 second. The capillary needle voltage was 3 kV in positive and 2 kV in negative ionization mode and the source temperature was maintained at 250 °C. Nitrogen was used as the nebulizer gas. Data acquisition was performed with Chromeleon 7. Reversed phase HPLC was carried out on a Luna Omega Polar C18 column (50 x 2.1 mm 1.6 μm) with a flow rate of 0.5 mL/min. Two mobile phases were used, mobile phase A: ammonium formate buffer solution at pH 3.5; mobile phase B: acetonitrile (Chromasolv Sigma-Aldrich), and they were employed to run a gradient conditions from 15 % B for 0.2 minutes, from 15 % to 95 % in 1.6 minutes, 98 % B for 0.65 minutes and 15 % B in 0.15 minutes and hold these conditions for 1.9 minutes in order to reequilibrate the column (Total Run Time 4.5 minutes). An injection volume of 0.8 μl was used.

**Table 3.** Retention time ($R_t$) in minutes, $[M+H]^+$ and/or [M-H]- peak and LCMS procedure

| Example | $R_t$ (min) | $[M+H]^+$ | $[M-H]^-$ | LC-MS |
|---|---|---|---|---|
| 1 | 6.22 | 331.2 | 329.4 | 1 |
| 2 | 6.15 | 337.1 | 335.1 | 1 |
| 3 | 6.88 | 379.1 | 377.1 | 1 |
| 4 | 5.48 | 436.0 | 434.4 | 1 |
| 5 | 5.88 | 343.1 | 341.1 | 1 |
| 6 | 5.80 | 424.1 | 422.3 | 1 |
| 7 | 6.45 | 351.3 | 349.2 | 1 |
| 8 | 6.38 | 351.3 | 349.4 | 1 |
| 9 | 6.74 | 365.3 | 363.5 | 1 |
| 10 | 6.47 | 373.3 | 371.4 | 1 |
| 11 | 5.87 | 395.3 | 393.4 | 1 |
| 12 | 6.31 | 500.2 | 498.7 | 1 |
| 13 | 5.77 | 525.0 | 523.6 | 1 |
| 14 | 7.34 | 508.9 | 506.9 | 1 |
| 15 | 5.82 | 349.2 | 347.3 | 1 |
| 16 | 6.53 | 391.3 | 389.6 | 1 |
| 17 | 6.08 | 363.2 | 361.5 | 1 |

(continued)

| Example | $R_t$ (min) | $[M+H]^+$ | $[M-H]^-$ | LC-MS |
|---|---|---|---|---|
| 18 | 5.37 | 400.4 | 398.3 | 1 |
| 19 | 5.95 | 388.4 | 386.3 | 1 |
| 20 | 5.67 | 356.2 | 354.5 | 1 |
| 21 | 6.91 | 373.3 | 371.5 | 1 |
| 22 | 6.11 | 360.2 | 358.5 | 1 |
| 23 | 6.25 | 331.2 | 329.4 | 1 |
| 24 | 6.12 | 516.1 | 514.6 | 1 |
| 25 | 6.19 | 530.2 | 528.8 | 1 |
| 26 | 5.41 | 454.3 | 542.6 | 1 |
| 27 | 5.09 | 426.3 | 424.5 | 1 |
| 28 | 5.13 | 384.2 | 382.2 | 1 |
| 29 | 6.26 | 464.5 | 462.6 | 1 |
| 30 | 5.97 | 450.4 | 448.6 | 1 |
| 31 | 5.73 | 466.4 | 464.6 | 1 |
| 32 | 5.28 | 410.3 | 408.5 | 1 |
| 33 | 5.58 | 460.2 | 458.6 | 1 |
| 34 | 5.06 | 388.4 | - | 1 |
| 35 | 6.28 | 500.3 | 498.7 | 1 |
| 36 | 5.58 | 454.3 | 542.6 | 1 |
| 37 | 5.88 | 486.4 | 484.7 | 1 |
| 38 | 6.50 | - | 515.9 | 1 |
| 39 | 2.93 | 424.2 | 422.1 | 2 |
| 40 | 5.74 | 458.1 | 456.6 | 1 |
| 41 | 5.86 | 472.3 | 470.6 | 1 |
| 42 | 4.11 | 439.1 | 437.1 | 1 |
| 43 | 5.23 | 414.4 | 412.1 | 1 |
| 44 | 4.78 | 467.4 | 465.6 | 1 |
| 45 | 5.62 | 402.4 | 400.1 | 1 |
| 47 | 6.17 | 531.5 | - | 1 |
| 48 | 6.06 | 531.3 | - | 1 |
| 49 | 5.44 | 414.4 | - | 1 |
| 50 | 5.48 | 402.4 | - | 1 |
| 51 | 6.46 | 377.3 | - | 1 |
| 52 | 5.18 | 483.3 | 481.6 | 1 |
| 53 | 5.60 | - | 549.7 | 1 |
| 54 | 4.63 | - | 535.4 | 1 |
| 55 | 4.62 | 506.3 | 504.6 | 1 |
| 56 | 4.69 | 507.3 | 505.7 | 1 |

(continued)

| Example | $R_t$ (min) | $[M+H]^+$ | $[M-H]^-$ | LC-MS |
|---|---|---|---|---|
| 57 | 4.67 | 522.2 | 520.7 | 1 |
| 58 | 3.94 | 430.3 | 428.3 | 1 |
| 59 | 5.30 | 559.3 | 558.1 | 1 |
| 61 | 2.71 | 432.6 | 430.4 | 1 |
| 62 | 4.67 | 522.4 | 520.8 | 1 |
| 63 | 4.67 | 522.4 | 520.7 | 1 |
| 64 | 5.75 | 464.5 | 462.9 | 1 |
| 65 | 6.38 | 351.5 | 349.2 | 1 |
| 66 | 5.26 | 374.3 | - | 1 |
| 67 | 5.24 | 388.3 | - | 1 |
| 68 | 3.14 | 362.2 | 360.1 | 2 |
| 69 | 3.16 | 347.2 | 245.2 | 2 |
| 70 | 2.90 | 497.2 | 495.2 | 2 |
| 71 | 2.74 | 481.2 | 479.2 | 2 |
| 72 | 2.70 | 511.4 | 509.2 | 2 |
| 73 | 2.86 | 529.3 | 527.2 | 2 |
| 74 | 3.03 | 438.2 | 436.0 | 2 |
| 75 | 3.10 | 517.2 | 515.2 | 2 |
| 76 | 3.21 | 524.3 | 522.3 | 2 |
| 77 | 3.00 | 518.2 | 516.3 | 2 |
| 78 | 2.98 | - | 522.2 | 2 |
| 79 | 2.85 | 533.2 | 535.2 | 2 |
| 80 | 2.70 | 550.2 | 548.3 | 2 |
| 81 | 2.86 | 549.2 | 547.2 | 2 |
| 82 | 2.90 | 524.3 | 522.3 | 2 |
| 83 | 2.56 | 432.2 | 430.3 | 2 |
| 84 | 2.49 | 448.2 | 446.2 | 2 |
| 85 | 2.47 | 438.2 | 436.2 | 2 |
| 86 | 1.98 | 517.2 | 515.2 | 2 |
| 87 | 2.26 | 509.2 | 507.2 | 2 |
| 88 | 2.22 | 521.3 | 523.3 | 2 |
| 89 | 2.24 | 518.3 | 516.3 | 2 |
| 90 | 2.74 | 431.2 | - | 2 |
| 91 | 2.62 | 447.2 | 445.5 | 2 |

***NMR Characterization***

$^1H$ NMR spectra were recorded on a Varian Mercury NMR 400 MHz spectrometer using $CDCl_3$, DMSO-d6 or $CD_3OD$ as solvents Chemical shifts ($\delta$) are reported in parts per million (ppm) relative to residual signal of non-fully

deuterated solvents pick for $^1H$ NMR assigned as 7.26 ppm for $CHCl_3$, 3.31 ppm for $CHD_2OD$ and 2.50 ppm for DMSO-$d_5$.

**Table 4.** NMR data of compounds

| Example | Structure | NMR |
|---|---|---|
| 1 | 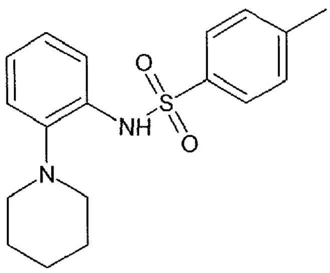 | $^1H$ NMR ($CDCl_3$) δ ppm 1.52 (br m, 2 H) 1.61 (m, 4 H) 2.32 (s, 3 H) 2.39 - 2.48 (m, 4 H) 6.94 - 7.01 (m, 1 H) 7.03 - 7.09 (m, 2 H) 7.18 (d, J=8.06 Hz, 2 H) 7.54 - 7.62 (m, 1 H) 7.68 (d, J=8.06 Hz, 2 H) 8.07 (NH) |
| 2 | 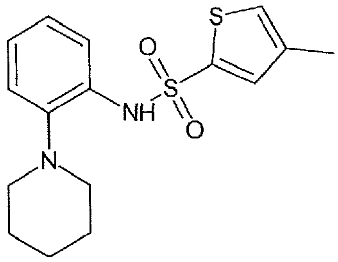 | $^1H$ NMR ($CDCl_3$) δ ppm 1.53 (m, 2 H) 1.64 (m, 4 H) 2.18 (s, 3 H) 2.43 - 2.53 (m, 4 H) 7.01 - 7.08 (br s, 2 H) 7.08 - 7.15 (br s, 2 H) 7.33 (d, J=1.37 Hz, 1 H) 7.60 - 7.67 (m, 1 H) 8.18 (NH) |
| 3 | 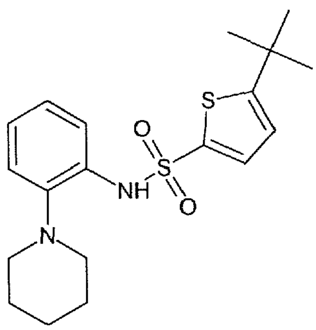 | $^1H$ NMR ($CDCl_3$) δ ppm 1.30 (s, 9 H) 1.53 (br s, 2 H) 1.60 - 1.68 (m, 4 H) 2.43 - 2.50 (m, 4 H) 6.67 (d, J=3.85 Hz, 1 H) 7.02 - 7.16 (m, 3 H) 7.24 - 7.30 (m, 1 H) 7.66 (d, J=8.06 Hz, 1 H) 8.15 (NH) |
| 4 | 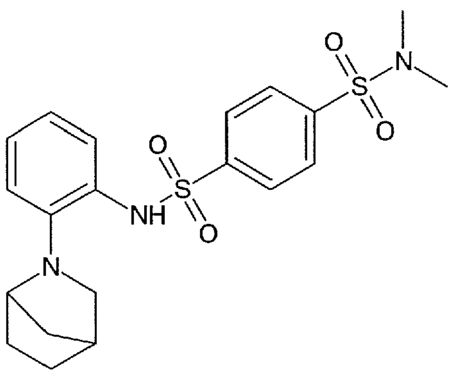 | $^1H$ NMR ($CDCl_3$) δ ppm 1.34 - 1.55 (m, 3 H) 1.60 - 1.72 (m, 3 H) 2.51 (br s, 1 H) 2.70 (s, 6 H) 2.75 (br s, 1 H) 2.99 (br s, 1 H) 3.24 (br s, 1 H) 6.89 - 6.97 (m, 2 H) 7.01 - 7.07 (m, 1 H) 7.38 (d, J=8.06 Hz, 1 H) 7.81 (d, J=8.34 Hz, 2 H) 7.94 (d, J=8.34 Hz, 2 H) |
| 5 | 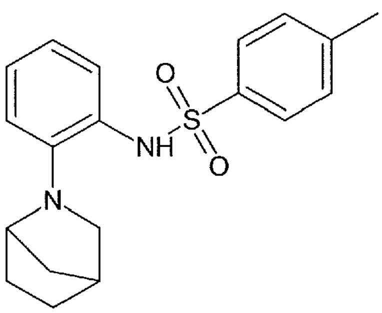 | $^1H$ NMR ($CDCl_3$) δ ppm 1.34 - 1.52 (m, 3 H) 1.58 - 1.74 (m, 3 H) 2.35 (s, 3 H) 2.50 (br s, 1 H) 2.79 (d, J=8.89 Hz, 1 H) 2.98 (dt, J=8.77, 2.90 Hz, 1 H) 3.25 (br s, 1 H) 6.88 - 7.00 (m, 3 H) 7.21 (d, J=8.06 Hz, 2 H) 7.40 (dd, J=7.84, 1.05 Hz, 1 H) 7.70 (d, J=8.25 Hz, 2 H) k |

(continued)

| Example | Structure | NMR |
|---|---|---|
| 6 | 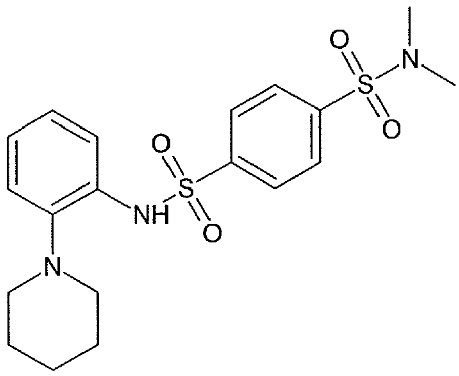 | $^1$H NMR ($CDCl_3$) δ ppm 1.52 (br s, 2 H) 1.57 - 1.66 (m, 4 H) 2.38 - 2.46 (m, 4 H) 2.67 (s, 6 H) 7.01 - 7.13 (m, 3 H) 7.58 (d, J=7.88 Hz, 1 H) 7.79 (d, J=8.34 Hz, 2 H) 7.95 (d, J=8.34 Hz, 2 H) |
| 7 | 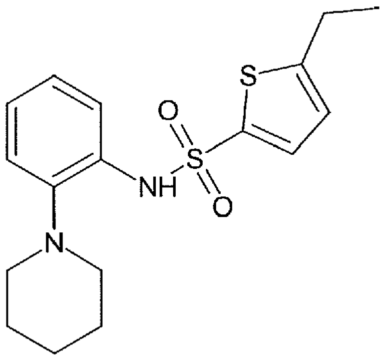 | $^1$H NMR ($CDCl_3$) δ ppm 1.23 (t, J=7.51 Hz, 3 H) 1.53 (br s, 2 H) 1.60 - 1.69 (m, 4 H) 2.44 - 2.53 (m, 4 H) 2.77 (q, J=7.51 Hz, 2 H) 6.65 (d, J=3.67 Hz, 1 H) 7.00 - 7.14 (m, 3 H) 7.32 (d, J=3.67 Hz, 1 H) 7.60 - 7.65 (m, 1 H) 8.17 (NH) |
| 8 | 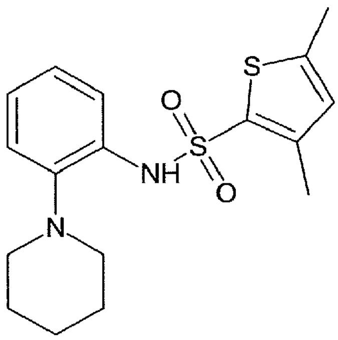 | $^1$H NMR ($CDCl_3$) δ ppm 1.55 (br s, 2 H) 1.67 (br s, 4 H) 2.28 (s, 3 H) 2.37 (s, 3 H) 2.59 (br s, 4 H) 6.49 (s, 1 H) 6.98 - 7.16 (m, 3 H) 7.59 (br d, J=7.70 Hz, 1 H) 8.25 (NH) |
| 9 | 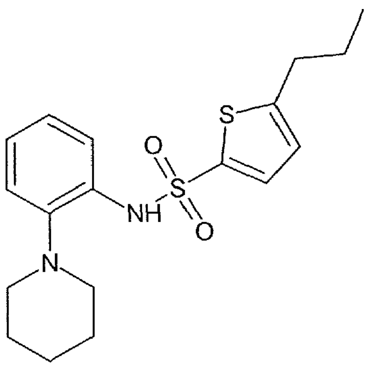 | $^1$H NMR ($CDCl_3$) δ ppm 0.90 (t, J=7.33 Hz, 3 H) 1.52 (br s, 2 H) 1.57 - 1.68 (br s, 6 H) 2.45 - 2.51 (m, 4 H) 2.70 (t, J=7.47 Hz, 2 H) 6.63 (d, J=3.67 Hz, 1 H) 6.99 - 7.08 (m, 1 H) 7.08 - 7.15 (m, 2 H) 7.32 (d, J=3.76 Hz, 1 H) 7.61 - 7.66 (m, 1 H) 8.17 (NH) |

(continued)

| Example | Structure | NMR |
|---|---|---|
| 10 | 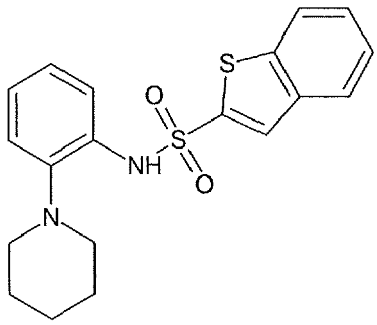 | $^1$H NMR ($CDCl_3$) δ ppm 1.52 (br s, 2 H) 1.64 (quin, J=5.34 Hz, 4 H) 2.44 - 2.53 (m, 4 H) 7.00 - 7.17 (m, 3 H) 7.41 (quint, J=7.26, 7.26, 7.26, 7.26, 1.19 Hz, 2 H) 7.70 (dd, J=8.06, 1.10 Hz, 1 H) 7.74 - 7.82 (m, 2 H) 7.84 (s, 1 H) 8.38 (NH) |
| 11 | 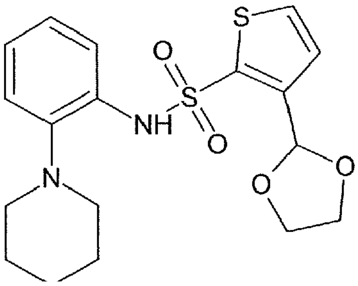 | $^1$H NMR ($CDCl_3$) δ ppm 1.54 (br s, 2 H) 1.64 - 1.72 (m, 4 H) 2.59 - 2.69 (m, 4 H) 3.96 - 4.07 (m, 4 H) 6.25 (s, 1 H) 6.97 - 7.14 (m, 3 H) 7.17 (d, J=5.13 Hz, 1 H) 7.39 (d, J=5.13 Hz, 1 H) 7.64 (dd, J=7.97, 1.01 Hz, 1 H) 8.50 (NH) |
| 12 | 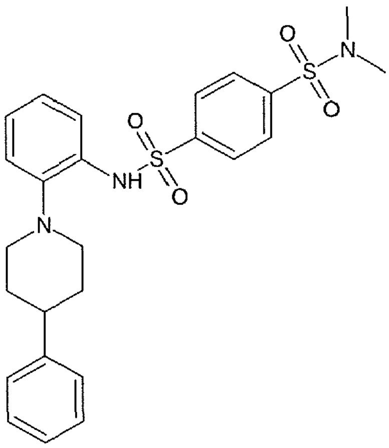 | $^1$H NMR ($CDCl_3$) δ ppm 1.76 - 1.95 (m, 4 H) 2.48 - 2.63 (m, 4 H) 2.65 - 2.76 (br s, 7 H) 7.06 - 7.11 (m, 1 H) 7.12-7.19 (m, 2 H) 7.23 - 7.30 (m, 3 H) 7.33 - 7.39 (m, 2 H) 7.62 (br d, J=7.97 Hz, 1 H) 7.80 (d, J=8.25 Hz, 2 H) 7.98 (br d, J=8.25 Hz, 2 H) 8.18 (NH) |
| 13 | 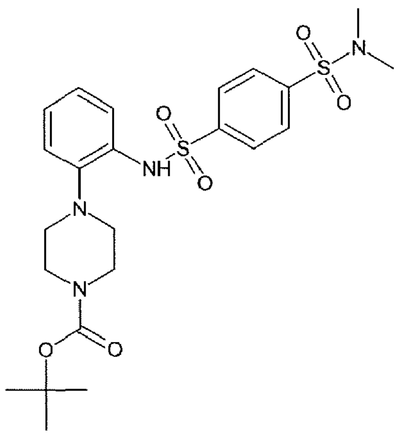 | $^1$H NMR ($CDCl_3$) δ ppm 1.48 (s, 9 H) 2.54 (br s, 4 H) 2.72 (s, 6 H) 3.52 (br s, 4 H) 7.06 - 7.12 (m, 2 H) 7.13 -7.19 (m, 1 H) 7.56 (d, J=7.97 Hz, 1 H) 7.84 (d, J=8.43 Hz, 2 H) 7.98 (d, J=8.34 Hz, 2 H) 8.12 (NH) |

(continued)

| Example | Structure | NMR |
|---|---|---|
| 14 | 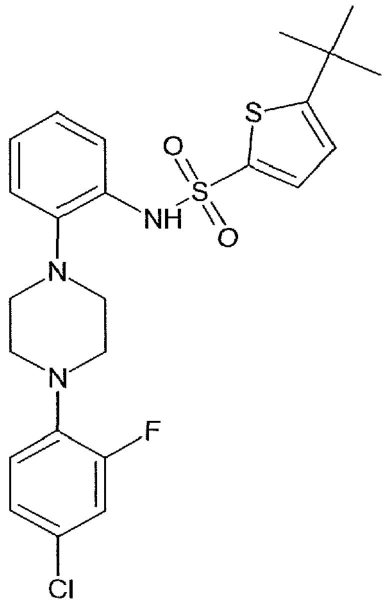 | $^1$H NMR ($CDCl_3$) δ ppm 1.31 (s, 8 H) 2.76 (br s, 4 H) 3.15 (br d, J=4.12 Hz, 4 H) 6.70 (d, J=3.85 Hz, 1 H) 6.89 - 6.96 (m, 1 H) 7.04 - 7.14 (m, 3 H) 7.17 - 7.23 (m, 2 H) 7.32 (d, J=3.76 Hz, 1 H) 7.68 (d, J=8.16 Hz, 1 H) 8.07 (NH) |
| 15 | 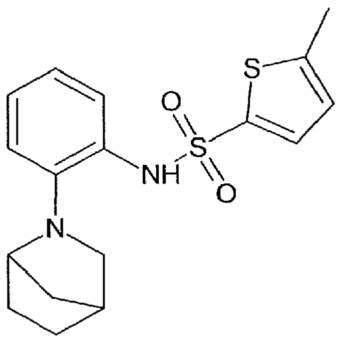 | $^1$H NMR ($CDCl_3$) δ ppm 1.33 - 1.56 (br s, 3H) 1.61 - 1.72 (m, 2 H) 1.77 (br d, J=9.62 Hz, 1 H) 2.45 (s, 3 H) 2.53 (br s, 1 H) 2.85 (d, J=8.89 Hz, 1 H) 3.00 - 3.08 (m, 1 H) 3.32 (br s, 1 H) 6.65 (d, J=3.12 Hz, 1 H) 6.91 - 7.06 (m, 3 H) 7.36 (d, J=3.57 Hz, 1 H) 7.48 (d, J=7.61 Hz, 1 H) 7.61 (br s, NH) |
| 16 | 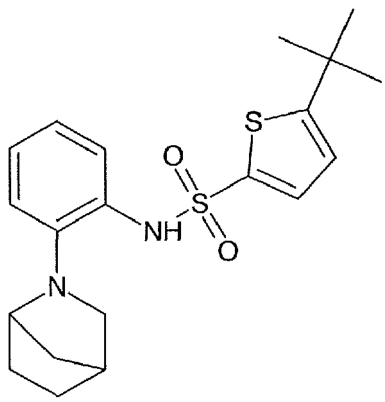 | $^1$H NMR ($CDCl_3$) δ ppm 1.32 (s, 9 H) 1.35 - 1.52 (br s, 3 H) 1.60 - 1.72 (br s, 2 H) 1.75 (br d, J=9.62 Hz, 1 H) 2.51 (br s, 1 H) 2.80 (d, J=8.89 Hz, 1 H) 3.02 (dt, J=8.77, 2.90 Hz, 1 H) 3.22 (br s, 1 H) 6.71 (d, J=3.85 Hz, 1 H) 6.92 - 7.06 (m, 3 H) 7.31 -7.35 (m, 1 H) 7.32 (d, J=3.85 Hz, 1 H) 7.49 - 7.53 (m, 1 H) |

(continued)

| Example | Structure | NMR |
|---|---|---|
| 17 | 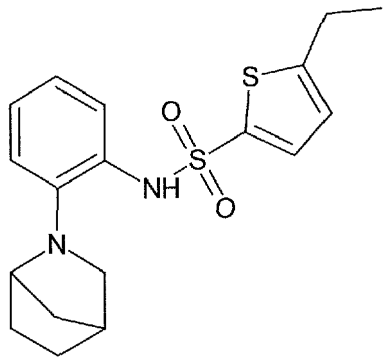 | $^{1}H$ NMR ($CDCl_3$) δ ppm 1.25 (t, J=7.51 Hz, 3 H) 1.36 - 1.54 (br s, 3 H) 1.60 - 1.73 (br s, 2 H) 1.76 (br d, J=9.62 Hz, 1 H) 2.52 (br s, 1 H) 2.75 - 2.87 (br s, 3 H) 3.03 (dt, J=8.80, 2.89 Hz, 1 H) 3.28 (br s, 1 H) 6.68 (d, J=3.76 Hz, 1 H) 6.92 - 7.05 (m, 3 H) 7.36 (d, J=3.76 Hz, 1 H) 7.49 (dd, J=7.84, 1.15 Hz, 1 H) |
| 18 | 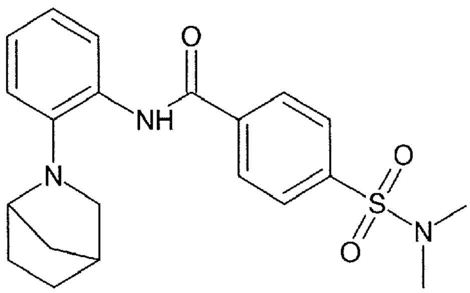 | $^{1}H$ NMR ($CDCl_3$) δ ppm 1.45 - 1.60 (br s, 3 H) 1.66 - 1.77 (br s, 2 H) 1.85 (br d, J=9.71 Hz, 1 H) 2.58 (br s, 1 H) 2.74 (s, 6 H) 2.96 (d, J=8.98 Hz, 1 H) 3.22 (br d, J=8.80 Hz, 1 H) 3.61 (br s, 1 H) 7.02 - 7.11 (m, 3 H) 7.88 (d, J=8.34 Hz, 2 H) 8.05 (br d, J=8.06 Hz, 2 H) 8.25 (br d, J=6.87 Hz, 1 H) 9.08 (NH) |
| 19 | 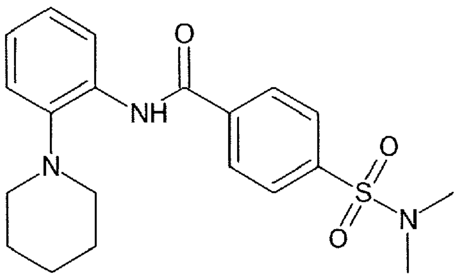 | $^{1}H$ NMR ($CDCl_3$) δ ppm 1.62 (br d, J=3.30 Hz, 2 H) 1.73 (quin, J=5.25 Hz, 4 H) 2.73 (s, 6 H) 2.83 (br t, J=4.99 Hz, 4 H) 7.06 - 7.13 (m, 1 H) 7.14 - 7.23 (m, 2 H) 7.91 (d, J=8.43 Hz, 2 H) 8.07 (d, J=8.43 Hz, 2 H) 8.51 (dd, J=8.02, 0.87 Hz, 1 H) 9.68 (NH) |
| 20 | 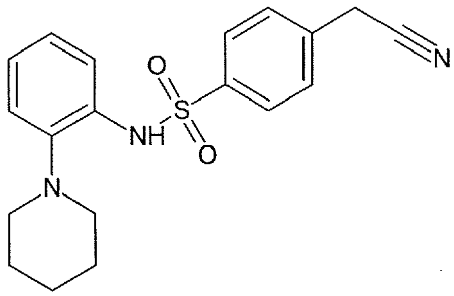 | $^{1}H$ NMR ($CDCl_3$) δ ppm 1.36 - 1.57 (m, 2 H) 1.57 - 1.68 (m, 4 H) 2.37 - 2.52 (m, 4 H) 3.76 (s, 2 H) 6.98 - 7.13 (br s, 3 H) 7.38 (d, J=8.34 Hz, 2 H) 7.56 (d, J=7.97 Hz, 1 H) 7.81 (d, J=8.34 Hz, 2 H) 8.1.3 (NH) |
| 21 | 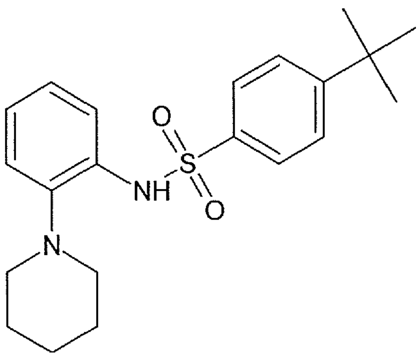 | $^{1}H$ NMR ($CDCl_3$) δ ppm 1.26 (s, 9 H) 1.50 (br s, 2 H) 1.53 - 1.62 (m, 4 H) 2.31 - 2.40 (m, 4 H) 6.97 - 7.14 (br s, 3 H) 7.40 (d, J=8.52 Hz, 2 H) 7.63 (d, J=8.38 Hz, 1 H) 7.70 (d, J=8.52 Hz, 2 H) 8.01 (NH) |

(continued)

| Example | Structure | NMR |
|---|---|---|
| 22 | 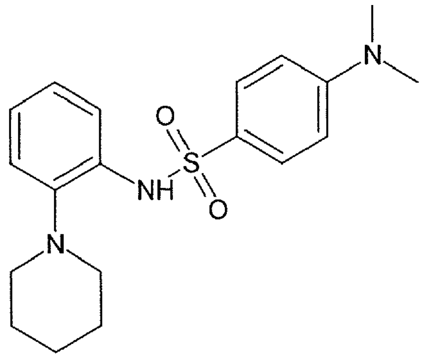 | $^1$H NMR ($CDCl_3$) $\delta$ ppm 1.51 (br s, 2 H) 1.55 - 1.68 (m, 4 H) 2.38 - 2.52 (m, 4 H) 2.92 (s, 6 H) 6.51 (d, J=9.07 Hz, 2 H) 6.92 - 7.07 (br s, 3 H) 7.53 - 7.63 (br s, 3 H) 7.98 (NH) |
| 23 | 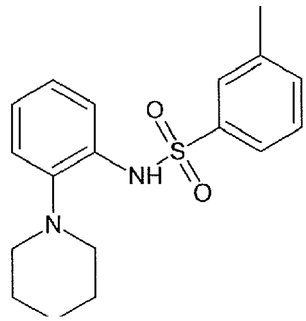 | $^1$H NMR ($CDCl_3$) $\delta$ ppm 1.51 (m, 2 H) 1.61 (quin, J=5.27 Hz, 4 H) 2.31 (s, 3 H) 2.38 - 2.43 (m, 4 H) 6.96 - 7.11 (br s, 3 H) 7.21 - 7.29 (m, 2 H) 7.56 (br d, J=7.15 Hz, 1 H) 7.59 - 7.64 (m, 2 H) 8.09 (NH) |
| 24 | 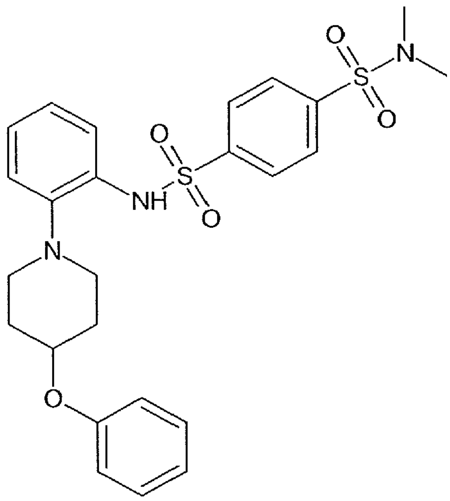 | $^1$H NMR ($CDCl_3$) $\delta$ ppm 1.91 (br s, 2 H) 2.02 (br s, 2 H) 2.47 (br s, 2 H) 2.67 (s, 6 H) 2.73 (br s, 2 H) 4.45 (br s, 1 H) 6.92 (d, J=8.06 Hz, 2 H) 6.94 - 6.99 (m, 1 H) 7.05 - 7.11 (m, 1 H) 7.11 - 7.19 (m, 2 H) 7.30 (t, J=7.88 Hz, 2 H) 7.60 (br d, J=7.79 Hz, 1 H) 7.81 (br d, J=8.25 Hz, 2 H) 7.97 (br d, J=8.25 Hz, 2 H) 8.13 (NH) |

(continued)

| Example | Structure | NMR |
|---|---|---|
| 25 | 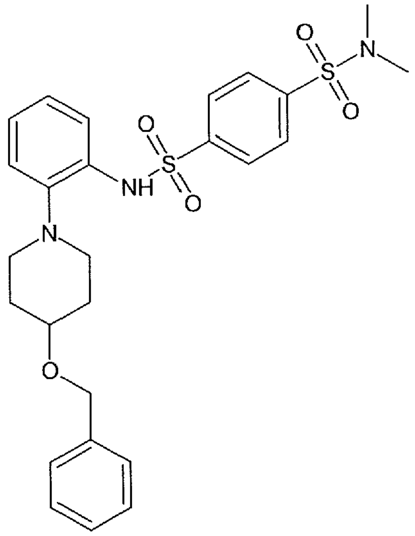 | $^1$H NMR ($CDCl_3$) δ ppm 1.74 (m, 2 H) 1.94 (m, 2 H) 2.42 (m, 2 H) 2.53 - 2.65 (m, 2 H) 2.69 (s, 6 H) 3.54 (m, 1 H) 4.56 (s, 2 H) 7.09 (tt, J=14.25, 7.29 Hz, 3 H) 7.27 - 7.33 (m, 1 H) 7.33 - 7.42 (m, 4 H) 7.60 (br d, J=7.79 Hz, 1 H) 7.80 (br d, J=8.06 Hz, 2 H) 7.96 (br d, J=8.06 Hz, 2 H) 8.12 (NH) |
| 26 | 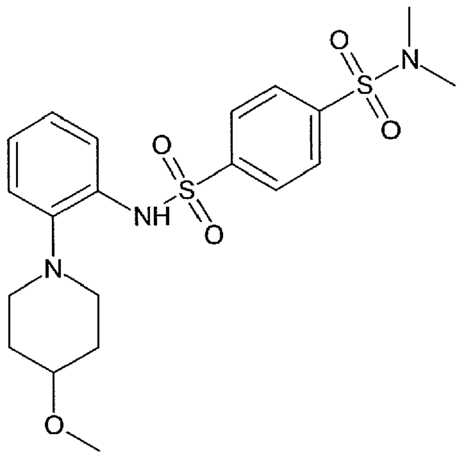 | $^1$H NMR ($CDCl_3$) δ ppm 1.64 (m, 2 H) 1.89 (m, 2 H) 2.39 (m, 2 H) 2.55 (m, 2 H) 2.67 (s, 6 H) 3.33 (br s, 4 H) 6.99 - 7.14 (m, 3 H) 7.57 (br d, J=7.79 Hz, 1 H) 7.79 (br d, J=8.25 Hz, 2 H) 7.94 (br d, J=8.25 Hz, 2 H) 8.11 (NH) |
| 27 | 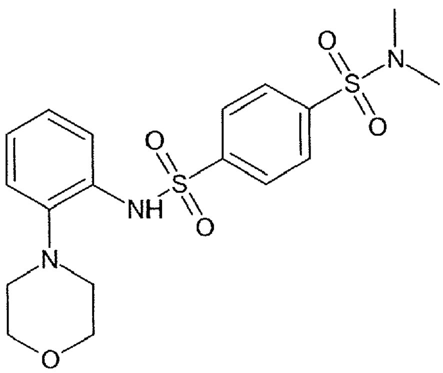 | $^1$H NMR ($CDCl_3$) δ ppm 2.56 (br s, 4 H) 2.70 (s, 6 H) 3.72 - 3.80 (m, 4 H) 7.06 - 7.17 (br s, 3 H) 7.55 (br d, J=7.97 Hz, 1 H) 7.82 (d, J=8.43 Hz, 2 H) 7.98 (d, J=8.43 Hz, 2 H) 8.09 (NH) |

(continued)

| Example | Structure | NMR |
|---|---|---|
| 28 | 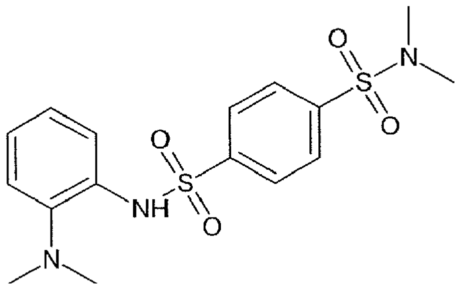 | $^1$H NMR ($CDCl_3$) δ ppm 2.40 (s, 7 H) 2.68 (s, 6 H) 6.99 - 7.16 (m, 3 H) 7.55 (dd, J=7.93, 1.42 Hz, 1 H) 7.81 (d, J=8.43 Hz, 2 H) 7.96 - 8.01 (d, J=8.43 Hz, 2 H) |
| 29 | 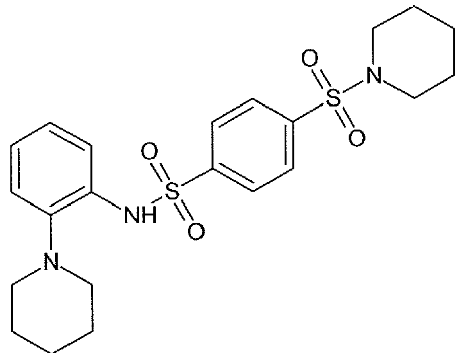 | $^1$H NMR ($CDCl_3$) δ ppm 1.33 - 1.42 (m, 2 H) 1.49 (m, 2 H) 1.53 - 1.64 (m, 8 H) 2.36 - 2.44 (m, 4 H) 2.92 (m, 4 H) 6.98 - 7.10 (m, 3 H) 7.56 (d, J=8.16 Hz, 1 H) 7.75 (d, J=8.34 Hz, 2 H) 7.92 (d, J=8.34 Hz, 2 H) 8.13 (NH) |
| 30 | 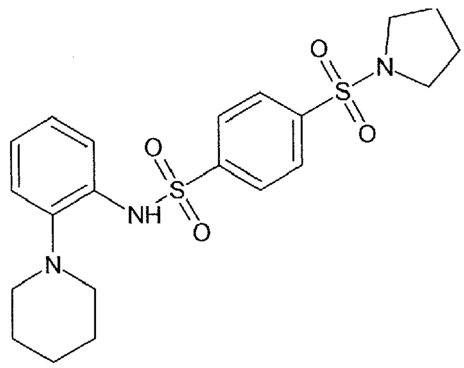 | $^1$H NMR ($CDCl_3$) δ ppm 1.51 (m, 2 H) 1.60 (quin, J=5.25 Hz, 4 H) 1.68 - 1.76 (m, 4 H) 2.37 - 2.44 (m, 4 H) 3.18 (br t, J=6.74 Hz, 4 H) 6.99 - 7.11 (br s, 3 H) 7.54 - 7.59 (m, 1 H) 7.81 - 7.87 (m, 2 H) 7.91 - 7.95 (m, 2 H) 8.15 (NH) |
| 31 | 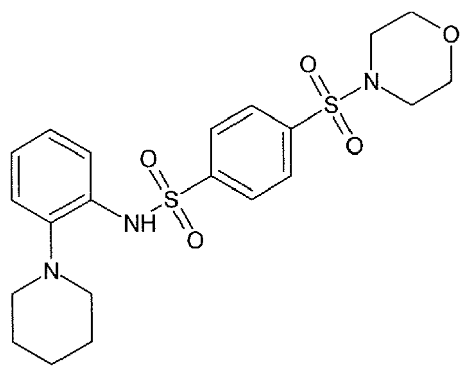 | $^1$H NMR ($CDCl_3$) δ ppm 1.49 (m, 2 H) 1.55 - 1.63 (m, 4 H) 2.36 - 2.43 (m, 4 H) 2.90 - 2.95 (m, 4 H) 3.64 - 3.70 (m, 4 H) 6.99 - 7.11 (br s, 3 H) 7.53 - 7.59 (m, 1 H) 7.76 (d, J=8.43 Hz, 2 H) 7.95 (d, J=8.43 Hz, 2 H) 8.18 (NH) |

(continued)

| Example | Structure | NMR |
|---|---|---|
| 32 | 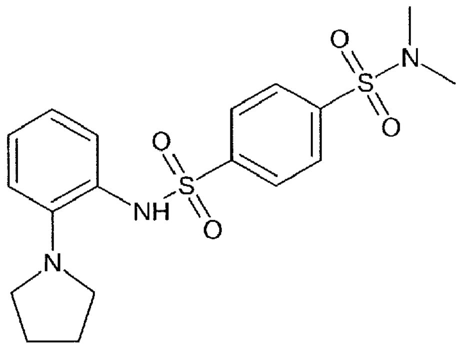 | $^1$H NMR ($CDCl_3$) δ ppm 1.88 (dt, J=6.37, 3.23 Hz, 4 H) 2.70 (s, 6 H) 2.77 (br t, J=6.37 Hz, 4 H) 6.99 - 7.09 (br s, 3 H) 7.44 - 7.50 (m, 1 H) 7.81 (d, J=8.43 Hz, 2 H) 7.95 (d, J=8.43 Hz, 2 H) |
| 33 | 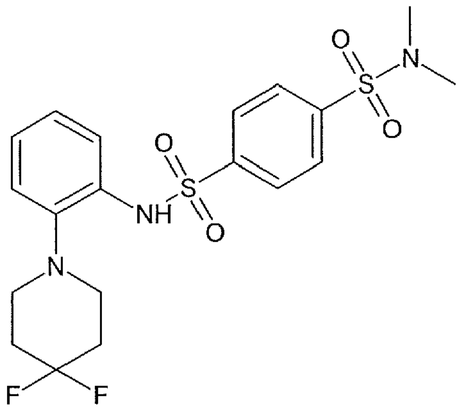 | $^1$H NMR ($CDCl_3$) δ ppm 2.01 - 2.14 (m, 4 H) 2.67 (m, 4 H) 2.71 (s, 6 H) 7.04 - 7.19 (br s, 3 H) 7.54 (d, J=8.25 Hz, 1 H) 7.84 (d, J=8.43 Hz, 2 H) 7.95 (NH) 7.99 (d, J=8.43 Hz, 2 H) |
| 34 | 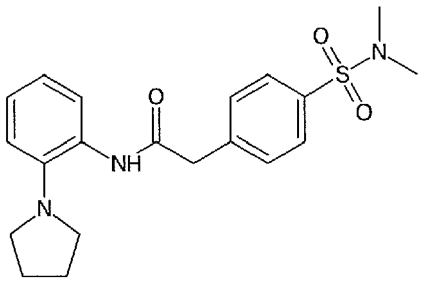 | $^1$H NMR ($CDCl_3$) δ ppm 1.76 (br s, 4 H) 2.70 (s, 6 H) 2.77 (br s, 4 H) 3.82 (s, 2 H) 6.97 - 7.10 (br s, 3 H) 7.53 (br d, J=8.06 Hz, 2 H) 7.77 (d, J=8.06 Hz, 2 H) 8.19 - 8.26 (m, 1 H) 8.31 (NH) |
| 35 | 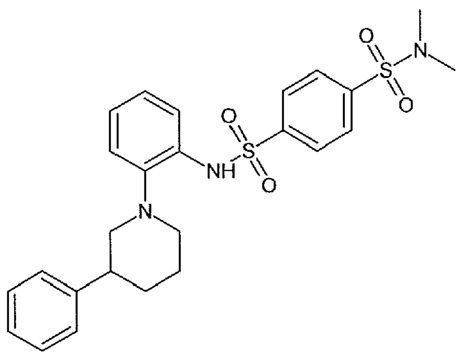 | $^1$H NMR ($CDCl_3$) δ ppm 1.49 - 1.65 (m, 2 H) 1.70 - 1.91 (m, 2 H) 2.00 - 2.08 (m, 1 H) 2.47 - 2.53 (m, 1 H) 2.58 - 2.63 (br s, 8 H) 2.84 - 2.94 (m, 1 H) 7.02 - 7.07 (m, 1 H) 7.09 - 7.15 (m, 2 H) 7.19 (br d, *J*=7.15 Hz, 2 H) 7.22 - 7.26 (m, 1 H) 7.28 - 7.36 (m, 2 H) 7.58 - 7.64 (m, 1 H) 7.70 (d, *J*=8.34 Hz, 2 H) 7.95 (d, *J*=8.34 Hz, 2 H) 8.17 (NH) |

(continued)

| Example | Structure | NMR |
|---|---|---|
| 36 | 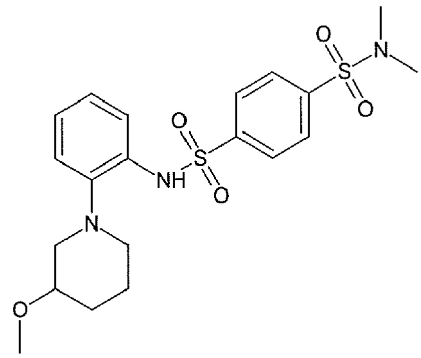 | $^1$H NMR ($CDCl_3$) δ ppm 1.49 - 1.65 (m, 2 H) 1.82 (m, 2 H) 2.52 - 2.63 (m, 3 H) 2.67 (br s, 7 H) 3.38 (m, 1 H) 3.46 (s, 3 H) 6.91 - 7.09 (br s, 3 H) 7.60 (d, *J*=7.88 Hz, 1 H) 7.78 (d, *J*=8.25 Hz, 2 H) 7.97 (d, *J*=8.25 Hz, 2 H) 8.76 (NH) |
| 37 | 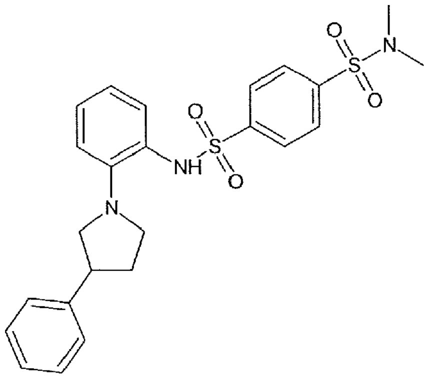 | $^1$H NMR ($CDCl_3$) δ ppm 2.02 - 2.13 (m, 1 H) 2.33 - 2.44 (m, 1 H) 2.64 (s, 6 H) 2.68 - 2.75 (m, 1 H) 3.00 - 3.14 (m, 3 H) 3.37 - 3.47 (m, 1 H) 7.01 - 7.15 (br s, 3 H) 7.23 - 7.31 (br s, 3 H) 7.34 - 7.41 (m, 2 H) 7.51 (dd, *J*=7.84, 1.24 Hz, 1 H) 7.70 - 7.75 (m, 2 H) 7.78 - 7.83 (m, 2 H) |
| 38 | 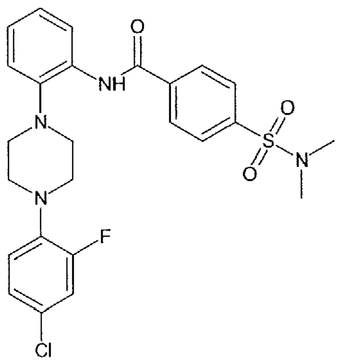 | $^1$H NMR ($CDCl_3$) δ ppm 2.75 (s, 6 H) 3.11 (br t, *J*=4.44 Hz, 4 H) 3.25 (m, 4 H) 6.93 (t, 7=8.98 Hz, 1 H) 7.06 - 7.12 (m, 2 H) 7.14 - 7.20 (m, 1 H) 7.24 - 7.34 (m, 2 H) 7.92 (d, *J*=8.34 Hz, 2 H) 8.08 (d, *J*=8.34 Hz, 2 H) 8.56 (d, *J*=7.61 Hz, 1 H) 9.58 (NH) |
| 39 | 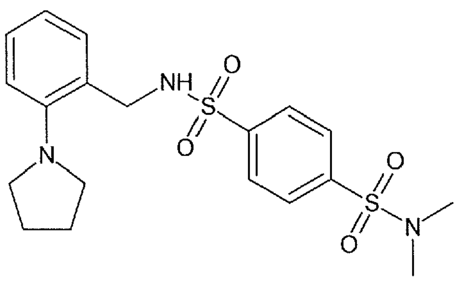 | $^1$H NMR ($CDCl_3$) δ ppm 1.86 (br t, J=6.19 Hz, 4 H) 2.64 (s, 6 H) 2.93 (br t, J=6.19 Hz, 4 H) 4.25 (s, 2 H) 6.68 - 6.76 (m, 1 H) 6.81 (d, J=8.00 Hz, 1 H) 6.86 (br d, J=7.25 Hz, 1 H) 7.00 - 7.09 (m, 1 H) 7.65 (d, J=8.38 Hz, 2 H) 7.75 (d, J=8.38 Hz, 2 H) |

(continued)

| Example | Structure | NMR |
|---|---|---|
| 40 | 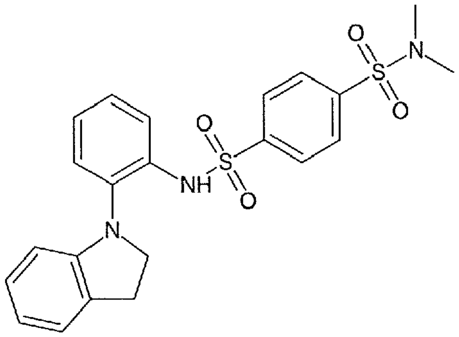 | $^{1}H$ NMR ($CDCl_3$) δ ppm 2.71 (s, 6 H) 3.03 - 3.15 (t, 2 H) 3.36 - 3.45 (t, 2 H) 5.68 (d, J=7.79 Hz, 1 H) 6.74 - 6.81 (m, 1 H) 6.87 - 6.93 (m, 1 H) 7.11 - 7.20 (m, 3 H) 7.23 - 7.31 (m, 1 H) 7.65 - 7.69 (m, 1 H) 7.73 (d, J=8.34 Hz, 1 H) 7.80 (d, J=8.52 Hz, 2 H) 7.92 (d, J=8.43 Hz, 2 H) |
| 41 | 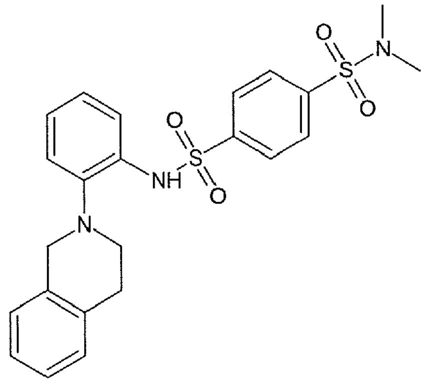 | $^{1}H$ NMR ($CDCl_3$) δ ppm 2.72 (s, 6 H) 2.84 - 2.92 (m, 2 H) 2.92 - 2.98 (m, 2 H) 3.70 (s, 2 H) 6.92 (br d, J=7.15 Hz, 1 H) 7.10 - 7.25 (m, 6 H) 7.63 - 7.69 (m, 1 H) 7.83 (d, J=8.34 Hz, 2 H) 7.95 (d, J=8.43 Hz, 2 H) 8.17 (NH) |
| 42 | 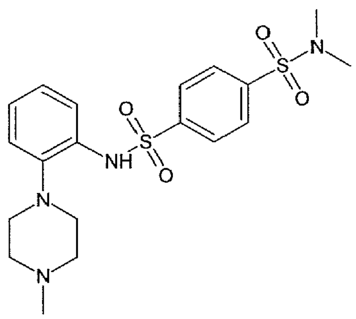 | $^{1}H$ NMR (DMSO-d6) δ ppm 2.30 (br s, 4 H) 2.48 (br s, 3 H) 2.55 (br s, 4 H) 2.59 (s, 6 H) 6.97 - 7.03 (m, 1 H) 7.07 (d, J=4.03 Hz, 2 H) 7.19 (d, J=7.70 Hz, 1 H) 7.86 - 7.91 (m, 2 H) 7.94 - 7.98 (m, 2 H) 8.24 (NH) |
| 43 | 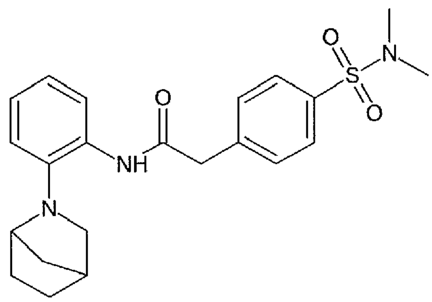 | $^{1}H$ NMR ($CDCl_3$) δ ppm 1.31 - 1.39 (m, 2 H) 1.42 - 1.52 (m, 2 H) 1.61 (br s, 2 H) 2.46 (br s, 1 H) 2.68 (br s, 1 H) 2.71 (s, 6 H) 2.90 (dt, J=8.75, 2.96 Hz, 1 H) 3.37 (br s, 1 H) 3.82 (s, 2 H) 6.94 - 7.03 (br s, 3 H) 7.54 (d, J=8.16 Hz, 2 H) 7.78 (d, J=8.16 Hz, 2 H) 8.11 (NH) 8.13 - 8.18 (m, 1 H) |

(continued)

| Example | Structure | NMR |
|---|---|---|
| 44 | 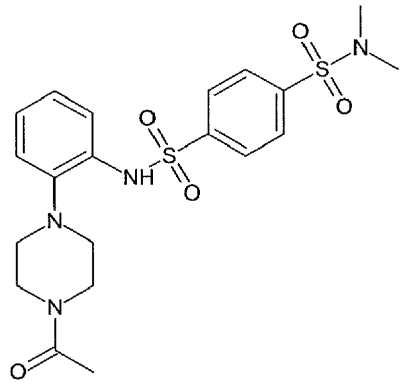 | $^1H$ NMR ($CDCl_3$) δ ppm 2.13 (s, 3 H) 2.52 - 2.60 (m, 4 H) 2.72 (s, 6 H) 3.51 - 3.59 (m, 2 H) 3.70 (m, 2 H) 7.08 (d, J=3.48 Hz, 2 H) 7.13 - 7.19 (m, 1 H) 7.55 (d, J=7.97 Hz, 1 H) 7.84 (d, J=8.43 Hz, 2 H) 7.99 (d, J=8.43 Hz, 2 H) |
| 45 | 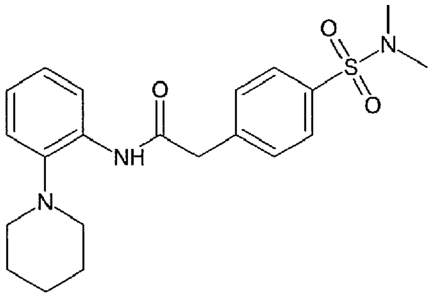 | $^1H$ NMR ($CDCl_3$) δ ppm 1.47 (br s, 5 H) 2.62 (m, 4 H) 2.70 (s, 6 H) 3.83 (s, 2 H) 6.96 - 7.05 (m, 1 H) 7.06 - 7.12 (m, 2 H) 7.54 (d, J=8.106 Hz, 2 H) 7.78 (d, J=8.06 Hz, 2 H) 8.35 (br d, J=7.79 Hz, 1 H) 8.67 (NH) |
| 47 | 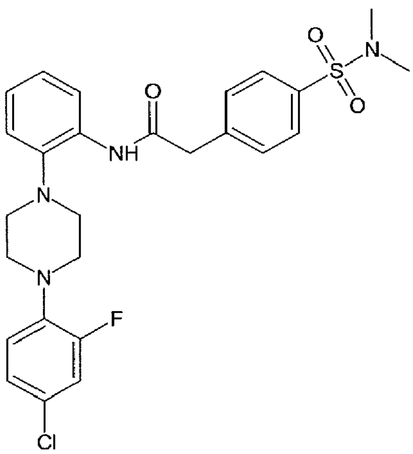 | $^1H$ NMR ($CDCl_3$) δ ppm 2.70 (s, 6 H) 2.87 (m, 4 H) 2.93 (m, 4 H) 3.86 (s, 2 H) 6.95 (t, J=8.94 Hz, 1 H) 7.02 - 7.11 (m, 2 H) 7.14 (br d, J=10.63 Hz, 1 H) 7.19 (t, J=7.33 Hz, 2 H) 7.55 (d, J=8.06 Hz, 2 H) 7.80 (d, J=8.16 Hz, 2 H) 8.41 (br d, J=8.06 Hz, 1 H) 8.65 (NH |
| 48 | 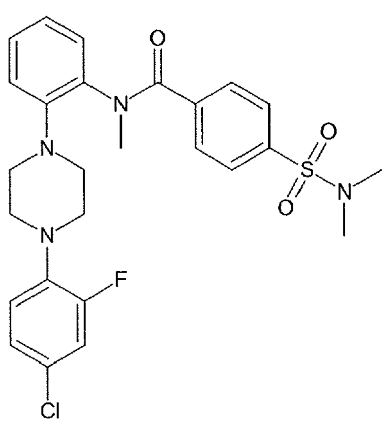 | $^1H$ NMR ($CDCl_3$) δ ppm 2.41 - 2.53 (m, 2 H) 2.59 (s, 6 H) 3.01 (br t, J=6.37 Hz, 4 H) 3.11 - 3.20 (m, 2 H) 3.53 (s, 3 H) 6.89 (br s 2 Hz, 2 H) 7.02 - 7.11 (m, 2 H) 7.13 (dd, J=7.56, 0.96 Hz, 1 H) 7.18 - 7.23 (m, 1 H) 7.30 (dd, J=7.79, 1.47 Hz, 1 H) 7.51 (br s, 4 H) |

(continued)

| Example | Structure | NMR |
|---|---|---|
| 49 | 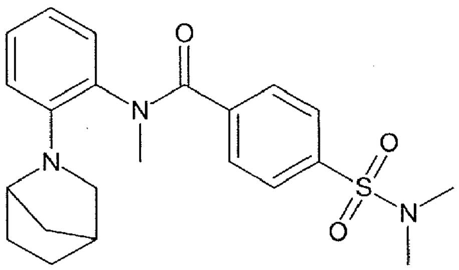 | $^1H$ NMR ($CDCl_3$) δ ppm 1.35 (m, 1 H) 1.51 - 1.70 (br s, 4 H) 1.78 - 1.87 (m, 1 H) 2.45 (d, *J*=8.06 Hz, 1 H) 2.57 (s, 6 H) 3.34 - 3.42 (m, 2 H) 3.47 (s, 3 H) 3.57 (dt, *J*=7.81, 2.97 Hz, 1 H) 6.34 (d, *J*=8.25 Hz, 1 H) 6.70 (t, *J*=7.51 Hz, 1 H) 6.94 - 7.00 (m, 1 H) 7.15 (dd, *J*=7.79, 1.37 Hz, 1 H) 7.33 (d, *J*=8.34 Hz, 2 H) 7.45 (d, *J*=8.34 Hz, 2 H) |
| 50 | 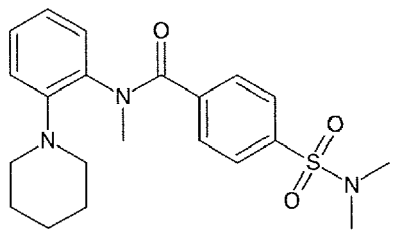 | $^1H$ NMR ($CDCl_3$) δ ppm 1.44 - 1.66 (br s, 6 H) 2.23 (m, 2 H) 2.58 (s, 6 H) 2.75 (m, 2 H) 3.51 (s, 3 H) 6.78 (br d, J=7.88 Hz, 1 H) 7.00 - 7.08 (m, 1 H) 7.10 - 7.17 (m, 1 H) 7.22 - 7.28 (m, 1 H) 7.49 (br s, 4 H) |
| 51 | 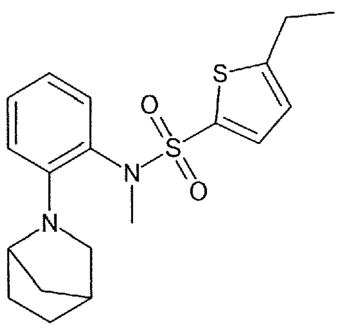 | $^1H$ NMR ($CDCl_3$) δ ppm 1.30 - 1.39 (br s, 5 H) 1.41 - 1.49 (m, 2 H) (m,2 H) 2.57 (br s, 2 H) 2.75 (br d, J=8.71 Hz, 1 H) 2.85 - 2.95 (m, 2 H) 3.20 (s, 3 H) 3.72 (m, 1 H) 6.42 - 6.53 (m, 2 H) 6.70 (br d, J=8.43 Hz, 1 H) 6.80 (br d, J=3.39 Hz, 1 H) 7.06 - 7.14 (m, 1 H) 7.21 (d, J=3.57 Hz, 1 H) |
| 52 | 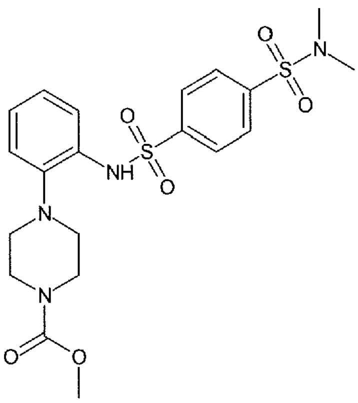 | $^1H$ NMR ($CDCl_3$) δ ppm 2.50 (m, 4 H) 2.71 (s, 6 H) 3.60 (m, 4 H) 4.11 (s, 3 H) 7.04 - 7.12 (m, 2 H) 7.14 - 7.30 (m, 1 H) 7.60 (d, *J*=7.80 Hz, 1 H) 7.89 (d, *J*=8.44, 2 H) 8.05 (d, *J*=8.44 Hz, 2 H) 8.10 (NH) |

(continued)

| Example | Structure | NMR |
|---|---|---|
| 53 | 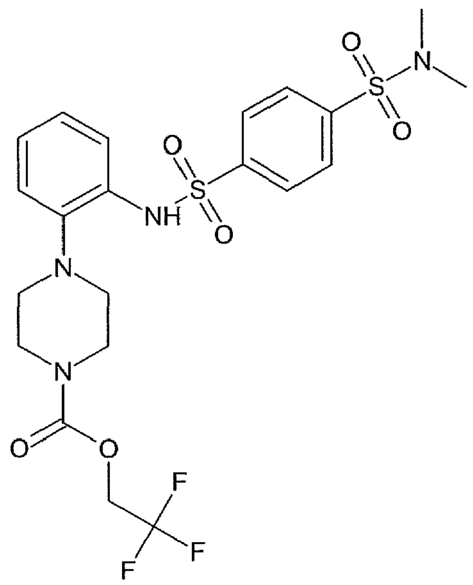 | $^1$H NMR ($CDCl_3$) δ ppm 2.51 (br s, 4 H) 2.65 (s, 6 H) 3.54 (br s, 4 H) 4.45 (q, J=8.50 Hz, 2 H) 6.98 - 7.06 (m, 2 H) 7.06 - 7.14 (m, 1 H) 7.48 (d, J=7.75 Hz, 1 H) 7.78 (d, J=8.50 Hz, 2 H) 7.93 (d, J=8.50 Hz, 2 H) |
| 54 | 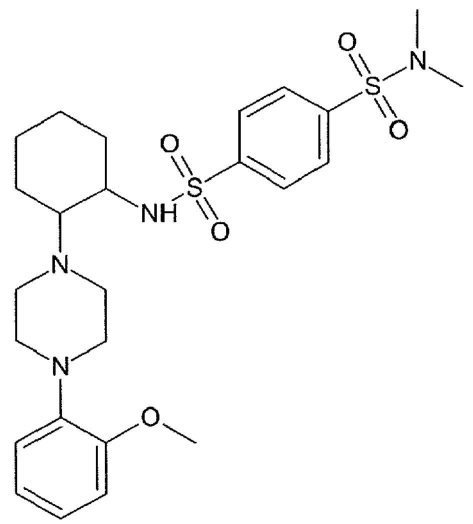 | $^1$H NMR ($CDCl_3$) δ ppm 1.08 - 1.33 (br s, 6 H) 1.68 (br s, 1 H) 1.80 (br s, 1 H) 1.90 (br s, 1 H) 2.25 - 2.41 (br s, 3 H) 2.49 (m, 2 H) 2.72 (s, 6 H) 2.71 - 2.72 (m, 2 H) 2.77 - 2.85 (m, 2 H) 3.02 (m, 2 H) 3.83 (s, 3 H) 6.85 (br d, J=7.88 Hz, 1 H) 6.89 - 6.97 (m, 2 H) 6.99 - 7.06 (m, 1 H) 7.90 (br d, J=8.25 Hz, 2 H) 8.06 (br d, J=8.34 Hz, 2 H) |
| 55 | 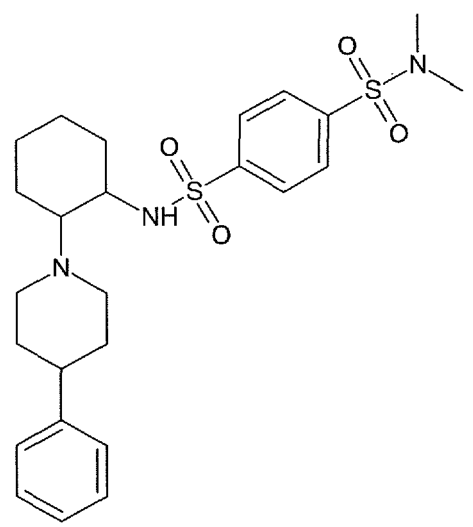 | $^1$H NMR ($CDCl_3$) δ ppm 1.06 - 1.39 (m, 6 H) 1.67 (br d, J=12.10 Hz, 2 H) 1.76 - 1.92 (m, 4 H) 2.08 (br s, 1 H) 2.25 - 2.47 (m, 3 H) 2.62 - 2.68 (m, 2 H) 2.70 (s, 6 H) 2.80 (br d, J=6.42 Hz, 1 H) 7.16 - 7.24 (br s, 3 H) 7.29 - 7.35 (m, 2 H) 7.90 (d, J=8.34 Hz, 2 H) 8.07 (d, J=8.34 Hz, 2 H) |

(continued)

| Example | Structure | NMR |
|---|---|---|
| 56 | 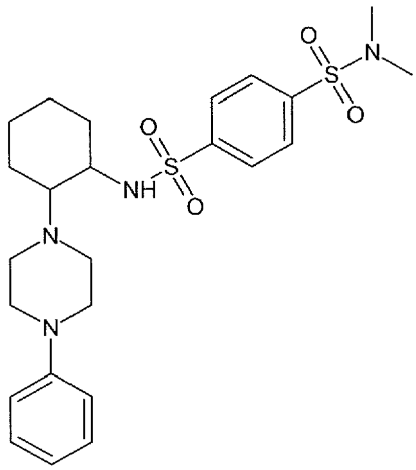 | $^1$H NMR ($CDCl_3$) δ ppm 1.07 - 1.30 (br s, 4 H) 1.68 (br d, J=7.51 Hz, 1 H) 1.78 (br s, 1 H) 1.87 (br d, J=11.82 Hz, 1 H) 2.25 - 2.40 (m, 2 H) 2.42 (br s, 4 H) 2.74 (s, 6 H) 2.82 - 2.90 (m, 1 H) 2.99 (br s, 2 H) 3.03 - 3.13 (m, 2 H) 6.84 - 6.91 (br s, 3 H) 7.27 (br t, J=7.79 Hz, 2 H) 7.92 (d, J=8.25 Hz, 2 H) 8.06 (d, J=8.25 Hz, 2 H) |
| 57 | 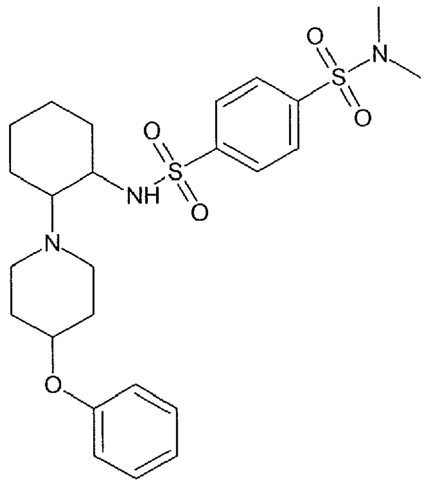 | $^1$H NMR ($CDCl_3$) δ ppm 1.12 - 1.31 (m, 4 H) 1.53 - 1.93 (m, 6 H) 2.12 (br t, J=8.16 Hz, 1 H) 2.21 - 2.30 (m, 2 H) 2.37 (br d, J=10.63 Hz, 2 H) 2.48 - 2.57 (m, 1 H) 2.71 (s, 6 H) 2.74 - 2.84 (m, 2 H) 4.24 (m, 1 H) 6.86 (br d, J=8.06 Hz, 2 H) 6.93 (t, J=7.33 Hz, 1 H) 7.26 (t, J=7.93 Hz, 2 H) 7.91 (d, J=8.34 Hz, 2 H) 8.07 (d, J=8.34 Hz, 2 H) |
| 58 | 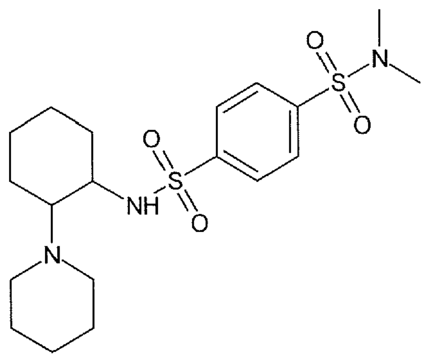 | $^1$H NMR ($CDCl_3$) δ ppm 0.97 - 1.27 (m, 4 H) 1.30 - 1.50 (m, 6 H) 1.64 (br d, J=9.16 Hz, 1 H) 1.76 (br t, J=13.38 Hz, 2 H) 2.13 (br s, 5 H) 2.37 (br d, J=12.83 Hz, 1 H) 2.66 - 2.72 (m, 1 H) 2.73 (s, 6 H) 7.90 (d, J=8.43 Hz, 2 H) 8.04 (d, J=8.43 Hz, 2 H) |

(continued)

| Example | Structure | NMR |
|---|---|---|
| 59 | 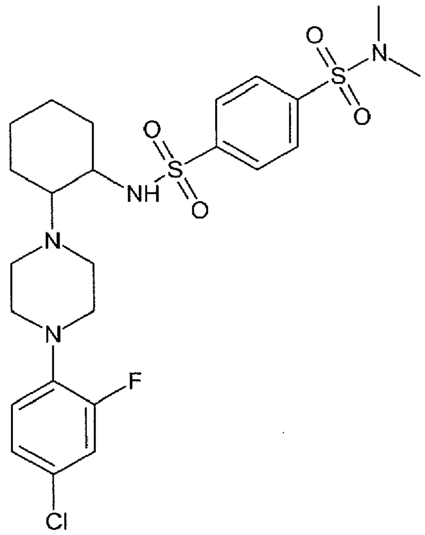 | $^1$H NMR ($CDCl_3$) δ ppm 1.08 - 1.29 (m, 4 H) 1.68 (br s, 1 H) 1.79 (br s, 1 H) 1.89 (br d, J=11.00 Hz, 1 H) 2.23 - 2.35 (m, 2 H) 2.45 (br s, 4 H) 2.75 (s, 6 H) 2.83 - 3.01 (m, 5 H) 6.83 (t, J=8.84 Hz, 1 H) 7.00 - 7.09 (br s, 2 H) 7.92 (d, J=8.43 Hz, 2 H) 8.07 (d, J=8.43 Hz, 2 H) |
| 61 | 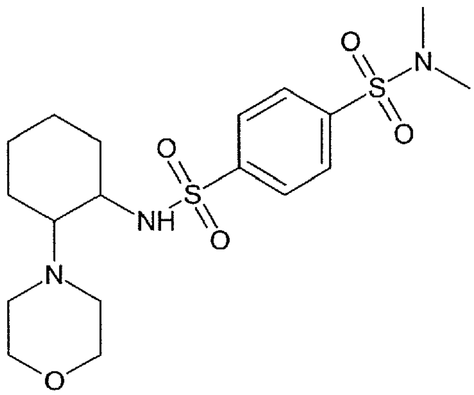 | $^1$H NMR ($CDCl_3$) δ ppm 1.02 - 1.24 (m, 2 H) 1.64 (m 1 H) 1.73 - 1.88 (br s, 2 H) 2.06 - 2.34 (m, 3 H) 2.73 (s, 6 H) 3.18 (m, 3 H) 3.44 - 3.61 (br s, 4 H) 3.75 (m, 3 H) 7.89 (d, *J*=8.25 Hz, 2 H) 8.04 (d, *J*=8.25 Hz, 2 H) |
| 62 | 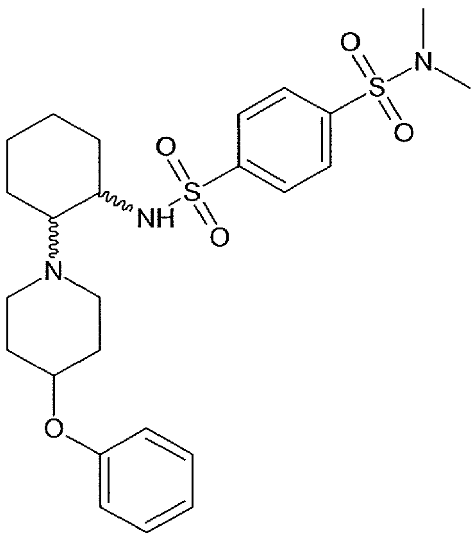 | $^1$H NMR ($CDCl_3$) δ ppm 1.12 - 1.31 (m, 4 H) 1.53 - 1.93 (m, 6 H) 2.12 (br t, J=8.16 Hz, 1 H) 2.21 - 2.30 (m, 2 H) 2.37 (br d, J=10.63 Hz, 2 H) 2.48 - 2.57 (m, 1 H) 2.71 (s, 6 H) 2.74 - 2.84 (m, 2 H) 4.24 (m, 1 H) 6.86 (br d, J=8.06 Hz, 2 H) 6.93 (t, J=7.33 Hz, 1 H) 7.26 (t, J=7.93 Hz, 2 H) 7.91 (d, J=8.34 Hz, 2 H) 8.07 (d, J=8.34 Hz, 2 H) |

(continued)

| Example | Structure | NMR |
|---|---|---|
| 63 | 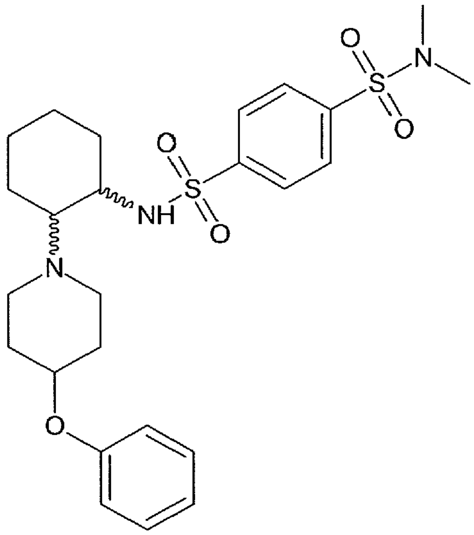 | $^1$H NMR ($CDCl_3$) δ ppm 1.12 - 1.31 (m, 4 H) 1.53 - 1.93 (m, 6 H) 2.12 (br t, J=8.16 Hz, 1 H) 2.21 - 2.30 (m, 2 H) 2.37 (br d, J=10.63 Hz, 2 H) 2.48 - 2.57 (m, 1 H) 2.71 (s, 6 H) 2.74 - 2.84 (m, 2 H) 4.24 (m, 1 H) 6.86 (br d, J=8.06 Hz, 2 H) 6.93 (t, J=7.33 Hz, 1 H) 7.26 (t, J=7.93 Hz, 2 H) 7.91 (d, J=8.34 Hz, 2 H) 8.07 (d, J=8.34 Hz, 2 H) |
| 64 | 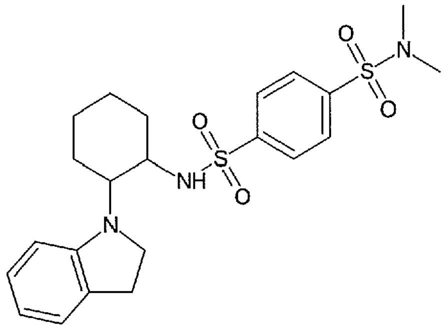 | $^1$H NMR ($CDCl_3$) δ ppm 1.18 - 1.38 (br s, 4 H) 1.66 - 1.76 (m, 2 H) 1.76 - 1.81 (m, 1 H) 2.35 (br d, *J*=11.82 Hz, 1 H) 2.59 - 2.69 (m, 2 H) 2.74 (s, 6 H) 2.77 - 2.86 (m, 2 H) 3.20 (br s, 2 H) 6.37 (br d, *J*=5.87 Hz, 1 H) 6.67 (t, *J*=7.33 Hz, 1 H) 6.98 - 7.08 (m, 2 H) 7.86 (d, *J*=8.25 Hz, 2 H) 7.99 (d, *J*=8.25 Hz, 2 H) |
| 65 | 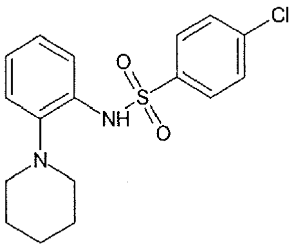 | $^1$H NMR ($CDCl_3$) δ ppm 1.46 - 1.58 (m, 2 H) 1.62 (quin, *J*=5.34 Hz, 4 H) 2.39 - 2.49 (m, 4 H) 6.97 - 7.12 (br s, 3 H) 7.36 (d, *J*=8.61 Hz, 2 H) 7.55 - 7.60 (m, 1 H) 7.74 (d, *J*=8.52 Hz, 2 H) 8.12 (NH) |
| 66 | 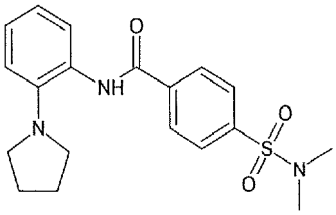 | $^1$H NMR ($CDCl_3$) δ ppm 1.97 (m, 4 H) 2.74 (s, 6 H) 3.06 (m, 4 H) 7.06 - 7.15 (m, 2 H) 7.16 - 7.23 (m, 1 H) 7.88 (d, *J*=8.25 Hz, 2 H) 8.03 (d, *J*=8.25 Hz, 2 H) 8.35 (br d, *J*=6.69 Hz, 1 H) 9.26 (NH) |
| 67 | 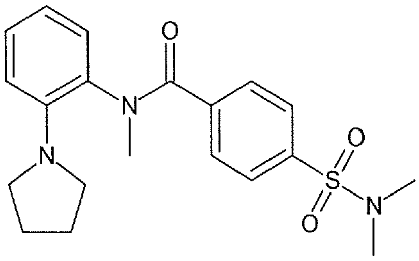 | $^1$H NMR ($CDCl_3$) δ ppm 1.71 - 1.85 (m, 2 H) 1.96 (m, 2 H) 2.57 (s, 6 H) 2.73 (br s, 2 H) 3.15 - 3.28 (m, 2 H) 3.46 (s, 3 H) 6.48 (br d, *J*=7.88 Hz, 1 H) 6.75 (t, *J*=7.51 Hz, 1 H) 7.02 (br t, *J*=7.61 Hz, 1 H) 7.11 (d, *J*=7.79 Hz, 1 H) 7.35 (br d, *J*=7.79 Hz, 2 H) 7.47 (br d, *J*=7.79 Hz, 2 H) |

(continued)

| Example | Structure | NMR |
|---|---|---|
| 68 | 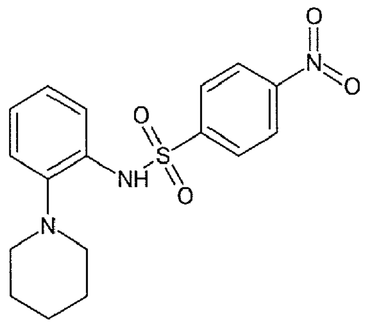 | $^1$H NMR ($CDCl_3$) δ ppm 1.47 (m, 2 H) 1.57 (quin, *J*=5.44 Hz, 4 H) 2.32 - 2.43 (m, 4 H) 6.90 - 7.08 (m, 3 H) 7.45 - 7.55 (m, 1 H) 7.87 - 7.97 (m, 2 H) 8.14 - 8.23 (m, 2 H) |
| 69 | 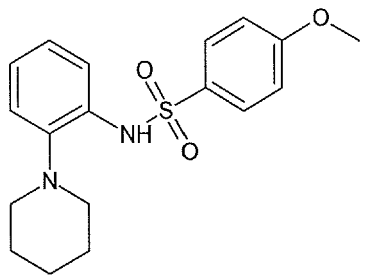 | $^1$H NMR ($CDCl_3$) δ ppm 1.55 (m, 2 H) 1.65 (m, 4 H) 2.43 - 2.53 (m, 4 H) 3.81 (s, 3 H) 6.82 - 6.90 (m, 2 H) 6.99 - 7.05 (m, 1 H) 7.06 - 7.12 (m, 2 H) 7.59 - 7.64 (m, 1 H) 7.72 - 7.81 (m, 2 H) 8.07 (NH) |
| 70 | 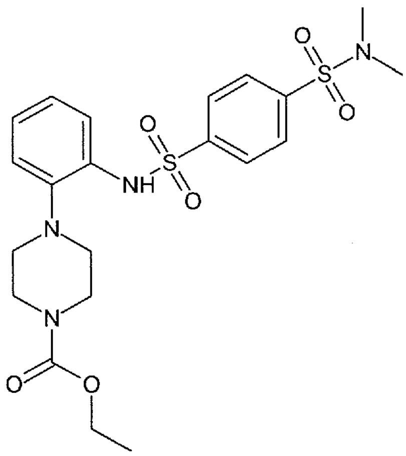 | $^1$H NMR ($CDCl_3$) δ ppm 1.29 (t, *J*=7.07, 3 H) 2.53 (m, 4 H) 2.73 (s, 6 H) 3.57 (m, 4 H) 4.18 (q, *J*=7.07 Hz, 2 H) 7.04 - 7.14 (m, 2 H) 7.14 - 7.21 (m, 1 H) 7.58 (d, *J*=7.88 Hz, 1 H) 7.85 (d, *J*=8.50, 2 H) 8.00 (d, *J*=8.50 Hz, 2 H) 8.08 (NH) |
| 71 | 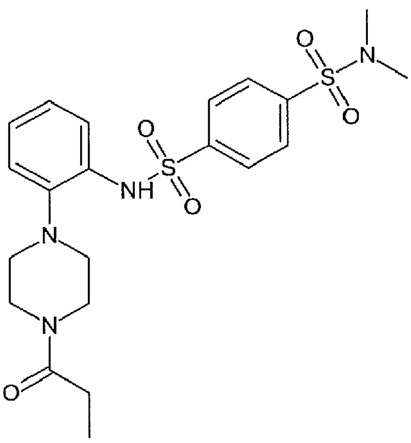 | $^1$H NMR (CDCl3) δ ppm 1.10 (t, *J*=7.44 Hz, 3 H) 2.30 (q, *J*=7.44 Hz, 2 H) 2.48 (m, 4 H) 2.65 (s, 6 H) 3.48 (m, 2 H) 3.64 (m, 2 H) 7.00 - 7.05 (m, 2 H) 7.07 - 7.13 (m, 1 H) 7.49 (d, *J*=8.00 Hz, 1 H) 7.75 - 7.81 (m, 2 H) 7.90 - 7.95 (m, 2 H) 7.98 (NH) |

(continued)

| Example | Structure | NMR |
|---|---|---|
| 72 | 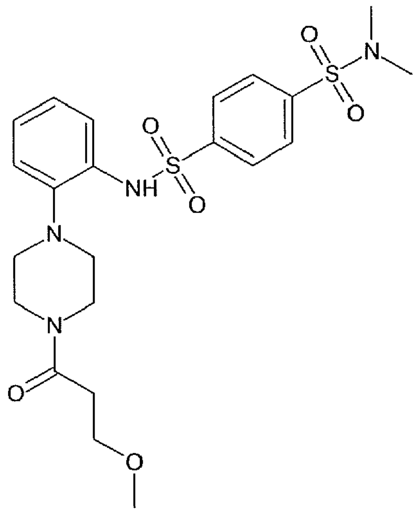 | $^{1}H$ NMR ($CDCl_3$) δ ppm 2.49 (m, 4 H) 2.56 (t, *J*=6.38 Hz, 2 H) 2.65 (s, 6 H) 3.29 (s, 3 H) 3.49 - 3.55 (m, 2 H) 3.65 (t, *J*=6.32 Hz, 4 H) 7.01 (m, 2 H) 7.06 - 7.12 (m, 1 H) 7.48 (d, *J*=8.00 Hz, 1 H) 7.76 - 7.80 (m, 2 H) 7.90 - 7.96 (m, 2 H) 7.99 (NH) |
| 73 | 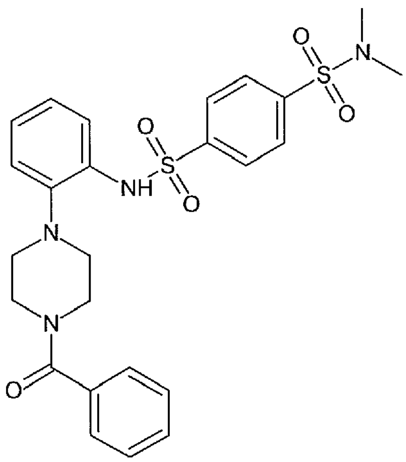 | $^{1}H$ NMR ($CDCl_3$) δ ppm 2.62 (m, 4 H) 2.71 (s, 6 H) 3.48 - 3.99 (m, 4 H) 7.07 - 7.20 (m, 3 H) 7.44 (m, 5 H) 7.56 (d, *J*=8.00 Hz, 1 H) 7.85 (d, *J*=8.38 Hz, 2 H) 8.01 (d, *J*=8.38 Hz, 2 H) 8.09 (NH) |
| 74 | 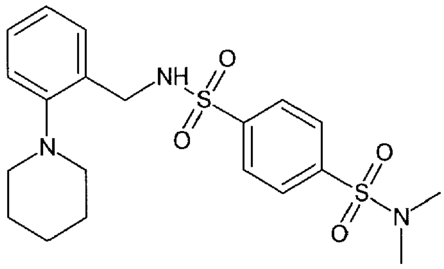 | $^{1}H$ NMR ($CDCl_3$) δ ppm 1.58 (m, 2 H) 1.65 - 1.80 (m, 4 H) 2.70 (s, 6 H) 2.74 - 2.79 (m, 4 H) 4.32 (s, 2 H) 6.88 - 6.98 (m, 1 H) 7.00 (d, *J*=7.63 Hz, 2 H) 7.12 - 7.22 (m, 1 H) 7.32 (NH) 7.69 - 7.77 (m, 2 H) 7.84 - 7.91 (m, 2 H) |

(continued)

| Example | Structure | NMR |
|---|---|---|
| 75 | 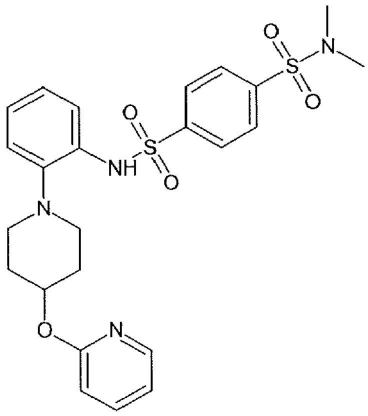 | $^1H$ NMR ($CDCl_3$) δ ppm 1.90 (m, 2 H) 2.07 - 2.16 (m, 2 H) 2.53 - 2.63 (m, 2 H) 2.69 (br s, 8 H) 5.21 (dt, *J*=7.66, 3.86 Hz, 1 H) 6.75 (d, *J*=8.25 Hz, 1 H) 6.85 - 6.90 (m, 1 H) 7.06 - 7.21 (m, 3 H) 7.56 - 7.67 (m, 2 H) 7.83 (d, *J*=8.50 Hz, 2 H) 7.99 (d, *J*=8.50 Hz, 2 H) 8.14 (dd, *J*=5.07, 1.31 Hz, 1 H) |
| 76 | 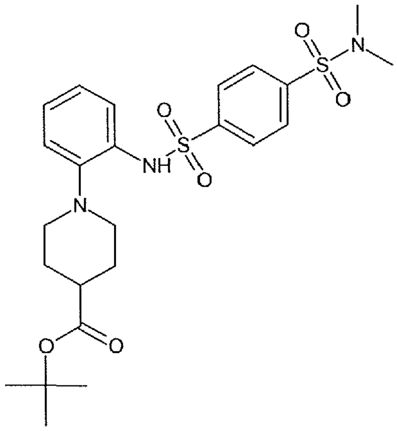 | $^1H$ NMR ($CDCl_3$) δ ppm 1.49 (s, 9 H) 1.71 - 1.83 (m, 2 H) 1.94 (br dd, *J*=13.07, 2.94 Hz, 2 H) 2.31 (tt, *J*=10.90, 3.99 Hz, 1 H) 2.52 (dd, *J*=7.57, 2.56 Hz, 4 H) 2.71 (s, 6 H) 7.03 - 7.16 (m, 3 H) 7.58 - 7.62 (m, 1 H) 7.83 (d, *J*=8.50 Hz, 2 H) 7.98 (d, *J*=8.50 Hz, 2 H) 8.09 (NH) |
| 77 | 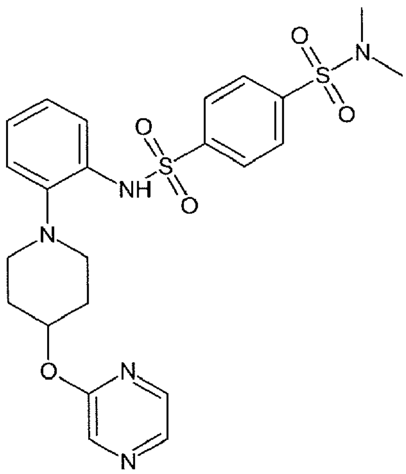 | $^1H$ NMR ($CDCl_3$) δ ppm 1.85 - 1.96 (m, 2 H) 2.06 - 2.16 (m, 2 H) 2.51 - 2.61 (m, 2 H) 2.70 (br s, 8 H) 5.19 (dt, *J*=7.47, 3.71 Hz, 1 H) 7.06 - 7.20 (m, 3 H) 7.61 (dd, *J*=7.82, 1.19 Hz, 1 H) 7.83 (d, *J*=8.50 Hz, 2 H) 7.99 (d, *J*=8.50 Hz, 2 H) 8.06 (dd, *J*=2.63, 1.38 Hz, 1 H) 8.12 (d, *J*=2.75 Hz, 1 H) 8.24 (d, *J*=0.88 Hz, 1 H) |

(continued)

| Example | Structure | NMR |
|---|---|---|
| 78 | 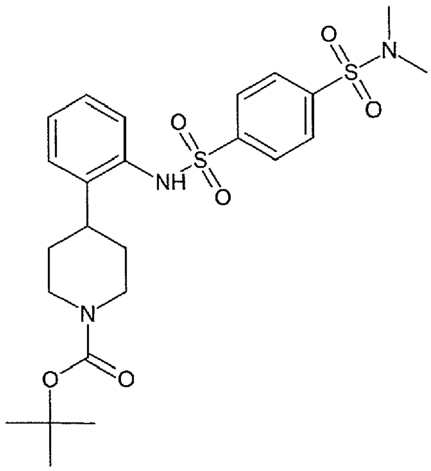 | $^1$H NMR ($CDCl_3$) δ ppm 1.47 (br s, 13 H) 2.70 (br s, 2 H) 2.75 (s, 6 H) 2.87 - 2.98 (m, 1 H) 4.16 (br s, 2 H) 6.96 (br d, *J*=7.88 Hz, 1 H) 7.11 (br t, *J*=7.19 Hz, 1 H) 7.19 - 7.31 (m, 2 H) 7.84 - 7.94 (m, 4 H) |
| 79 | 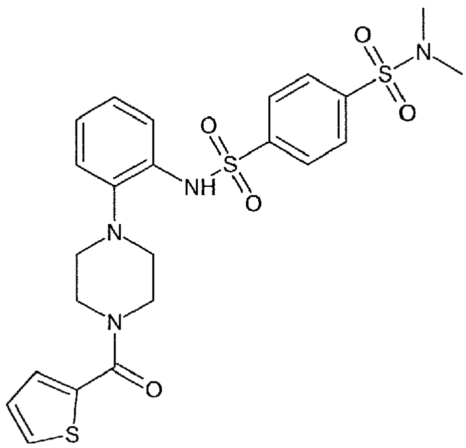 | $^1$H NMR ($CDCl_3$) δ ppm 2.64 (br t, *J*=4.57 Hz, 4 H) 2.71 (s, 6 H) 3.85 (m, 4 H) 7.04 - 7.22 (m, 4 H) 7.32 (d, *J*=3.50 Hz, 1 H) 7.48 (d, *J*=5.00 Hz, 1 H) 7.55 (d, *J*=7.88 Hz, 1 H) 7.85 (d, *J*=8.38 Hz, 2 H) 8.01 (br d, *J*=8.38 Hz, 2 H) 8.08 (NH) |
| 80 | 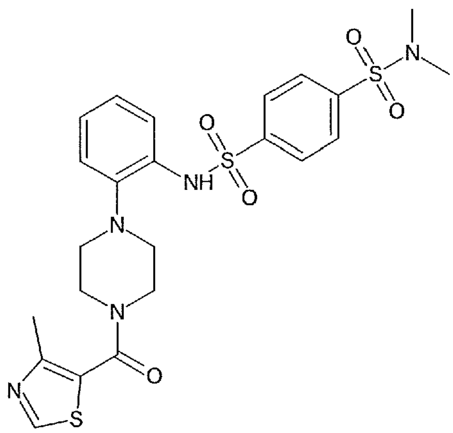 | $^1$H NMR ($CDCl_3$) δ ppm 2.53 (s, 3 H) 2.65 (br t, *J*=4.63 Hz, 4 H) 2.72 (s, 6 H) 3.73 (m, 4 H) 7.06 - 7.20 (m, 4 H) 7.53 (d, *J*=7.88 Hz, 1 H) 7.85 (d, *J*=8.38 Hz, 2 H) 8.01 (d, *J*=8.38 Hz, 3 H) 8.78 (NH) |

(continued)

| Example | Structure | NMR |
| --- | --- | --- |
| 81 | 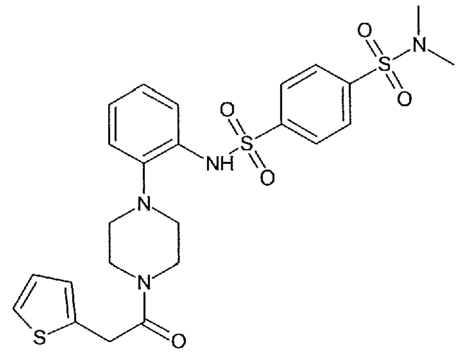 | $^1$H NMR ($CDCl_3$) δ ppm 2.41 - 2.48 (m, 2 H) 2.56 (br d, *J*=4.38 Hz, 2 H) 2.72 (s, 6 H) 3.59 (m, 2 H) 3.73 (m, 2 H) 3.95 (s, 2 H) 6.92 (d, *J*=3.25 Hz, 1 H) 6.97 (dd, *J*=5.00, 3.50 Hz, 1 H) 7.02 - 7.11 (m, 2 H) 7.15 (td, *J*=7.66, 1.56 Hz, 1 H) 7.21 - 7.24 (m, 1 H) 7.53 (dd, *J*=8.07, 1.06 Hz, 1 H) 7.84 (d, *J*=8.38 Hz, 2 H) 7.99 (d, *J*=8.38 Hz, 2 H) |
| 82 | 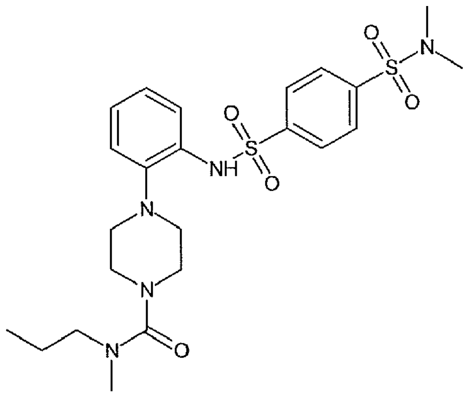 | $^1$H NMR ($CDCl_3$) δ ppm 0.90 (t, *J*=7.38 Hz, 3 H) 1.59 (dq, *J*=14.76, 7.38 Hz, 2 H) 2.55 (br s, 4 H) 2.72 (s, 6 H) 2.85 (s, 3 H) 3.17 (br t, *J*=7.38 Hz, 2 H) 3.29 (br s, 4 H) 7.04 - 7.19 (m, 3 H) 7.58 (br d, *J*=8.00 Hz, 1 H) 7.83 (br d, *J*=8.25 Hz, 2 H) 7.98 (br d, *J*=8.38 Hz, 2 H) 8.08 (NH) |
| 83 | 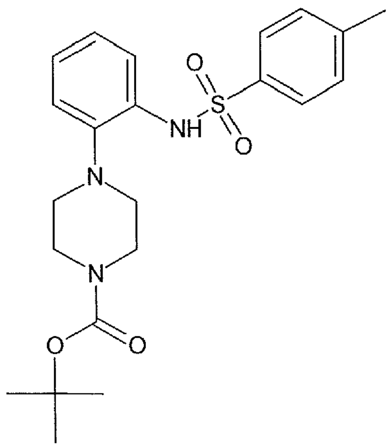 | $^1$H NMR ($CDCl_3$) δ ppm 1.48 (s, 9 H) 2.35 (s, 3 H) 2.38 - 2.51 (m, 4 H) 3.46 (br s, 4 H) 7.02 - 7.06 (m, 2 H) 7.09 - 7.16 (m, 1 H) 7.21 (d, *J*=8.13 Hz, 2 H) 7.59 - 7.63 (m, 1 H) 7.67 (d, *J*=8.13 Hz, 2 H) 7.89 (N H) |

(continued)

| Example | Structure | NMR |
|---|---|---|
| 84 | 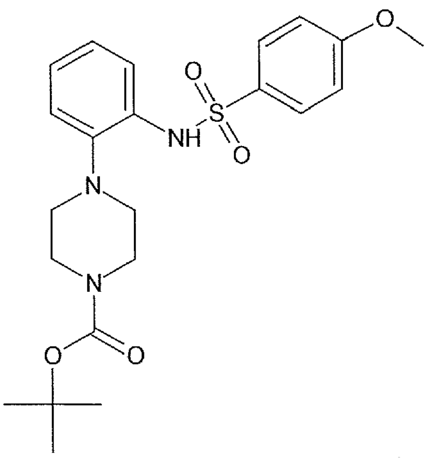 | $^1$H NMR ($CDCl_3$) δ ppm 1.48 (s, 9 H) 2.45 (br s, 4 H) 3.47 (br s, 4 H) 3.80 (s, 3 H) 6.84 - 6.90 (m, 2 H) 7.00 - 7.07 (m, 2 H) 7.10 - 7.16 (m, 1 H) 7.59 - 7.63 (m, 1 H) 7.69 - 7.75 (m, 2 H) 7.88 (NH) |
| 85 | 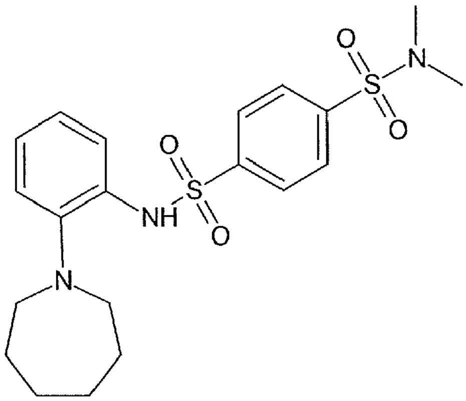 | $^1$H NMR ($CDCl_3$) δ ppm 1.62 - 1.76 (br s, 8 H) 2.65 - 2.73 (br s, 10 H) 6.99 - 7.13 (m, 3 H) 7.59 (d, *J*=7.75 Hz, 1 H) 7.81 (d, *J*=8.38 Hz, 2 H) 7.97 (d, *J*=8.38 Hz, 2 H) 8.31 (NH) |
| 86 | 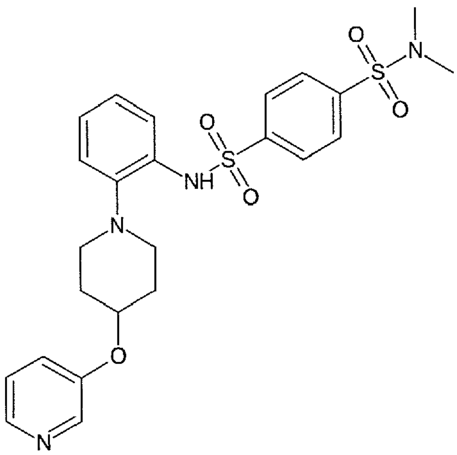 | $^1$H NMR ($CDCl_3$) δ ppm 1.86 (m, 2 H) 1.98 (m, m2 H) 2.43 (m, 2 H) 2.62 (s, 6 H) 2.70 (m, 2 H) 4.42 (br s, 1 H) 6.98 - 7.12 (m, 3 H) 7.16 (m, 2 H) 7.52 (br d, *J*=7.88 Hz, 1 H) 7.75 (br d, *J*=8.25 Hz, 2 H) 7.91 (br d, *J*=8.25 Hz, 2 H) 8.04 (NH) 8.17 (m, 1 H) 8.27 (m, 1 H) |

(continued)

| Example | Structure | NMR |
|---|---|---|
| 87 | 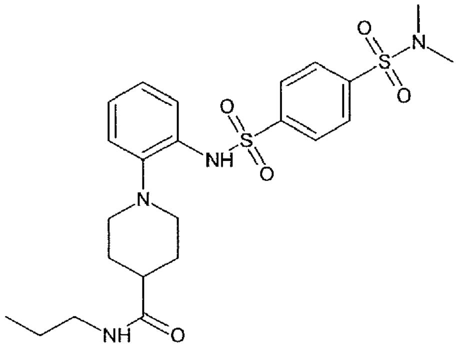 | $^1$H NMR ($CD_3OD$) δ ppm 0.95 (t, *J*=7.44 Hz, 3 H) 1.55 (sxt, *J*=7.25 Hz, 2 H) 1.67 - 1.78 (m, 2 H) 1.84 (qd, *J*=12.15, 3.81 Hz, 2 H) 2.27 (tt, *J*=11.57, 3.94 Hz, 1 H) 2.39 - 2.47 (m, 2 H) 2.49 - 2.61 (m, 2 H) 2.68 (s, 6 H) 3.17 (t, *J*=7.07 Hz, 2 H) 7.08 - 7.21 (m, 3 H) 7.58 (dd, *J*=7.57, 1.56 Hz, 1 H) 7.90 (d, *J*=8.50 Hz, 2 H) 8.03 (d, *J*=8.50 Hz, 2 H) |
| 88 | 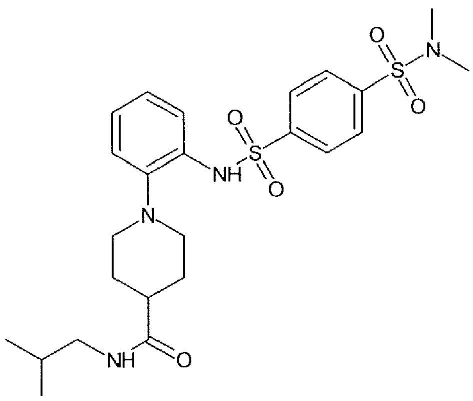 | $^1$H NMR ($CD_3OD$) δ ppm 0.92 (d, *J*=6.63 Hz, 6 H) 1.66 - 1.76 (m, 2 H) 1.77 - 1.90 (br s, 3 H) 2.22 - 2.34 (m, 1 H) 2.42 (br d, *J*=11.38 Hz, 2 H) 2.50 - 2.60 (m, 2 H) 2.66 (s, 6 H) 3.02 (br d, *J*=6.88 Hz, 2 H) 7.06 - 7.19 (m, 3 H) 7.52 - 7.60 (m, 1 H) 7.88 (br d, *J*=8.25 Hz, 2 H) 8.01 (br d, *J*=8.38 Hz, 2 H) |
| 89 | 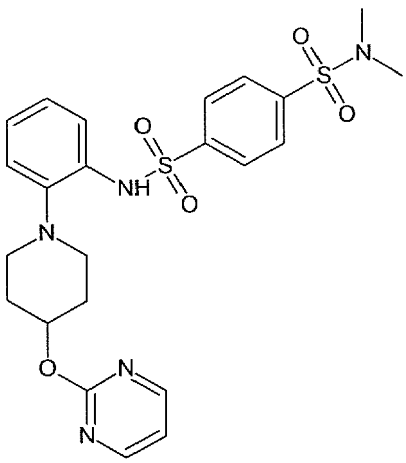 | $^1$H NMR ($CDCl_3$) δ ppm 1.97 (br s, 2 H) 2.07 (br d, *J*=3.50 Hz, 2 H) 2.50 (br s, 2 H) 2.67 (s, 6 H) 2.76 (br s, 2 H) 5.18 (br s, 1 H) 6.94 (t, *J*=4.75 Hz, 1 H) 7.03 - 7.18 (br s, 3 H) 7.56 - 7.64 (m, 1 H) 7.80 (d, *J*=8.25 Hz, 2 H) 7.97 (d, *J*=8.50 Hz, 2 H) 8.14 (NH) 8.48 - 8.54 (m, 2 H) |
| 90 | 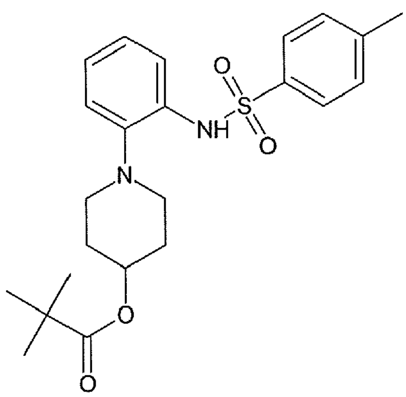 | $^1$H NMR ($CDCl_3$) δ ppm 1.48 (s, 9 H) 1.71 - 1.82 (m, 2 H) 1.91 (br dd, *J*=13.26, 3.25 Hz, 2 H) 2.28 (tt, *J*=10.90, 4.11 Hz, 1 H) 2.35 (s, 3 H) 2.44 - 2.56 (m, 4 H) 6.97 - 7.07 (m, 2 H) 7.07 - 7.13 (m, 1 H) 7.20 (d, *J*=8.25 Hz, 2 H) 7.61 (dd, *J*=8.13, 1.25 Hz, 1 H) 7.69 (d, *J*=8.25 Hz, 2 H) 7.94 (NH) |

(continued)

| Example | Structure | NMR |
|---|---|---|
| 91 | 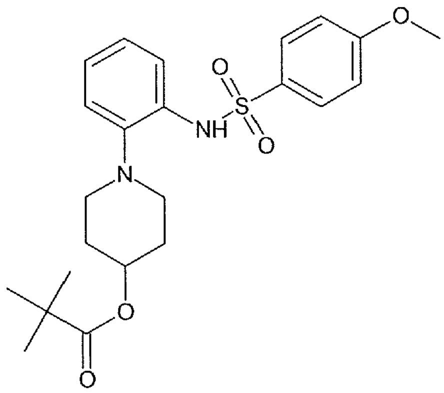 | $^1$H NMR ($CDCl_3$) $\delta$ ppm 1.48 (s, 9 H) 1.71 - 1.82 (m, 2 H) 1.92 (br dd, $J$=13.26, 3.13 Hz, 2 H) 2.28 (tt, $J$=10.87, 4.08 Hz, 1 H) 2.44 - 2.58 (m, 4 H) 3.80 (s, 3 H) 6.85 - 6.90 (m, 2 H) 6.97 - 7.07 (m, 2 H) 7.07 7.13 (m, 1 H) 7.60 (dd, $J$=8.00, 1.00 Hz, 1 H) 7.74 (d, $J$=8.88 Hz, 2 H) 7.92 (NH) |

**Pharmacology**

**CELLULAR TRPML1 ASSAY**

**Buffers and reagents**

- PBS (D-PBS without calcium and magnesium; EuroClone)
- Trypsin (Trypsin 0.05%, EDTA 0.02% in PBS; EuroClone)
- DMSO (Sigma)
- Ca2+ free Tyrode's buffer: in-house solution (130 mM NaCl, 5 mM KCl, 1 mM MgCl2, 5 mM NaHCO3, 20 mM HEPES in water at pH 7.4; sterile filtered).
- Opti-MEM (Gibco)
- Agonist: ML-SA1 (Sigma); stock: 60 mM in DMSO, stored at -20°C
- Blocker: ML-SI3 (in house synthesis); stock: 20 mM in DMSO, stored at -20°C

**Cell line**

The final clone for the TRPML1 assay is HEK T-REx/GCaMP6f/TRPML1.

GCaMP6fis a genetically encoded calcium indicator that is stably expressed in this cell line and used as a fluorescent read-out.

**Assay protocol**

Experiments are performed in 384 MTP format. Cells are seeded at 15000 cells/well either in 25 $\mu$l/well of growth medium or in 20 $\mu$l/well of Optimem + 0.5% FBS without selection antibiotics. Twenty-four hours later, cells are assayed for the response to various compounds using the $Ca^{2+}$ sensitive GCaMP6f protein stably expressed in the cells as readout.

The experiment is performed in a 384-well format according to the following procedures for either $Ca^{2+}$ free or Optimem conditions:

- $Ca^{2+}$ free condition (in absence of extracellular $Ca^{2+}$):

- 24h after seeding, pre-incubate the cells at room temperature for about 10'.
- Then remove the culture medium and replace it with 20 μL of $Ca^{2+}$ free Tyrode's buffer.
- Start the experiment at the $FLIPR^{TETRA}$ by injecting 10 μL/w of 3x concentrated test compounds and controls in $Ca^{2+}$ free Tyrode's buffer. Monitor the kinetic response over a period of 300 seconds.
- Final DMSO concentration: 0.5%

• Optimem condition (in presence of extracellular $Ca^{2+}$):

- 24h after seeding, pre-incubate the cells at room temperature for about 10'.
- Start the experiment at the $FLIPR^{TETRA}$ by injecting 10 μL/w of 3x concentrated test compounds and controls in $Ca^{2+}$ free Tyrode's buffer. Monitor the kinetic response over a period of 300 seconds.
- Final DMSO concentration: 0.5%

Data from $FLIPR^{TETRA}$ measurements are analyzed with the Genedata Screener© software.

An example of plate layout is reported in Figure 1.

**Data analysis**

| | |
|---|---|
| **Compound % Activity** | • Calculate the Kinetic Response Value (KRV) as: $MAX_{CA}$/Baseline = [max(sec5...290)/Mean (sec 1...sec2)] • Normalize the KRV to the median (<>) of Neutral and Stimulator control wells: $Activity[\%]_Ago = 100^{*} \left( \frac{x - < NeutralControls >}{< StimulatorControls > - < NeutralControls >} \right)$ |

**Table 5: Activity of TRPML1 agonists**

| Example | $EC_{50}$(μM) |
|---|---|
| 1 | 12.7 |
| 2 | 24.9 |
| 3 | 30.4 |
| 4 | 8.4 |
| 5 | >50 |
| 6 | 1.5 |
| 7 | 9.9 |
| 8 | 11.7 |
| 9 | 7.6 |
| 10 | >50 |
| 11 | 33.5 |
| 12 | 2.9 |
| 13 | 2.0 |
| 14 | >50 |
| 15 | >50 |

(continued)

| Example | $EC_{50}$(μM) |
|---|---|
| 16 | 14.6 |
| 17 | 18.2 |
| 18 | >50 |
| 19 | >50 |
| 20 | >50 |
| 21 | 37.5 |
| 22 | >50 |
| 23 | 13.2 |
| 24 | 2.5 |
| 25 | 1.7 |
| 26 | 3.9 |
| 27 | 11.8 |
| 28 | >50 |
| 29 | >50 |
| 30 | 15.2 |
| 31 | >50 |
| 32 | 20.5 |
| 33 | 16.6 |
| 34 | >50 |
| 35 | 8.1 |
| 36 | 7.3 |
| 37 | 6.6 |
| 38 | >50 |
| 39 | 8.1 |
| 40 | >50 |
| 41 | 47.4 |
| 42 | >50 |
| 43 | >50 |
| 44 | >50 |
| 45 | >50 |
| 47 | >50 |
| 48 | 6.1 |
| 49 | 22.8 |
| 50 | >50 |
| 51 | >50 |
| 52 | 7.2 |
| 53 | 3.6 |
| 54 | 18.6 |

(continued)

| Example | $EC_{50}$(μM) |
|---|---|
| 55 | 6.1 |
| 56 | 5.6 |
| 57 | 3.2 |
| 58 | >50 |
| 59 | 22.3 |
| 61 | 39.5 |
| 62 | >50 |
| 63 | 1.7 |
| 64 | >50 |
| 65 | >50 |
| 66 | >50 |
| 67 | >50 |
| 68 | >50 |
| 69 | 15.4 |
| 70 | 3.2 |
| 71 | 17.3 |
| 72 | >50 |
| 73 | 5.9 |
| 74 | 2.7 |
| 75 | 1.0 |
| 76 | 0.77 |
| 77 | 0.98 |
| 78 | 7.2 |
| 79 | 8.1 |
| 80 | 18.7 |
| 81 | 8.7 |
| 82 | 6.7 |
| 83 | 2.3 |
| 84 | 4.0 |
| 85 | 15.5 |
| 86 | 5.9 |
| 87 | 20.0 |
| 88 | 8.4 |
| 89 | 2.8 |
| 90 | 2.1 |
| 91 | 3.6 |

**TRPML1 - AUTOMATED PATCH-CLAMP**

**Cell culture conditions**

HEK T-REx/GcaMP6f/TRPML1 cells were cultured in DMEM High Glucose (Lonza BioWhittaker cat. BE12-604F/U1; 500 mL) supplemented with Fetal Bovine Serum TET-FREE (Euroclone cat. EC S0182L; 50 mL), Penicillin-Streptomycin (BioWhittaker, cat. DE17-602E; 5 mL of 100x Solution), G418 (Sigma, cat. G8168; 0.25 mg/ml) and Zeocyn (InvivoGen, cat. ant-zn-1; 50 μg/ml)

**Experimental protocol**

HEK T-REx/GcaMP6f/TRPML1 cells were seeded 48 or 72 hours before experiment, at a concentration of $8*10^6/4*10^6$ cells onto a T225 flask. Just before the experiments cells were washed twice with D-PBS w/o $Ca^{2+}/Mg^{2+}$ (Euroclone cat. ECB4004L) and detached from the flask with trypsin-EDTA (Sigma cat. T4174; diluted 1/10). Cells were then re-suspended in the suspension solution: 25 mL EX-CELL ACF CHO medium (Sigma cat. C5467); 0.625 mL HEPES (Sigma cat. H0887); 0.25 mL of 100x Penicilin/Streptomycin (Euroclone cat. ECB3001D), 0.1 mL of Soybean Trypsin Inhibitor 10 mg/mL (Sigma cat. T6522) and placed on the QPatch 16X.

**Ligands and buffer**

Compounds:

2 (two) agonists solubilized 100% DMSO (20 mM) were tested in three concentrations (0.5, 5, 50 μM). Dilution from stock were prepared just before the experiments in the extracellular solution (0.25% final DMSO concentration). DMSO solution was obtained from AppliChem (cat. A3672)

Patch clamp solutions:

- Intracellular solution (mM): 120 CsMeSO3, 4 NaCl, 2 MgCl2, 10 EGTA, 20 HEPES, 2 Mg-ATP (pH 7.2 with CsOH).
- Extracellular solution (mM): 140 NaGluconate, 5 KCl, 1 MgCl2, 2 CaC12, 10 Glucose, 10 MES (pH 4.6 with HCl).

Patch clamp analysis - QPatch 16X

For the voltage clamp experiments on TRPML1, data were sampled at 5 KHz. After establishment of the seal and the passage in the whole cell configuration, the cells were held at -20 mV and the TRPML1 current was evoked using the voltage protocol illustrated in **Figure 2,** applied every 5 sec. (50 ms at -20 mV; 20 ms at -120 mV, 1000 ms ramp from -120 to +20 mV; 20 ms at +40 mV, and back to -20 mV); in the absence (vehicle period, i.e. 0.25% DMSO) and in the presence of increasing concentrations (3) of the compound under investigation.

**Data analysis**

Agonists: For data collection, the Sophion proprietary software was used and the analysis was performed off-line using Excel and GraphPad Prism (V 8.00). Results are expressed as TRPML1 current fold increase evoked by the compound under investigation (*i.e.* current in the presence of the compound / current in vehicle).

**Table 6.** QPatch activity of TRPML1 agonists

| Example | **Qpatch Assay TRPML1 agonism Current fold increase Mean ± SEM (n)** |
|---|---|
| 1 | 30 μM: 14.5 ± 6.0 (4) |
| 6 | 3.0 μM: 32.7 ± 8.9 (3) |
| 39 | 30 μM: 82.0 ± 63.9 (3) |
| 52 | 3.0 μM: 10.4 ± 3.8 (3) |
| 53 | 3.0 μM: 88.3 ± 59.9 (3) |

(continued)

| Example | **Qpatch Assay TRPML1 agonism Current fold increase Mean ± SEM (n)** |
|---|---|
| 69 | 3.0 μM: 43.9 ± 27.1 (3) |
| 70 | 3.0 μM: 22.5 ± 10.6 (3) |
| 73 | 30 μM: 11.3 (1) |
| 76 | 30 μM: 244 ± 27.6 (4) |

**TRPML1 - MANUAL PATCH CLAMP**

Endolysosomal electrophysiology was performed in isolated enlarged endolysosomes using a modified patch-clamp method (Dong et al., 2010). HEK T-REx/GcaMP6f/TRPML1 cells were treated with 0.8 mM PIKfive inhibitor overnight. Whole-endolysosome recordings were performed on manually isolated enlarged endolysosomes (Dong et al., 2010). In brief, a patch pipette was pressed against a cell and quickly pulled away to slice the cell membrane. Enlarged endolysosomes were released into a dish and a gigaseal between the patch pipette and the enlarged endolysosome was obtained. Negative pressure or voltage steps of several hundred millivolts with millisecond duration were then applied to break into the vacuolar membrane.

Bath (internal/cytoplasmic) solution contained 140 mM K-gluconate, 4 mM NaCl, 1 mM EGTA, 2 mM $MgCl_2$, 0.39 mM $CaCl_2$, 20 mM HEPES (pH was adjusted with KOH to 7.2). The pipette (luminal) solution was standard extracellular solution (modified Tyrode's: 145 mM NaCl, 5 mM KCl, 2 mM $CaCl_2$, 1 mM $MgCl_2$, 10 mM HEPES, 10 mM MES, 10 mM glucose; the pH was adjusted with NaOH to pH 4.6).

Agonists solubilized 100% DMSO (20 mM) were tested in single concentration (2 μM). Dilution from stock were prepared just before the experiments in the extracellular solution (0.1% final DMSO concentration). DMSO solution was obtained from AppliChem (cat. A3672).

All bath solutions were applied via a perfusion system that allowed us to achieve complete solution exchange within a few seconds.

Data were collected using an HEKA EPC 10 patch-clamp amplifier, and PatchMaster v2x73.5 software (HEKA Elektronik). Whole-endolysosome currents were digitized at 20 kHz. All experiments were conducted at room temperature, and all recordings were analyzed with pClamp 10.7 (Molecular Devices) and GraphPad Prism (V8.00).

TRPML1 current was evoked using the voltage protocol illustrated in **Figure 3**, applied every 5 sec. (10 ms at -80 mV; 500 ms ramp from -120 to +40 mV; 10 ms at -80 mV); in the absence (vehicle period, i.e. 0.1% DMSO) and in the presence of the compound (agonist) under investigation.

**Table 7.** Secondary Assays Agonism - Manual Patch

| **Example** | **Concentration (μM)** | **Lysosomal Patch-Clamp Current fold increase Mean ± SEM (n)** |
|---|---|---|
| 6 | 2.0 | 6.2 ± 1.6 (3) |
| 57 | 2.0 | 27.5 ± 10.8 (2) |

**AUTOPHAGY ASSAY**

**Buffers and reagents**

- Tyrode's buffer with 2 mM Ca2+ in-house solution (130 mM NaCl, 5 mM KCl, 2 mM CaCl2, 1 mM MgCl2, 5 mM NaHCO3, 20 mM HEPES in water at pH 7.4; sterile filtered)
- DMSO (Sigma)
- Bis-benzimide H 33342 trihydrochloride (Hoechst 33342; Sigma-Aldrich)
- Reference molecule (agonist): Torin-1, (#4247, Tocris) stock 1.2 mM in DMSO, stored at -20°C

**Reporter of Autophagy and Cell line**

Reporter Rosella: a fluorescent-based chimera constitutes by two fluorescent proteins, Green and Red variants, acting as a bimodal indicator of pH. The green fluorescent variant of the constructs is pH sensitive (pKa= 6.9) while the

red variant is not pH sensitive and it is used as reference. The reporter is fused to sequence for autophagosome localization (LC3 tag).

The reporter Rosella have been stably expressed in HeK293 cell line and used as read-out.

**Assay protocol**

Experiments have been performed in 384 MTP poly-lysine coated well format. Cells have been seeded in 384-w at a density of 6000 cells/well in 25 μl/well complete growth medium without antibiotics. Twenty-four hours later, cells have been treated with compounds and incubated for further 18 hours. Then the cells have been imaged and assayed for the response to various compounds using the reporter for autophagy expressed in the cells as readout.

The experiments were performed in a 384-well format according to the following procedure:

- 24h after seeding, cells were incubated with compounds at the desired concentration, and with the reference molecule (agonist) Torin-1 at a top concentration of 1 μM (max signal), for 18 hours at 37°C and 5% CO2. The final percentage of DMSO was 0.3% in all the conditions.
- Then the culture medium was carefully removed to avoid cells detachment and to discard red phenol into the medium and that can interfere with the measurements.
- Staining of the nuclei was obtained by incubating the cells with 8 μM/well of Hoechst 3342 in standard Tyrode's buffer for 20 min at RT
- Then the cells were carefully washed two times with standard Tyrode's buffer
- Finally, the samples were acquired by recording three fluorescence emission channels (green, red and blue) at 20X magnification and with at least 3-4 fields of view per well in an Operetta CLS microscope (PerkinElmer).
- Image analysis was performed by using Harmony software (PerkinElmer). The image analysis involved the following steps: flat-field illumination correction, nuclei segmentation, cell segmentations and identification of vesicles/granules within the cytosol compartments and representing the autophagy vesicles. Measurements of signal intensity ratio (Green to Red) and number of autophagy vesicles per cells were used to obtain information on the effects of compounds on the autophagy flux.
- Data from image analysis measurements were finally loaded and analyzed for normalization and fitting procedures in Genedata Screener© software.

**Table 8:** Autophagy assay results for TRPML1 agonists

| Example | Secondary Assays Autophagy qAC50 - (μM) |
|---|---|
| 6 | 8.0 |
| 57 | 0.51 |

**Claims**

**1.** A compound of formula (I)

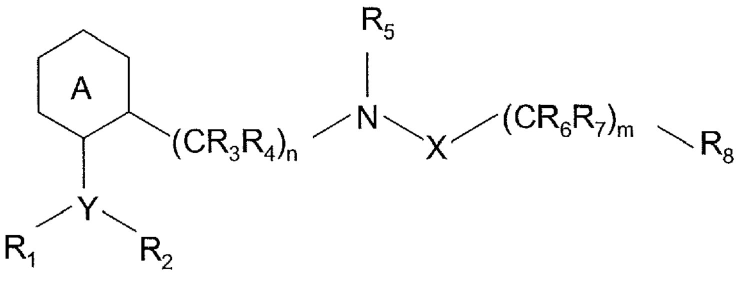

(I)

or a stereoisomer thereof, or a salt of any of the foregoing, wherein:

A is a six membered aliphatic, aromatic or heteroaromatic ring, optionally substituted by one or more substituents selected from the group consisting of $C_1$-$C_4$ alkyl, $C_1$-$C_4$ alkyloxy, $C_3$-$C_6$ cycloalkyl, halogen, and cyano.

Y is chosen from CH, or N;

each of $R_1$ and $R_2$ independently is a $C_1$-$C_4$ alkyl group or $R_1$ and $R_2$ taken together with Y form a four, five, six or seven membered heteroaliphatic ring, containing at least one nitrogen atom, optionally fused with a six membered aromatic ring, optionally substituted by one or more substituents selected from the group consisting of:

- halogen;
- $C_1$-$C_4$ alkyl, $C_1$-$C_4$ alkyloxy, $C_1$-$C_4$ alkylcarbonyl, $C_1$-$C_4$ alkoxycarbonyl, wherein the $C_1$-$C_4$ alkyl chain is optionally substituted by one or more halogen and $C_1$-$C_4$-alkyloxy;
- phenyl, benzyl, benzoyl, phenoxy, benzyloxy, pyrazin-2-yl-oxy, pyridin-2-yl-oxy, pyridin-3-yl-oxy, pyrimidin-2-yl-oxy thiophene-2-carbonyl, 1,3 thiazole-5-carbonyl, thiophene-2-yl-acetyl, wherein the aromatic ring is optionally substituted by one or more halogen, $C_1$-$C_4$ alkyl, $C_1$-$C_4$-alkyloxy;
- a $C_1$-$C_2$ alkylene bridge; and
- $R_{11}R_{12}N$-CO- wherein each of $R_{11}$ and $R_{12}$ is $C_1$-$C_4$ alkyl;

X is -CO- or -$SO_2$-;

each of $R_3$, $R_4$, $R_5$, $R_6$ and $R_7$ independently is hydrogen or $C_1$-$C_4$ alkyl;

each of m and n independently is 0 or 1;

$R_8$ is a five or six membered monocyclic or a nine or ten membered bicyclic aromatic or heteroaromatic compound, optionally substituted by one or more substituents selected from the group consisting of halogen, pentafluorosulfanyl ($SF_5$), trifluoromethylthio ($SCF_3$), $C_1$-$C_4$ alkyl or $C_1$-$C_6$ cycloalkyl, and 1,3-dioxolan-2-yl, optionally substituted by a cyano group, -$NO_2$, $C_1$-$C_4$ alkyloxy, $C_1$-$C_4$ alkylthio, -$SO_2NR_9R_{10}$ or -$NR_9R_{10}$, wherein each of $R_9$ and $R_{10}$ independently is hydrogen or $C_1$-$C_4$ alkyl or $R_9$ and $R_{10}$ are taken together to the nitrogen atom to which they are bound to form a five or six membered heteroaliphatic ring;

provided that when $R_1$ and $R_2$ taken together with Y form a piperazine substituted by phenyl or benzyl, then $R_8$ is not phenyl.

**2.** A compound of formula (I) according to claim 1 or a stereoisomer thereof, or a salt of any of the foregoing, wherein A is cyclohexyl or phenyl, provided that when $R_1$ and $R_2$ taken together with Y form a piperazine substituted by phenyl or benzyl, then $R_8$ is not phenyl.

**3.** A compound of formula (I) according to claim 1 or 2 or a stereoisomer thereof, or a salt of any of the foregoing, wherein Y is N and $R_1$ and $R_2$ are methyl.

**4.** A compound of formula (I) according to claim 1 or 2, or a stereoisomer thereof, or a salt of any of the foregoing, wherein $R_1$ and $R_2$ taken together with Y form a piperidine, piperazine, pyrrolidine, azetidine, azepane, morpholine, 1, 2, 3, 4-tetrahydroisoquinoline or 2,3- dihydroindole, wherein each of said moieties is optionally substituted by: halogen, preferably fluorine or chlorine; $C_1$-$C_4$ alkyl, preferably methyl, ethyl, n-propyl or tert-butyl; $C_1$-$C_4$ alkyloxy, preferably methoxy; $C_1$-$C_4$ alkylcarbonyl (wherein the $C_1$-$C_4$ alkyl group is optionally substituted by one or more halogen, preferably fluorine or chlorine, or $C_1$-$C_4$-alkyloxy, preferably methoxy), preferably methylcarbonyl, methoxyethylcarbonyl; $C_1$-$C_4$ alkoxycarbonyl (wherein the $C_1$-$C_4$ alkyl group is optionally substituted by one or more halogen, preferably fluorine or chlorine or $C_1$-$C_4$-alkyloxy, preferably methoxy), preferably methoxycarbonyl, ethoxycarbonyl, tert-butoxycarbonyl, 2,2,2-trifluoroethoxycarbonyl; $C_1$-$C_4$ alkylaminocarbonyl, preferably propylaminocarbonyl or isobutylaminocarbonyl; $C_1$-$C_4$ alkylcarbonyl, preferably isobutylcarbonyl; a compound containing an aromatic ring selected from phenyl, benzyl, phenoxy, benzyloxy, pyrazin-2-yl-oxy and pyridin-2-yl-oxy (wherein the aromatic ring is optionally substituted by one or more halogen, preferably fluorine or chlorine, or $C_1$-$C_4$-alkyloxy); or a $C_1$-$C_2$ alkylene bridge, preferably a methylene bridge, provided that when $R_1$ and $R_2$ taken together with Y form a piperazine substituted by phenyl or benzyl, then $R_8$ is not phenyl.

**5.** A compound of formula (I) according to any one of the preceding claims or a stereoisomer thereof, or a salt of any of the foregoing, wherein $R_3$, $R_4$, $R_5$, $R_6$ and $R_7$ are hydrogen or methyl, provided that when $R_1$ and $R_2$ taken together with Y form a piperazine substituted by phenyl or benzyl, then $R_8$ is not phenyl.

**6.** A compound of formula (I) according to claim 5 or a stereoisomer thereof, or a salt of any of the foregoing, wherein $R_3$, $R_4$, $R_5$, $R_6$ and $R_7$ are hydrogen, provided that when $R_1$ and $R_2$ taken together form a piperazine substituted by phenyl or benzyl, then $R_8$ is not phenyl.

**7.** A compound of formula (I) according to any one of the preceding claims or a stereoisomer thereof, or a salt of any of the foregoing, wherein m and n are both 0, provided that when $R_1$ and $R_2$ taken together form a piperazine

substituted by phenyl or benzyl, then Rs is not phenyl.

**8.** A compound of formula (I) according to any one of the preceding claims or a stereoisomer thereof, or a salt of any of the foregoing, wherein one of m and n is 0 and the other is 1, provided that when $R_1$ and $R_2$ taken together form a piperazine substituted by phenyl or one of m and n is 0 and the other is 1.

**9.** A compound of formula (I) according to any one of the preceding claims or a stereoisomer thereof, or a salt of any of the foregoing, wherein $R_8$ is selected from the group consisting of phenyl, thiophene, benzothiophene, optionally substituted by one or more substituents selected from the group consisting of halogen, preferably fluorine or chlorine or bromo, or pentafluorosulfanyl ($SF_5$), or trifluoromethylthio ($SCF_3$); $C_1$-$C_4$ alkyl optionally substituted by a cyano group, preferably methyl, ethyl, n-propyl, tert-butyl or cyanomethyl; -$NO_2$; $C_1$-$C_4$ alkoxy, preferably methoxy, ethoxy; $C_1$-$C_4$ alkylthio, preferably methylthio, ethylthio; -$SO_2NR_9R_{10}$ or -$NR_9R_{10}$, wherein each of $R_9$ and $R_{10}$ independently is hydrogen or $C_1$-$C_4$ alkyl, preferably methyl, ethyl, most preferably both $R_9$ and $R_{10}$ are methyl, or $R_9$ and $R_{10}$ are taken together with the nitrogen atom to which they are bound to form a five or six membered heteroaliphatic ring, optionally containing one or two oxygen atoms, preferably piperidine, pyrrolidine, morpholine, 1,3 dioxolane, provided that when $R_1$ and $R_2$ taken together with Y form a piperazine substituted by phenyl or benzyl, then $R_8$ is not phenyl.

**10.** A compound of formula (I) according to claim 1 or a stereoisomer thereof, or a salt of any of the foregoing, wherein:

A is phenyl or cyclohexyl;
Y is N and $R_1$ and $R_2$ taken together with Y form a cyclic compound selected from piperidine, pyrrolidine, piperazine, azete, azepane, 2-azabicyclo[2.2.1]heptane, 4-phenylpiperidine, tert-butyl piperazine-1-carboxylate, 4-phenoxypiperidine, 4-(benzyloxy)piperidine, 4-methoxypiperidine, morpholine, dimethylamine, pyrrolidine, 4,4-difluoropiperidine, 3-phenylpiperidine, 3-methoxypiperidine, 1-(4-chloro-2-fluorophenyl)piperazine, 3-phenylpyrrolidine, 2,3-dihydro-1H-indole, 1,2,3,4-tetrahydroquinoline, methyl piperazine-1-carboxylate, 2,2,2-trifluoroethyl piperazine-1-carboxylate, ethyl piperazine-1-carboxylate, 1-(piperazin-1-yl)propan-1-one, 3-methoxy-1-(piperazin-1-yl)propan-1-one, 1-benzoylpiperazine, 2-(piperidin-4-yloxy)pyridine, 2-(piperidin-4-yloxy)pyrazine, tert-butyl piperidine-4-carboxylate, tert-butyl piperidine-1-carboxylate, 4-(pyridin-2-yl)piperidine, 4-(pyrazin-2-yl)piperidine, 4-(thiophene-2-carbonyl)piperazine, 4-(4-methyl-1,3-thiazole-5-carbonyl)piperazine, 4-(piperazin-1-yl)-2-(thiophen-2-yl)ethan-1-one, N-methyl-N-propylpiperazine-1-carboxamide, 4-(thiophene-2-carbonyl)piperazine, 4-(4-methyl-1,3-thiazole-5-carbonyl)piperazine, 4-(piperazin-1-yl)-2-(thiophen-2-yl)ethan-1-one, N-methyl-N-propylpiperazine-1-carboxamide, 3-(piperidin-4-yloxy)pyridine, N-propylpiperidine-4-carboxamide, N-(2-methylpropyl)piperidine-4-carboxamide, 2-(piperidin-4-yloxy)pyrimidine, piperidin-4-yl 2,2-dimethylpropanoate;
both m and n are 0 or one of m and n is 0 and the other is 1;
$R_3$, $R_4$, $R_6$ and $R_7$ are hydrogen;
$R_5$ is hydrogen or methyl;
X is -CO- or -$SO_2$-;
$R_8$ is selected from phenyl, thiophene or benzothiophene, optionally substituted by one or more substituents selected from: dimethylsulfamoyl, methyl, ethyl, propyl, tert-butyl, 1,3-dioxolan-2-yl, cyanomethyl, monoalkylamino, dialkylamino, piperidine-1-sulfonyl, pyrrolidine-1-sulfonyl, morpholine-4-sulfonyl, fluoro, chloro, bromo, $SF_5$, $SCF_3$, nitro, and $C_1$-$C_4$ alkoxy.

**11.** A compound of formula (I) according to claim 1 or a stereoisomer thereof, or a salt of any of the foregoing, selected from the group consisting of:

4-methyl-N-[2-(piperidin-1-yl)phenyl]benzene-1-sulfonamide
4-methyl-N-[2-(piperidin-1-yl)phenyl]thiophene-2-sulfonamide
5-tert-butyl-N-[2-(piperidin-1-yl)phenyl]thiophene-2-sulfonamide
N4-(2-{2-azabicyclo[2.2.1]heptan-2-yl}phenyl)-N1,N1-dimethylbenzene-1,4-disulfonamide
N-(2- {2-azabicyclo[2.2.1]heptan-2-yl}phenyl)-4-methylbenzene-1-sulfonamide
N1,N1-dimethyl-N4-[2-(piperidin-1-yl)phenyl]benzene-1,4-disulfonamide
5-ethyl-N-[2-(piperidin-1-yl)phenyl]thiophene-2-sulfonamide
3,5-dimethyl-N-[2-(piperidin-1-yl)phenyl]thiophene-2-sulfonamide
N-[2-(piperidin-1-yl)phenyl]-5-propylthiophene-2-sulfonamide
N-[2-(piperidin-1-yl)phenyl]-1-benzothiophene-2-sulfonamide
3-(1,3-dioxolan-2-yl)-N-[2-(piperidin-1-yl)phenyl]thiophene-2-sulfonamide
N1,N1-dimethyl-N4-[2-(4-phenylpiperidin-1-yl)phenyl]benzene-1,4-disulfonamide tert-butyl 4-{2-[4-(dimethylsulfamoyl)benzenesulfonamido]phenyl}piperazine-1-carboxylate
5-tert-butyl-N-{2-[4-(4-chloro-2-fluorophenyl)piperazin-1-yl]phenyl}thiophene-2-sulfonamide
N-(2- {2-azabicyclo[2.2.1]heptan-2-yl}phenyl)-5-methylthiophene-2-sulfonamide
N-(2-{2-azabicyclo[2.2.1]heptan-2-yl}phenyl)-5-tert-butylthiophene-2-sulfonamide
N-(2-{2-azabicyclo[2.2.1]heptan-2-yl}phenyl)-5-ethylthiophene-2-sulfonamide
N-(2-{2-azabicyclo[2.2.1]heptan-2-yl}phenyl)-4-(dimethylsulfamoyl)benzamide
4-(dimethylsulfamoyl)-N-[2-(piperidin-1-yl)phenyl]benzamide
4-(cyanomethyl)-N-[2-(piperidin-1-yl)phenyl]benzene-1-sulfonamide
4-tert-butyl-N-[2-(piperidin-1-yl)phenyl]benzene-1-sulfonamide
4-(dimethylamino)-N-[2-(pipendin-1-yl)phenyl]benzene-1-sulfonamide
3 -methyl-N-[2-(piperidin-1 -yl)phenyl]benzene-1 -sulfonamide
N1,N1-dimethyl-N4-[2-(4-phenoxypiperidin-1-yl)phenyl]benzene-1,4-disulfonamide
N4-{2-[4-(benzyloxy)piperidin-1-yl]phenyl}-N1,N1-dimethylbenzene-1,4-disulfonamide
N4-[2-(4-methoxypiperidin-1-yl)phenyl]-N1,N1-dimethylbenzene-1,4-disulfonamide
N1,N1-dimethyl-N4-[2-(morpholin-4-yl)phenyl]benzene-1,4-disulfonamide
N4-[2-(dimethylamino)phenyl]-N1,N1-dimethylbenzene-1,4-disulfonamide
N-[2-(piperidin-1-yl)phenyl]-4-(piperidine-1-sulfonyl)benzene-1-sulfonamide
N-[2-(piperidin-1-yl)phenyl]-4-(pyrrolidine-1-sulfonyl)benzene-1-sulfonamide
4-(morpholine-4-sulfonyl)-N-[2-(piperidin-1-yl)phenyl]benzene-1-sulfonamide
N1,N1-dimethyl-N4-[2-(pyrrolidin-1-yl)phenyl]benzene-1,4-disulfonamide
N4-[2-(4,4-difluoropiperidin-1-yl)phenyl]-N1,N1-dimethylbenzene-1,4-disulfonamide
2-[4-(dimethylsulfamoyl)phenyl]-N-[2-(pyrrolidin-1-yl)phenyl]acetamide
N1,N1-dimethyl-N4-[2-(3-phenylpiperidin-1-yl)phenyl]benzene-1,4-disulfonamide
N4-[2-(3-methoxypiperidin-1-yl)phenyl]-N1,N1-dimethylbenzene-1,4-disulfonamide
N1,N1-dimethyl-N4-[2-(3-phenylpyrrolidin-1-yl)phenyl]benzene-1,4-disulfonamide
N-{2-[4-(4-chloro-2-fluorophenyl)piperazin-1-yl]phenyl}-4-(dimethylsulfamoyl)benzamide
N1,N1-dimethyl-N4-{[2-(pyrrolidin-1-yl)phenyl]methyl}benzene-1,4-disulfonamide
N4-[2-(2,3-dihydro-1H-indol-1-yl)phenyl]-N1,N1-dimethylbenzene-1,4-disulfonamide
N1,N1-dimethyl-N4-[2-(1,2,3,4-tetrahydroisoquinolin-2-yl)phenyl]benzene-1,4-disulfonamide
N1,N1-dimethyl-N4-[2-(4-methylpiperazin-1-yl)phenyl]benzene-1,4-disulfonamide
N-(2-{2-azabicyclo[2.2.1]heptan-2-yl}phenyl)-2-[4-(dimethylsulfamoyl)phenyl]acetamide
N4-[2-(4-acetylpiperazin-1-yl)phenyl]-N1,N1-dimethylbenzene-1,4-disulfonamide
2-[4-(dimethylsulfamoyl)phenyl]-N-[2-(piperidin-1-yl)phenyl]acetamide
N-{2-[4-(4-chloro-2-fluorophenyl)piperazin-1-yl]phenyl}-2-[4-(dimethylsulfamoyl)phenyl] acetamide
N-{2-[4-(4-chloro-2-fluorophenyl)piperazin-1-yl]phenyl}-4-(dimethylsulfamoyl)-N-methylbenzamide
N-(2-{2-azabicyclo[2.2.1]heptan-2-yl}phenyl)-4-(dimethylsulfamoyl)-N-methylbenzamide
4-(dimethylsulfamoyl)-N-methyl-N-[2-(piperidin-1-yl)phenyl]benzamide
N-(2-{2-azabicyclo[2.2.1]heptan-2-yl}phenyl)-5-ethyl-N-methylthiophene-2-sulfonamide
methyl 4-{2-[4-(dimethylsulfamoyl)benzenesulfonamido]phenyl}piperazine-1-carboxylate 2,2,2-trifluoroethyl 4-{2-[4-(dimethylsulfamoyl)benzenesulfonamido]phenyl}piperazine-1-carboxylate
N4-{2-[4-(2-methoxyphenyl)piperazin-1-yl]cyclohexyl}-N1,N1-dimethylbenzene-1,4-disulfonamide
N1,N1-dimethyl-N4-[2-(4-phenylpiperidin-1-yl)cyclohexyl]benzene-1,4-disulfonamide
N1,N1-dimethyl-N4-[2-(4-phenylpiperazin-1-yl)cyclohexyl]benzene-1,4-disulfonamide
N1,N1-dimethyl-N4-[2-(4-phenoxypiperidin-1-yl)cyclohexyl]benzene-1,4-disulfonamide
N1,N1-dimethyl-N4-[2-(piperidin-1-yl)cyclohexyl]benzene-1,4-disulfonamide
N4-{2-[4-(4-chloro-2-fluorophenyl)piperazin-1-yl]cyclohexyl}-N1,N1-dimethylbenzene-1,4-disulfonamide
N1,N1-dimethyl-N4-[2-(morpholin-4-yl)cyclohexyl]benzene-1,4-disulfonamide
N1,N1-dimethyl-N4-[2-(4-phenoxypiperidin-1-yl)cyclohexyl]benzene-1,4-disulfonamide
N1,N1-dimethyl-N4-[2-(4-phenoxypiperidin-1-yl)cyclohexyl]benzene-1,4-disulfonamide
N4-[2-(2,3-dihydro-1H-indol-1-yl)cyclohexyl]-N1,N1-dimethylbenzene-1,4-disulfonamide
4-chloro-N-[2-(piperidin-1-yl)phenyl]benzene-1-sulfonamide
4-(dimethylsulfamoyl)-N-[2-(pyrrolidin-1-yl)phenyl]benzamide
4-(dimethylsulfamoyl)-N-methyl-N-[2-(pyrrolidin-1-yl)phenyl]benzamide
4-nitro-N-[2-(piperidin-1-yl)phenyl]benzene-1-sulfonamide
4-methoxy-N-[2-(piperidin-1-yl)phenyl]benzene-1-sulfonamide ethyl 4-{2-[4-(dimethylsulfamoyl)benzenesulfonamido]phenyl}piperazine-1-carboxylate
N1,N1-dimethyl-N4-[2-(4-propanoylpiperazin-1-yl)phenyl]benzene-1,4-disulfonamide
N4-{2-[4-(3-methoxypropanoyl)piperazin-1-yl]phenyl}-N1,N1-dimethylbenzene-1,4-disulfonamide

N4-[2-(4-benzoylpiperazin-1-yl)phenyl]-N1,N1-dimethylbenzene-1,4-disulfonamide
N1,N1-dimethyl-N4-{[2-(piperidin-1-yl)phenyl]methyl}benzene-1,4-disulfonamide
N1,N1-dimethyl-N4-{2-[4-(pyridin-2-yloxy)piperidin-1-yl]phenyl}benzene-1,4-disulfonamide
tert-butyl 1-{2-[4-(dimethylsulfamoyl)benzenesulfonamido]phenyl}piperidine-4-carboxylate
N1,N1-dimethyl-N4-{2-[4-(pyrazin-2-yloxy)piperidin-1-yl]phenyl}benzene-1,4-disulfonamide
tert-butyl 4-{2-[4-(dimethylsulfamoyl)benzenesulfonamido]phenyl}piperidine-1-carboxylate
N1,N1-dimethyl-N4-{2-[4-(thiophene-2-carbonyl)piperazin-1-yl]phenyl}benzene-1,4-disulfonamide
N1,N1-dimethyl-N4-{2-[4-(4-methyl-1,3-thiazole-5-carbonyl)piperazin-1-yl]phenyl}benzene-1,4-disulfonamide
N1,N1-dimethyl-N4-(2-{4-[2-(thiophen-2-yl)acetyl]piperazin-1-yl}phenyl)benzene-1,4-disulfonamide
4-{2-[4-(dimethylsulfamoyl)benzenesulfonamido]phenyl}-N-methyl-N-propylpiperazine-1-carboxamide

**12.** A process for preparing a compounds of formula (I) according to claim 1 or a stereoisomer thereof, or a salt of any of the foregoing comprising the step of reacting a compound of formula (II) or formula (III):

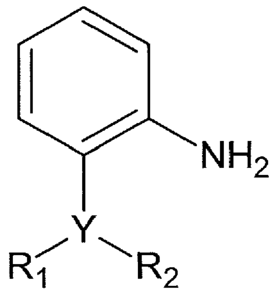

(II)

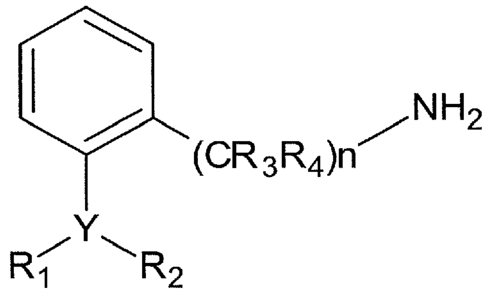

(III)

wherein Y, $R_1$, $R_2$, $R_3$, $R_4$ and n are as defined in claim 1, with a compound of formula (IV) or formula (V)

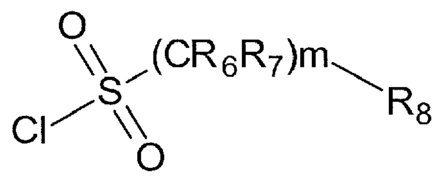

(IV)

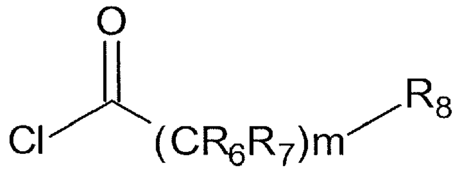

(V)

wherein $R_6$, $R_7$, m and $R_8$ are as defined in claim 1 .

**13.** A process for preparing a compounds of formula (I) according to claim 1 or a stereoisomer thereof, or a salt of any of the foregoing comprising the step of reacting a compound of formula (VI):

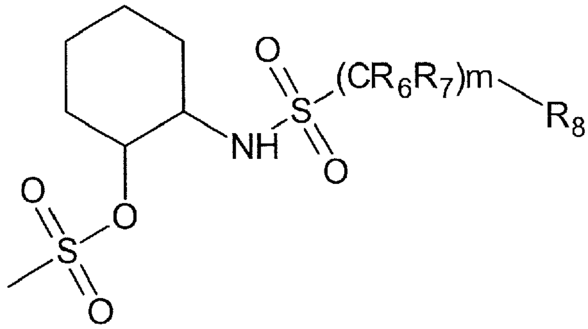

(VI)

wherein $R_6$, $R_7$, m and $R_8$ are as defined above; with a suitably substituted amine.

**14.** A process for preparing a compounds of formula (I) according to claim 1 or a stereoisomer thereof, or a salt of any of the foregoing comprising the step of reacting a compound of formula (VII) or compound of formula (VIII) with $CH_3I$

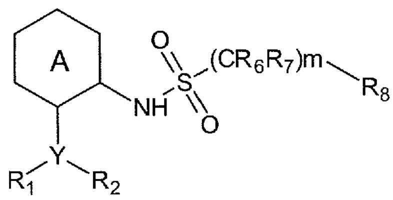

(VII)

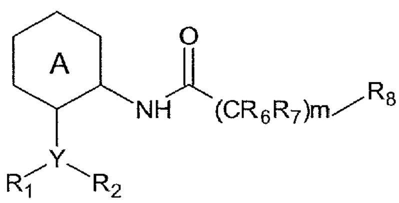

(VIII)

wherein A, Y, $R_1$, $R_2$, $R_6$, $R_7$, m and $R_8$ are as defined in claim 1.

**15.** A process for preparing a_compounds of formula (I) according to claim 1 or a stereoisomer thereof, or a salt of any of the foregoing comprising the step of reacting a compound of formula (IX)

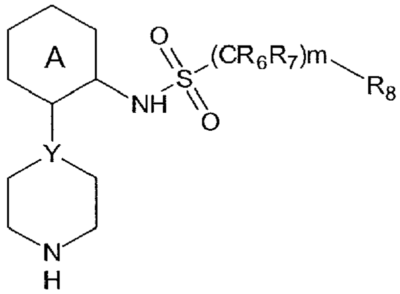

(IX)

wherein A, Y, $R_6$, $R_7$, m and $R_8$ are as defined above, with a compound of formula (X)

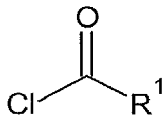

(X)

wherein $R_1$ is as defined above.

**16.** A pharmaceutical composition comprising a compound of formula (I) according to claim 1, or a stereoisomer thereof, or a salt of any of the foregoing and a pharmaceutically acceptable carrier.

**17.** A compound of formula (I) according to claim 1, or a stereoisomer thereof, or a salt of any of the foregoing for use as a medicament.

**18.** A compound of formula (I) according to claim 1, or a stereoisomer thereof, or a salt of any of the foregoing for use in the treatment of a TRPML1- mediated disorder or disease.

**19.** A compound of formula (I) for the use according to claim 18, or a stereoisomer thereof, or a salt of any of the foregoing wherein the TRPML1- mediated disorder or disease is a neurodegenerative disorder or disease.

**20.** A compound of formula (I) for the use according to claim 19, or a stereoisomer thereof, or a salt of any of the foregoing, wherein the neurodegenerative disorder or disease is selected from Alzheimer's disease and other dementia conditions such as Lewy body dementia, fronto-temporal dementia and other tauopathies; amyotrophic lateral sclerosis, multiple sclerosis, Parkinson's disease and other parkinsonian syndromes; HIV-induced neuroinflammation; essential tremors; other spinocerebellar degenerations, neuropathies such as Charcot-Marie-Tooth neuropathy.

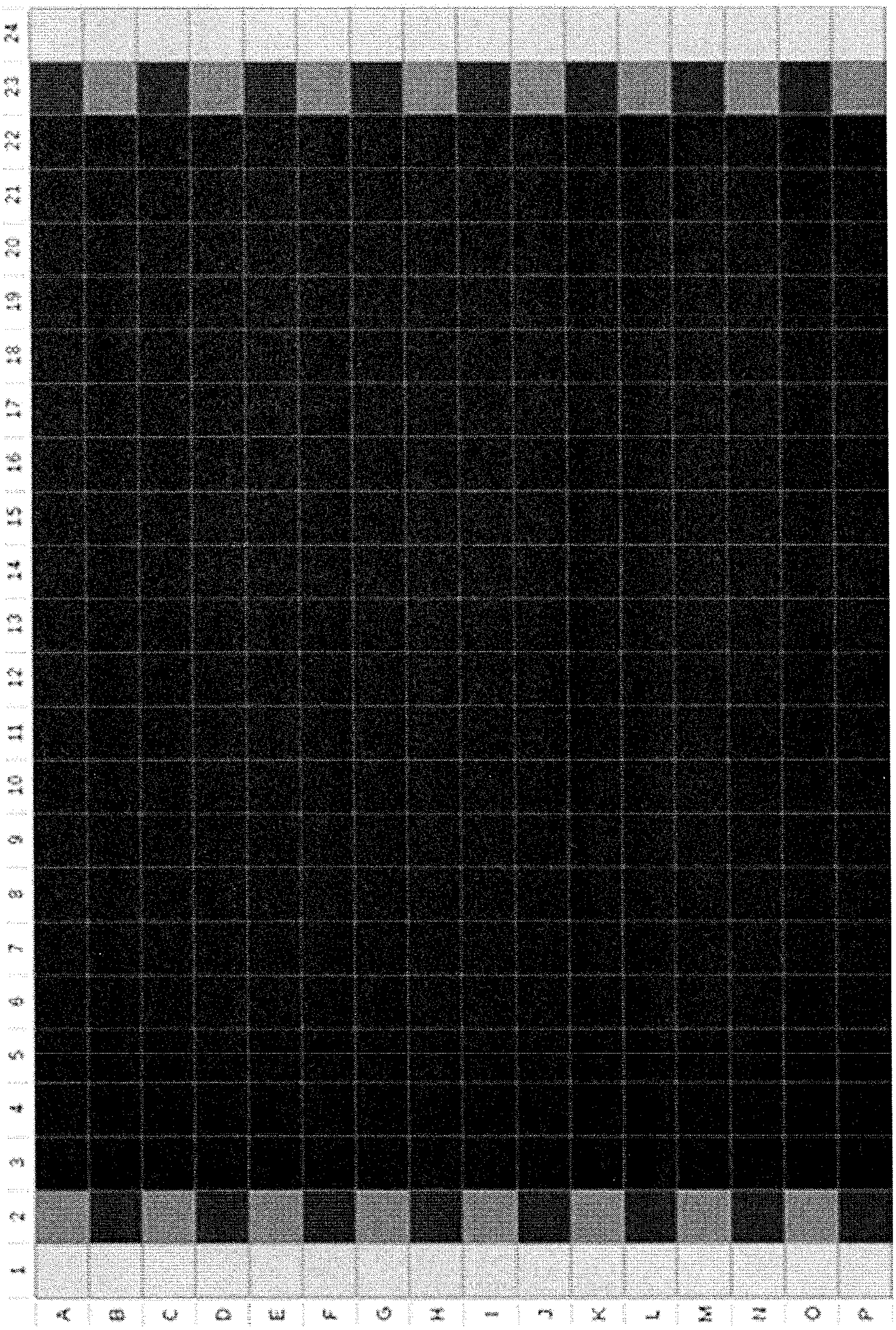
1 2 3 4 5 6 7 8 9 10 11 12 13 14 15 16 17 18 19 20 21 22 23 24
A B C D E F G H I J K L M N O P

Figure 1

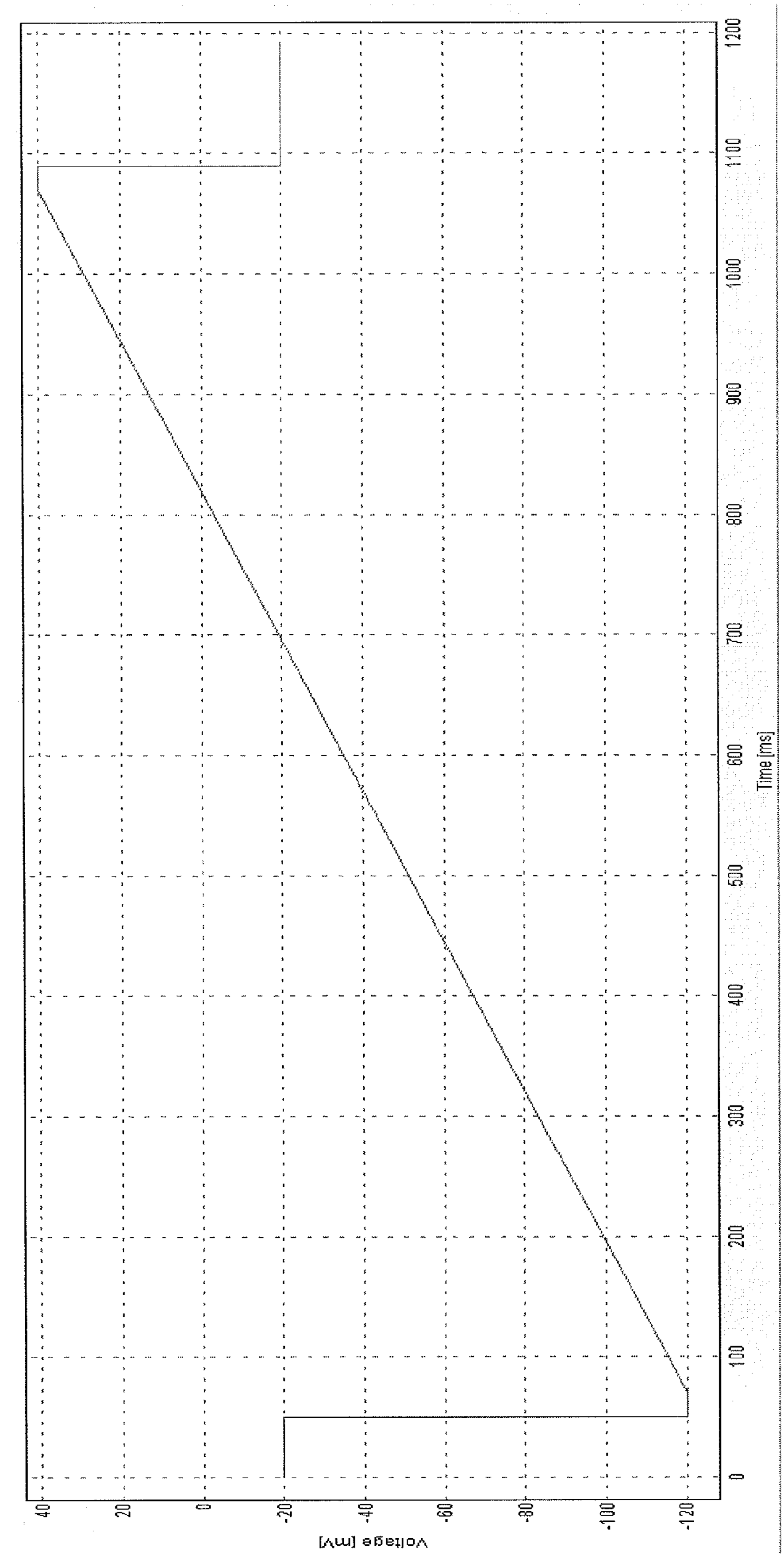
Voltage [mV]
40
20
0
-20
-40
-60
-80
-100
-120
Time [ms]
0
100
200
300
400
500
600
700
800
900
1000
1100
1200

Figure 2

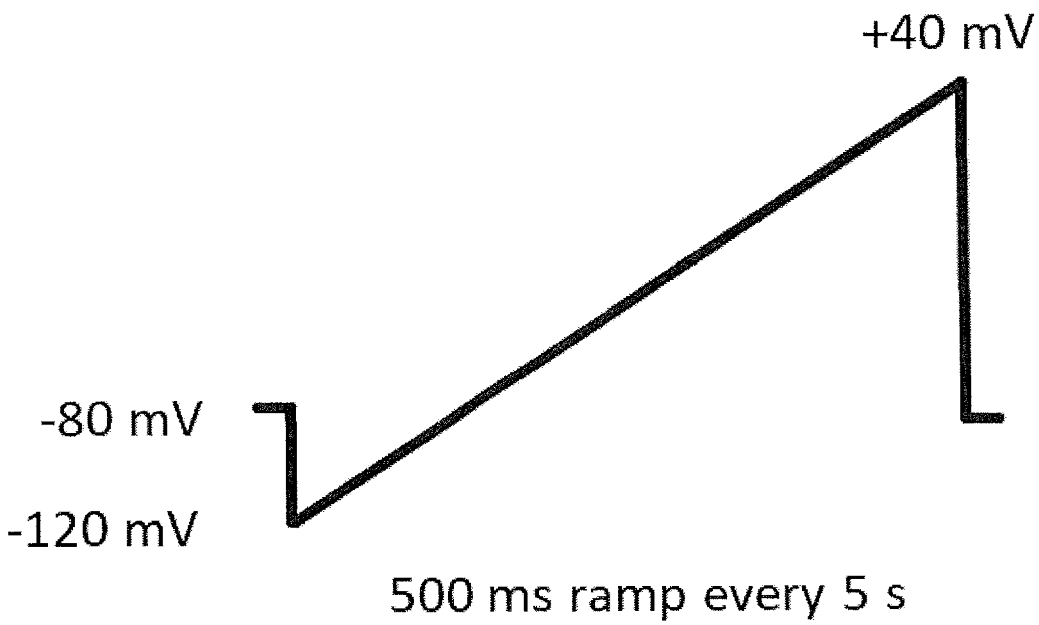


Figure 3

Europäisches Patentamt
European Patent Office
Office européen des brevets

# EUROPEAN SEARCH REPORT

Application Number
EP 19 20 8888

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2018/005713 A1 (LIANG CONGXIN [US]) 4 January 2018 (2018-01-04)<br>* claim 1 *<br>* examples 1-18 *<br>----- | 1-11, 13-20 | INV.<br>A61P25/02<br>A61P25/28<br>C07D207/06<br>C07D209/08<br>C07D211/14<br>C07D211/38<br>C07D211/46<br>C07D217/04<br>C07D277/32<br>C07D295/04<br>C07D295/205<br>C07D333/18<br>C07D333/24<br>C07D333/70<br>C07D401/12<br>C07D409/04<br>C07D471/08 |
| X | GRIMM C ET AL: "Small Molecule Activators of TRPML3", CHEMISTRY AND BIOLOGY, CURRENT BIOLOGY, LONDON, GB, vol. 17, no. 2, 26 February 2010 (2010-02-26), pages 135-148, XP026924867, ISSN: 1074-5521, DOI: 10.1016/J.CHEMBIOL.2009.12.016 [retrieved on 2010-02-25] | 1,2,4-7, 9-11, 14-20 | |
| Y | * table 1; compound SF21 *<br>* page 139, left column, 3-rd paragraph *<br>----- | 13 | |
| X | LISE BRÉTHOUS ET AL: "Synthesis and Nicotinic Receptor Activity of Chemical Space Analogues of N -(3 R )-1-Azabicyclo[2.2.2]oct-3-yl-4-chlorobenzamide (PNU-282,987) and 1,4-Diazabicyclo[3.2.2]nonane-4-carboxylic Acid 4-Bromophenyl Ester (SSR180711)", JOURNAL OF MEDICINAL CHEMISTRY, vol. compou55, no. 10, 16 May 2012 (2012-05-16), pages 4605-4618, XP055665311, US ISSN: 0022-2623, DOI: 10.1021/jm300030r<br>* compounds 7a, b, e, f *<br>-----<br>-/-- | 1,2,4-7, 9,10,16 | TECHNICAL FIELDS SEARCHED (IPC)<br>A61P<br>C07D |

~~The present search report has been drawn up for all claims~~

1

| Place of search | Date of completion of the search | Examiner |
|---|---|---|
| Munich | 10 February 2020 | Gutke, Hans-Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 19 20 8888

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/023667 A1 (HOFFMANN LA ROCHE [CH]; KOLCZEWSKI SABINE [DE]; PINARD EMMANUEL [FR]) 3 March 2011 (2011-03-03)<br>* claims 1, 17 *<br>* examples 20, 28, 47, 51, 56 *<br>* page 16, scheme 3 *<br>----- | 1-7,9, 10,16-20 | |
| X | US 6 977 265 B2 (ROCHE PALO ALTO LLC [US]) 20 December 2005 (2005-12-20)<br>* claims 1, 25 *<br>* example 2 *<br>----- | 1,2,4-7, 9,10, 16-19 | |
| X | TAKUJI HIROSE ET AL: "Switching of Enantioselectivity in the Catalytic Addition of Diethylzinc to Aldehydes by Regioisomeric Chiral 1,3-Amino Sulfonamide Ligands",<br>JOURNAL OF ORGANIC CHEMISTRY,<br>vol. 76, no. 13,<br>19 March 2011 (2011-03-19), pages 5413-5428, XP055665907,<br>US<br>ISSN: 0022-3263, DOI: 10.1021/jo200834n<br>* compounds 3c-i *<br>----- | 1,2,4-6, 8-10,16 | TECHNICAL FIELDS SEARCHED (IPC) |

-/--

1

~~The present search report has been drawn up for all claims~~

| Place of search | Date of completion of the search | Examiner |
|---|---|---|
| Munich | 10 February 2020 | Gutke, Hans-Jürgen |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 19 20 8888

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | COSTA DE B R ET AL: "SYNTHESIS AND EVALUATION OF S-SUBSTITUTED CIS-N-METHYL-2-(1-PYRROLIDINYL)CYCLOHEXYLAMINES AS HIGH AFFINITY S RECEPTOR LIGANDS. IDENTIFICATION OF A NEW CLASS OF HIGHLY POTENT AND SELECTIVE S RECEPTOR PROBES", JOURNAL OF MEDICINAL CHEMISTRY, AMERICAN CHEMICAL SOCIETY, US, vol. 33, no. 11, 1 January 1990 (1990-01-01), pages 3100-3110, XP000910297, ISSN: 0022-2623, DOI: 10.1021/JM00173A030 * page 3202; compounds 17, 2, 3 * * table II * * table V; compounds 2,29,35,38,43, * * scheme 3 * ----- | 1,2,4,5, 8-10,16 | |
| Y | DIOURY ET AL: "Synthesis of a tricyclic tetraazatriacetic ligand for gadolinium(III) as potential contrast agent for MRI", TETRAHEDRON, ELSEVIER SCIENCE PUBLISHERS, AMSTERDAM, NL, vol. 63, no. 1, 2 December 2006 (2006-12-02), pages 204-214, XP005788634, ISSN: 0040-4020, DOI: 10.1016/J.TET.2006.10.024 * schemes 2; and scheme 3, step c * ----- | 13 | TECHNICAL FIELDS SEARCHED (IPC) |

1

~~The present search report has been drawn up for all claims~~

| Place of search | Date of completion of the search | Examiner |
|---|---|---|
| Munich | 10 February 2020 | Gutke, Hans-Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

Application Number
EP 19 20 8888

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

13(completely); 1-11, 14-20(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 19 20 8888

The Search Division considers that the present European patent application does not comply with the requirements of unity of invention and relates to several inventions or groups of inventions, namely:

1. claims: 13(completely); 1-11, 14-20(partially)

   compounds of formula I wherein ring A is a six-membered aliphatic ring

   ---

2. claims: 12(completely); 1-11, 14-20(partially)

   compounds of formula I wherein ring A is a six-membered aromatic ring

   ---

3. claims: 1, 3-9, 14-20(all partially)

   compounds of formula I wherein ring A is a six-membered heteroaromatic ring

   ---

# ANNEX TO THE EUROPEAN SEARCH REPORT ON EUROPEAN PATENT APPLICATION NO. EP 19 20 8888

This annex lists the patent family members relating to the patent documents cited in the above-mentioned European search report.
The members are as contained in the European Patent Office EDP file on
The European Patent Office is in no way liable for these particulars which are merely given for the purpose of information.

10-02-2020

| Patent document cited in search report | Publication date | Patent family member(s) | Publication date |
|---|---|---|---|
| WO 2018005713 A1 | 04-01-2018 | US 2019248764 A1 | 15-08-2019 |
| | | WO 2018005713 A1 | 04-01-2018 |
| WO 2011023667 A1 | 03-03-2011 | AR 077960 A1 | 05-10-2011 |
| | | AU 2010288615 A1 | 09-02-2012 |
| | | BR 112012003976 A2 | 29-03-2016 |
| | | CA 2767901 A1 | 03-03-2011 |
| | | CN 102482203 A | 30-05-2012 |
| | | EP 2470498 A1 | 04-07-2012 |
| | | ES 2532660 T3 | 30-03-2015 |
| | | IL 217526 A | 29-10-2015 |
| | | JP 5538542 B2 | 02-07-2014 |
| | | JP 2013503128 A | 31-01-2013 |
| | | KR 20120047300 A | 11-05-2012 |
| | | SG 178576 A1 | 29-03-2012 |
| | | TW 201110962 A | 01-04-2011 |
| | | US 2011053904 A1 | 03-03-2011 |
| | | US 2012022042 A1 | 26-01-2012 |
| | | WO 2011023667 A1 | 03-03-2011 |
| US 6977265 B2 | 20-12-2005 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018005713 A


### Non-patent literature cited in the description


- *CHEMICAL ABSTRACTS,* 2006277-40-1, 39643-31-7, 1250695-37-4, 252758-95-5, 170017-74-0, 1018636-14-0, 1018624-48-0
- *CHEMICAL ABSTRACTS,* 98-59-9, 69815-97-0, 179400-17-0, 677782-39-7, 56921-00-7, 1314938-98-1, 883156-08-5, 90001-64-2, 103011-38-7, 55854-45-0
- *CHEMICAL ABSTRACTS,* 1017472-14-8, 29171-70-8
- *CHEMICAL ABSTRACTS,* 39643-31-7
- *CHEMICAL ABSTRACTS,* 98-59-9
- *CHEMICAL ABSTRACTS,* 69815-97-0
- *CHEMICAL ABSTRACTS,* 179400-17-0
- *CHEMICAL ABSTRACTS,* 1250695-37-4
- *CHEMICAL ABSTRACTS,* 677782-39-7
- *CHEMICAL ABSTRACTS,* 56921-00-7
- *CHEMICAL ABSTRACTS,* 1314938-98-1
- *CHEMICAL ABSTRACTS,* 883146-08-5
- *CHEMICAL ABSTRACTS,* 90001-64-2
- *CHEMICAL ABSTRACTS,* 103011-38-7
- *CHEMICAL ABSTRACTS,* 252758-95-5
- *CHEMICAL ABSTRACTS,* 170017-74-0
- *CHEMICAL ABSTRACTS,* 2006277-40-1
- *CHEMICAL ABSTRACTS,* 55854-45-0
- *CHEMICAL ABSTRACTS,* 29171-70-8
- *CHEMICAL ABSTRACTS,* 28338-22-9
- *CHEMICAL ABSTRACTS,* 15084-51-2
- *CHEMICAL ABSTRACTS,* 19715-49-2
- *CHEMICAL ABSTRACTS,* 1899-93-0
- *CHEMICAL ABSTRACTS,* 1018636-14-0
- *CHEMICAL ABSTRACTS,* 1018624-48-0
- *CHEMICAL ABSTRACTS,* 5585-33-1
- *CHEMICAL ABSTRACTS,* 2836-03-5
- *CHEMICAL ABSTRACTS,* 921090-59-7
- *CHEMICAL ABSTRACTS,* 165669-32-9
- *CHEMICAL ABSTRACTS,* 465514-13-0
- *CHEMICAL ABSTRACTS,* 21627-58-7
- *CHEMICAL ABSTRACTS,* 854044-39-6
- *CHEMICAL ABSTRACTS,* 1017472-14-8
- *CHEMICAL ABSTRACTS,* 1602318-01-3
- *CHEMICAL ABSTRACTS,* 1272785-78-0
- *CHEMICAL ABSTRACTS,* 72752-53-5
- *CHEMICAL ABSTRACTS,* 180629-70-3
- *CHEMICAL ABSTRACTS,* 246247-91-6
- *CHEMICAL ABSTRACTS,* 180605-36-1
- *CHEMICAL ABSTRACTS,* 75-36-5
- *CHEMICAL ABSTRACTS,* 79-22-1
- *CHEMICAL ABSTRACTS,* 27746-99-2
- *CHEMICAL ABSTRACTS,* 35386-24-4
- *CHEMICAL ABSTRACTS,* 771-99-3
- *CHEMICAL ABSTRACTS,* 92-54-6
- *CHEMICAL ABSTRACTS,* 3202-33-3
- *CHEMICAL ABSTRACTS,* 110-89-4
- *CHEMICAL ABSTRACTS,* 515160-75-5
- *CHEMICAL ABSTRACTS,* 110-91-8
- *CHEMICAL ABSTRACTS,* 496-15-1
- *CHEMICAL ABSTRACTS,* 98-60-2
- *CHEMICAL ABSTRACTS,* 98-74-8
- *CHEMICAL ABSTRACTS,* 98-68-0
- *CHEMICAL ABSTRACTS,* 541-41-3
- *CHEMICAL ABSTRACTS,* 79-03-8
- *CHEMICAL ABSTRACTS,* 4244-59-1
- *CHEMICAL ABSTRACTS,* 98-88-4
- *CHEMICAL ABSTRACTS,* 72752-54-6
- *CHEMICAL ABSTRACTS,* 199105-03-8
- *CHEMICAL ABSTRACTS,* 5271-67-0
- *CHEMICAL ABSTRACTS,* 54237-09-1
- *CHEMICAL ABSTRACTS,* 39098-97-0
- *CHEMICAL ABSTRACTS,* 51493-02-8
- *CHEMICAL ABSTRACTS,* 51627-46-4
- *CHEMICAL ABSTRACTS,* 107-10-8

- *CHEMICAL ABSTRACTS,* 78-81-9